# EUROPEAN PATENT APPLICATION

(11) **EP 4 685 240 A2**
(43) Date of publication of application: **28.01.2026**
(21) Application number: 25211643.9
(22) Date of filing: 07.02.2023
(51) Int. Cl.: C12Q 1/6806

(54) **REFERENCE BEADS FOR LINKING IMAGING AND SEQUENCING READOUTS WITH SINGLE-CELL RESOLUTION**

(30) Priority: 08.02.2022 US 202263307727 P
(62) Divisional of application: 23710159.7
(71) Applicant: Becton, Dickinson and Company, Franklin Lakes, NJ 07417-1880 (US)
(72) Inventor: SPUHLER, Philipp Stefan, San Jose, CA 95131 (US); SONG, Hye-Won, San Jose, CA 95131 (US)
(74) Representative: Carpmaels & Ransford LLP

(57) **Abstract**

Disclosed herein include systems, methods, compositions, and kits for associating single cell sequencing data with imaging data (e.g., phenotypic data). In some embodiments, particle indexing oligonucleotides are associated with reference particles. Reference particles can comprise one or more detectable moieties. The particle indexing oligonucleotide can comprise a particle type identifier (PTI) and a unique particle identifier (UPI). The PTI and the UPI can be employed to identify of the imaged partition from which a sequenced nucleic acid target molecule originated.

## Description

### RELATED APPLICATIONS

This application claims the benefit under 35 U.S.C. §119(e) of U.S. Provisional Patent Application Ser. No. 63/307,727, filed February 8, 2022, the content of this related application is incorporated herein by reference in its entirety for all purposes.

### BACKGROUND

### Field

The present disclosure relates generally to the field of molecular biology, for example determining gene expression using molecular barcoding.

### Description of the Related Art

Current technology allows measurement of gene expression of single cells in a massively parallel manner (e.g., >10000 cells) by attaching cell specific oligonucleotide barcodes to poly(A) mRNA molecules from individual cells as each of the cells is co-localized with a barcoded reagent bead in a compartment. Current single-cell multi-omics workflows also possess the capacity for capturing phenotypic data (e.g., imaging data) of single cells in said compartment. There is a need for compositions, systems, and methods for associating single cell sequencing data with imaging data (e.g., phenotypic data).

### SUMMARY

There are provided, in some embodiments, reference particles. In some embodiments, the reference particle comprises: one or more detectable moieties, or precursors thereof; and a particle indexing oligonucleotide, wherein the particle indexing oligonucleotide comprises a particle type identifier (PTI) and/or a unique particle identifier (UPI).

In some embodiments, the particle type identifier identifies the one or more detectable moieties associated with the reference particle. In some embodiments, the particle type identifier identifies the one or more detectable moieties associated with the reference particle and the density of said one or more detectable moieties associated with the reference particle. In some embodiments, the unique particle identifier is selected from a diverse set of unique particle identifiers. In some embodiments, the diverse set of unique particle identifiers comprises at least about 100 different unique particle identifiers, at least about 1000 different unique particle identifiers, at least about 10000 different unique particle identifiers, or at least about 100000 different unique particle identifiers.

In some embodiments, the one or more detectable moieties is at least about 2, 3, 4, 5, 6, or 7, different detectable moieties. In some embodiments, the detectable moiety comprises an optical moiety, a luminescent moiety, a magnetic moiety, an electrochemically active moiety, a nanoparticle, or a combination thereof. In some embodiments, the luminescent moiety comprises a chemiluminescent moiety, an electroluminescent moiety, a photoluminescent moiety, or a combination thereof. In some embodiments, the photoluminescent moiety comprises a fluorescent moiety, a phosphorescent moiety, or a combination thereof. In some embodiments, the fluorescent moiety comprises a fluorescent dye. In some embodiments, the nanoparticle comprises a quantum dot. In some embodiments, the particle type identifier and/or unique particle identifier is at least about 4 nucleotides in length.

In some embodiments, the reference particle comprises a synthetic particle. In some embodiments, the reference particle is disruptable. In some embodiments, the reference particle comprises a bead. In some embodiments, the bead comprises a Sepharose bead, a streptavidin bead, an agarose bead, a magnetic bead, a conjugated bead, a protein A conjugated bead, a protein G conjugated bead, a protein A/G conjugated bead, a protein L conjugated bead, an oligo(dT) conjugated bead, a silica bead, a silica-like bead, an anti-biotin microbead, an anti-fluorochrome microbead, or any combination thereof. In some embodiments, the reference particle comprises a material selected from the group consisting of polydimethylsiloxane (PDMS), polystyrene, glass, polypropylene, agarose, gelatin, hydrogel, paramagnetic, ceramic, plastic, glass, methylstyrene, acrylic polymer, titanium, latex, Sepharose, cellulose, nylon, silicone, and any combination thereof. In some embodiments, the reference particle comprises a disruptable hydrogel particle.

In some embodiments, the particle indexing oligonucleotide is 6-60 nucleotides in length. In some embodiments, the particle indexing oligonucleotide is immobilized on the reference particle, partially immobilized on the reference particle, enclosed in the reference particle, partially enclosed in the reference particle, or a combination thereof. In some embodiments, the particle indexing oligonucleotide is associated with the reference particle via one or more cleavable linkers. In some embodiments, the one or more cleavable linkers comprise acid-labile linkers, base-labile linkers, photocleavable linkers, enzyme-cleavable linkers, or any combination thereof. In some embodiments, the particle indexing oligonucleotide comprises a sequence complementary to a capture sequence configured to capture the particle indexing oligonucleotide. In some embodiments, the sequence complementary to the capture sequence comprises a poly(dA) region. In some embodiments, the particle indexing oligonucleotide comprises a particle linker functional group. In some embodiments, the reference particle comprises a particle functional group. In some embodiments, the particle functional group and the particle linker functional group are associated with each other. In some embodiments, the particle linker functional group and the particle functional group are individually selected from the group consisting of C6, biotin, streptavidin, primary amine(s), aldehyde(s), ketone(s), and any combination thereof. In some embodiments, the particle indexing oligonucleotide is associated with the reference particle through a particle linker. In some embodiments, the particle linker comprises a carbon chain, optionally the carbon chain comprises 2-30 carbons. In some embodiments, the particle linker comprises 5' amino modifier C12 (5AmMC12), or a derivative thereof. In some embodiments, the particle indexing oligonucleotide is 50-500 nucleotides in length. In some embodiments, the particle indexing oligonucleotide is conjugated to the reference particle through a chemical group selected from the group consisting of a UV photocleavable group, a streptavidin, a biotin, an amine, and a combination thereof. In some embodiments, the particle indexing oligonucleotide is non-covalently attached to the particle. In some embodiments, the particle indexing oligonucleotide is double-stranded or single-stranded, and wherein the particle indexing oligonucleotide comprises DNA and/or RNA.

There are provided, in some embodiments, populations of reference particles. In some embodiments, the population of reference particles comprises: two or more reference particles disclosed herein, wherein at least two of the two or more reference particles differ from each other with respect to the identity of the one or more detectable moieties associated with the reference particle and/or the density of said one or more detectable moieties associated with the reference particle. In some embodiments, at least 3, 4, 5, 6, 7, 8, 9, 10, 11, or 12, of the two or more reference particles differ from each other with respect to the one or more detectable moieties associated with the reference particle and/or the density of said one or more detectable moieties on the reference particle.

There are provided, in some embodiments, methods of assigning sequencing data to partitions. In some embodiments, the method comprises: partitioning a plurality of solid supports and a plurality of single cells to the plurality of partitions, wherein the plurality of solid supports each comprise a plurality of oligonucleotide barcodes each comprising a cell label, wherein oligonucleotide barcodes associated with the same solid support comprise the same cell label sequence, and wherein oligonucleotide barcodes associated with different solid supports comprise different cell label sequences; partitioning the reference particles disclosed herein and/or the population of reference particle disclosed herein to the plurality of partitions, barcoding the particle indexing oligonucleotides using the plurality of oligonucleotide barcodes to generate a plurality of barcoded particle indexing oligonucleotides; barcoding the copies of a nucleic acid target from at least one of the plurality of single cells using the plurality of oligonucleotide barcodes to generate a plurality of barcoded nucleic acid molecules; obtaining imaging data of the plurality of partitions to identify the detectable signature of each partition; obtaining sequencing data comprising a plurality of sequencing reads of the barcoded particle indexing oligonucleotides, or products thereof, and the plurality of barcoded nucleic acid molecules, or products thereof: identifying the PTI and/or UPI associated with each cell label sequence in the sequencing data; and assigning each of the plurality of sequencing reads to a partition of the plurality of partitions based on the PTI and/or UPI associated with each cell label sequence in the sequencing data.

The method can comprise: obtaining phenotypic data of the plurality of single cells; and associating the sequencing data and the phenotypic data of at least one cell of the plurality of single cells based on the PTI and/or UPI of at least one barcoded particle indexing oligonucleotide, or product thereof, of the plurality of barcoded particle indexing oligonucleotides, or products thereof, in the sequencing data. In some embodiments, imaging each partition yields phenotypic data. In some embodiments, imaging comprises microscopy, time-lapse imaging microscopy, fluorescence microscopy, multi-photon microscopy, quantitative phase microscopy, surface enhanced Raman spectroscopy, videography, manual visual analysis, automated visual analysis, and combinations thereof.

The method can comprise: associating the plurality of sequencing reads of the barcoded particle indexing oligonucleotides, or products thereof, of each partition, with the detectable signature of each partition. In some embodiments, each partition of the plurality of partitions comprises a unique detectable signature, and wherein the detectable signature of each partition varies depending on the identity of the reference particle(s) in said partitions. In some embodiments, identifying the detectable signature of each partition comprises detecting the emissions of the one or more detectable moieties of each partition, optionally said emissions comprise fluorescence emissions.

In some embodiments, the particle indexing oligonucleotide is configured to be detachable from the reference particle. In some embodiments, the particle indexing oligonucleotide is configured detach from the reference particle during cell lysis. The method can comprise: dissociating the particle indexing oligonucleotide from the reference particle. In some embodiments, dissociating the particle indexing oligonucleotide from the reference particle comprises detaching the partition indexing oligonucleotide from the partition by UV photocleaving, chemical treatment, heating, enzyme treatment, or any combination thereof. In some embodiments, the dissociating occurs after barcoding the particle indexing oligonucleotides. In some embodiments, the dissociating occurs before barcoding the particle indexing oligonucleotides. In some embodiments, the dissociating occurs during cell lysis. In some embodiments, the partitions comprise micro-wells or droplets.

There are provided, in some embodiments, compositions. In some embodiments, the composition comprises: a micro-well array, wherein the micro-well array comprises at least 100 micro-wells, wherein each micro-well has a volume ranging from about 1,000 µm3 to about 786,000 µm3, wherein each micro-well comprises one or more reference particles disclosed herein. The composition can comprise: a cartridge, wherein the cartridge comprises at least one of: an inlet port, an outlet port, a pump, a valve, a vent, a reservoir, a sample collection chamber, a temperature control apparatus, or any combination thereof.

In some embodiments, the composition comprises: a cartridge, wherein the cartridge comprises at least one of: an inlet port, an outlet port, a pump, a valve, a vent, a reservoir, a sample collection chamber, a temperature control apparatus, or any combination thereof, wherein the cartridge comprises a micro-well array, wherein the micro-well array comprises at least 100 micro-wells, wherein each micro-well has a volume ranging from about 1,000 µm3 to about 786,000 µm3, wherein each micro-well comprises one or more reference particles disclosed herein.

The composition can comprise: a buffer. The composition can comprise: one or more reagents for a reverse transcription reaction. The composition can comprise: one or more reagents for an amplification reaction. In some embodiments, the cartridge comprises a transparent window for optical imaging of the at least 100 microwells. The composition can comprise: an imaging system configured to capture and process images of all or a portion of the at least 100 microwells, wherein the imaging system further comprises an illumination subsystem, an imaging subsystem, and a processor. In some embodiments, the imaging system is configured to perform bright-field, dark-field, fluorescence, or quantitative phase imaging.

### BRIEF DESCRIPTION OF THE DRAWINGS

FIG. 1 illustrates a non-limiting exemplary barcode.
FIG. 2 shows a non-limiting exemplary workflow of barcoding and digital counting.
FIG. 3 is a schematic illustration showing a non-limiting exemplary process for generating an indexed library of targets barcoded at the 3' -ends from a plurality of targets.
FIG. 4A depicts a non-limiting exemplary workflow provided herein.
FIG. 4B depicts a non-limiting exemplary workflow provided herein.
FIG. 5 depict non-limiting exemplary workflows of a currently available method and a method described herein (e.g., a cell secretion assay).
FIG. 6 depicts a non-limiting exemplary schematic of the reference particles (e.g., reference beads) provided herein situated within partitions (e.g., microwells). A mixture of reference particles having red, green, or blue fluor colors is depicted.
FIG. 7 depicts a non-limiting exemplary schematic of a reference particle (e.g., reference bead) provided herein. The reference particle is depicted having fluor colors red (R) and green (G).
FIG. 8 depicts a non-limiting exemplary schematic of reference particle populations (e.g., reference bead populations) provided herein. The reference particles are depicted having fluor colors red (R) and/or green (G).
FIG. 9 depicts a non-limiting exemplary schematic of reference particle populations (e.g., reference bead populations) provided herein wherein a variety of reference particle types (e.g., reference bead types) can be generated by varying the density of detectable moieties (e.g., fluor colors) conjugated to the reference beads. The reference particles are depicted having fluor colors red (R) and/or green (G).
FIG. 10 depicts a non-limiting exemplary schematic of reference particle populations (e.g., reference bead populations) provided herein. The reference particles are depicted having fluor colors red (R) and/or green (G).
FIG. 11 depicts a non-limiting exemplary schematic of 4 partitions (e.g., microwells), each with a unique combination of reference beads, and how the reference beads can be counted by imaging and by sequencing readouts. A mixture of reference particles having red, green, or blue fluor colors is depicted.
FIG. 12 depicts a non-limiting exemplary schematic of a reference particle (e.g., reference bead) provided herein. A mixture of reference particles having red (R), green (G), or blue (B) fluor colors is depicted.
FIG. 13 depicts a non-limiting exemplary sample workflow provided herein. A mixture of reference particles having red, green, or blue fluor colors is depicted.
FIG. 14 depicts a calculation for the number of reference beads that can be loaded for a given microwell geometry and imaging system.
FIG. 15A depicts non-limiting exemplary probability distribution function (PDF) for reference beads loaded microwells for a given lambda (mean beads per microwell) and with 1 bead type.
FIG. 15B depicts a non-limiting expected number of microwells containing a given number of beads for a given lambda.
FIGS. 16A-16B depict the same analysis as FIGS. 15A-15B if the number of bead types is increased from 1 to 2.
FIG. 17 shows the same analysis as FIGS. 15A-15B and FIGS. 16A-16B, extended for a range of bead types (0-10) and a range of lambdas (0.1 - 100).

### DETAILED DESCRIPTION

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made, without departing from the spirit or scope of the subject matter presented herein. It will be readily understood that the aspects of the present disclosure, as generally described herein, and illustrated in the Figures, can be arranged, substituted, combined, separated, and designed in a wide variety of different configurations, all of which are explicitly contemplated herein and made part of the disclosure herein.

All patents, published patent applications, other publications, and sequences from GenBank, and other databases referred to herein are incorporated by reference in their entirety with respect to the related technology.

Quantifying small numbers of nucleic acids, for example messenger ribonucleotide acid (mRNA) molecules, is clinically important for determining, for example, the genes that are expressed in a cell at different stages of development or under different environmental conditions. However, it can also be very challenging to determine the absolute number of nucleic acid molecules (e.g., mRNA molecules), especially when the number of molecules is very small. One method to determine the absolute number of molecules in a sample is digital polymerase chain reaction (PCR). Ideally, PCR produces an identical copy of a molecule at each cycle. However, PCR can have disadvantages such that each molecule replicates with a stochastic probability, and this probability varies by PCR cycle and gene sequence, resulting in amplification bias and inaccurate gene expression measurements. Stochastic barcodes with unique molecular labels (also referred to as molecular indexes (MIs)) can be used to count the number of molecules and correct for amplification bias. Stochastic barcoding, such as the Precise^{™} assay (Cellular Research, Inc. (Palo Alto, CA)) and Rhapsody^{™} assay (Becton, Dickinson and Company (Franklin Lakes, NJ)), can correct for bias induced by PCR and library preparation steps by using molecular labels (MLs) to label mRNAs during reverse transcription (RT).

The Precise^{™} assay can utilize a non-depleting pool of stochastic barcodes with large number, for example 6561 to 65536, unique molecular label sequences on poly(T) oligonucleotides to hybridize to all poly(A)-mRNAs in a sample during the RT step. A stochastic barcode can comprise a universal PCR priming site. During RT, target gene molecules react randomly with stochastic barcodes. Each target molecule can hybridize to a stochastic barcode resulting to generate stochastically barcoded complementary ribonucleotide acid (cDNA) molecules). After labeling, stochastically barcoded cDNA molecules from microwells of a microwell plate can be pooled into a single tube for PCR amplification and sequencing. Raw sequencing data can be analyzed to produce the number of reads, the number of stochastic barcodes with unique molecular label sequences, and the numbers of mRNA molecules.

There are provided, in some embodiments, reference particles. In some embodiments, the reference particle comprises: one or more detectable moieties, or precursors thereof; and a particle indexing oligonucleotide, wherein the particle indexing oligonucleotide comprises a particle type identifier (PTI) and/or a unique particle identifier (UPI).

There are provided, in some embodiments, populations of reference particles. In some embodiments, the population of reference particles comprises: two or more reference particles disclosed herein, wherein at least two of the two or more reference particles differ from each other with respect to the identity of the one or more detectable moieties associated with the reference particle and/or the density of said one or more detectable moieties associated with the reference particle. In some embodiments, at least 3, 4, 5, 6, 7, 8, 9, 10, 11, or 12, of the two or more reference particles differ from each other with respect to the one or more detectable moieties associated with the reference particle and/or the density of said one or more detectable moieties on the reference particle.

There are provided, in some embodiments, methods of assigning sequencing data to partitions. In some embodiments, the method comprises: partitioning a plurality of solid supports and a plurality of single cells to the plurality of partitions, wherein the plurality of solid supports each comprise a plurality of oligonucleotide barcodes each comprising a cell label, wherein oligonucleotide barcodes associated with the same solid support comprise the same cell label sequence, and wherein oligonucleotide barcodes associated with different solid supports comprise different cell label sequences; partitioning the reference particles disclosed herein and/or the population of reference particle disclosed herein to the plurality of partitions, barcoding the particle indexing oligonucleotides using the plurality of oligonucleotide barcodes to generate a plurality of barcoded particle indexing oligonucleotides; barcoding the copies of a nucleic acid target from at least one of the plurality of single cells using the plurality of oligonucleotide barcodes to generate a plurality of barcoded nucleic acid molecules; obtaining imaging data of the plurality of partitions to identify the detectable signature of each partition; obtaining sequencing data comprising a plurality of sequencing reads of the barcoded particle indexing oligonucleotides, or products thereof, and the plurality of barcoded nucleic acid molecules, or products thereof; identifying the particle type identifier (PTI) and/or unique particle identifier (UPI) associated with each cell label sequence in the sequencing data; and assigning each of the plurality of sequencing reads to a partition of the plurality of partitions based on the particle type identifier (PTI) and/or unique particle identifier (UPI) associated with each cell label sequence in the sequencing data.

There are provided, in some embodiments, compositions. In some embodiments, the composition comprises: a micro-well array, wherein the micro-well array comprises at least 100 micro-wells, wherein each micro-well has a volume ranging from about 1,000 µm3 to about 786,000 µm3, wherein each micro-well comprises one or more reference particles disclosed herein. The composition can comprise: a cartridge, wherein the cartridge comprises at least one of: an inlet port, an outlet port, a pump, a valve, a vent, a reservoir, a sample collection chamber, a temperature control apparatus, or any combination thereof.

In some embodiments, the composition comprises: a cartridge, wherein the cartridge comprises at least one of: an inlet port, an outlet port, a pump, a valve, a vent, a reservoir, a sample collection chamber, a temperature control apparatus, or any combination thereof, wherein the cartridge comprises a micro-well array, wherein the micro-well array comprises at least 100 micro-wells, wherein each micro-well has a volume ranging from about 1,000 µm3 to about 786,000 µm3, wherein each micro-well comprises one or more reference particles disclosed herein.

### Definitions

Unless defined otherwise, technical and scientific terms used herein have the same meaning as commonly understood by one of ordinary skill in the art to which the present disclosure belongs. *See, e.g.,* Singleton et al., Dictionary of Microbiology and Molecular Biology 2nd ed., J. Wiley & Sons (New York, NY 1994); Sambrook et al., Molecular Cloning, A Laboratory Manual, Cold Spring Harbor Press (Cold Spring Harbor, NY 1989). For purposes of the present disclosure, the following terms are defined below.

As used herein, the term "adaptor" can mean a sequence to facilitate amplification or sequencing of associated nucleic acids. The associated nucleic acids can comprise target nucleic acids. The associated nucleic acids can comprise one or more of spatial labels, target labels, sample labels, indexing label, or barcode sequences (e.g., molecular labels). The adaptors can be linear. The adaptors can be pre-adenylated adaptors. The adaptors can be double- or single-stranded. One or more adaptor can be located on the 5' or 3' end of a nucleic acid. When the adaptors comprise known sequences on the 5' and 3' ends, the known sequences can be the same or different sequences. An adaptor located on the 5' and/or 3' ends of a polynucleotide can be capable of hybridizing to one or more oligonucleotides immobilized on a surface. An adaptor can, in some embodiments, comprise a universal sequence. A universal sequence can be a region of nucleotide sequence that is common to two or more nucleic acid molecules. The two or more nucleic acid molecules can also have regions of different sequence. Thus, for example, the 5' adaptors can comprise identical and/or universal nucleic acid sequences and the 3' adaptors can comprise identical and/or universal sequences. A universal sequence that may be present in different members of a plurality of nucleic acid molecules can allow the replication or amplification of multiple different sequences using a single universal primer that is complementary to the universal sequence. Similarly, at least one, two (e.g., a pair) or more universal sequences that may be present in different members of a collection of nucleic acid molecules can allow the replication or amplification of multiple different sequences using at least one, two (e.g., a pair) or more single universal primers that are complementary to the universal sequences. Thus, a universal primer includes a sequence that can hybridize to such a universal sequence. The target nucleic acid sequence-bearing molecules may be modified to attach universal adaptors (e.g., non-target nucleic acid sequences) to one or both ends of the different target nucleic acid sequences. The one or more universal primers attached to the target nucleic acid can provide sites for hybridization of universal primers. The one or more universal primers attached to the target nucleic acid can be the same or different from each other.

As used herein the term "associated" or "associated with" can mean that two or more species are identifiable as being co-located at a point in time. An association can mean that two or more species are or were within a similar container. An association can be an informatics association. For example, digital information regarding two or more species can be stored and can be used to determine that one or more of the species were co-located at a point in time. An association can also be a physical association. In some embodiments, two or more associated species are "tethered", "attached", or "immobilized" to one another or to a common solid or semisolid surface. An association may refer to covalent or non-covalent means for attaching labels to solid or semi-solid supports such as beads. An association may be a covalent bond between a target and a label. An association can comprise hybridization between two molecules (such as a target molecule and a label).

As used herein, the term "complementary" can refer to the capacity for precise pairing between two nucleotides. For example, if a nucleotide at a given position of a nucleic acid is capable of hydrogen bonding with a nucleotide of another nucleic acid, then the two nucleic acids are considered to be complementary to one another at that position. Complementarity between two single-stranded nucleic acid molecules may be "partial," in which only some of the nucleotides bind, or it may be complete when total complementarity exists between the single-stranded molecules. A first nucleotide sequence can be said to be the "complement" of a second sequence if the first nucleotide sequence is complementary to the second nucleotide sequence. A first nucleotide sequence can be said to be the "reverse complement" of a second sequence, if the first nucleotide sequence is complementary to a sequence that is the reverse (i.e., the order of the nucleotides is reversed) of the second sequence. As used herein, a "complementary" sequence can refer to a "complement" or a "reverse complement" of a sequence. It is understood from the disclosure that if a molecule can hybridize to another molecule it may be complementary, or partially complementary, to the molecule that is hybridizing.

As used herein, the term "digital counting" can refer to a method for estimating a number of target molecules in a sample. Digital counting can include the step of determining a number of unique labels that have been associated with targets in a sample. This methodology, which can be stochastic in nature, transforms the problem of counting molecules from one of locating and identifying identical molecules to a series of yes/no digital questions regarding detection of a set of predefined labels.

As used herein, the term "label" or "labels" can refer to nucleic acid codes associated with a target within a sample. A label can be, for example, a nucleic acid label. A label can be an entirely or partially amplifiable label. A label can be entirely or partially sequencable label. A label can be a portion of a native nucleic acid that is identifiable as distinct. A label can be a known sequence. A label can comprise a junction of nucleic acid sequences, for example a junction of a native and non-native sequence. As used herein, the term "label" can be used interchangeably with the terms, "index", "tag," or "label-tag." Labels can convey information. For example, in various embodiments, labels can be used to determine an identity of a sample, a source of a sample, an identity of a cell, and/or a target.

As used herein, the term "non-depleting reservoirs" can refer to a pool of barcodes (e.g., stochastic barcodes) made up of many different labels. A non-depleting reservoir can comprise large numbers of different barcodes such that when the non-depleting reservoir is associated with a pool of targets each target is likely to be associated with a unique barcode. The uniqueness of each labeled target molecule can be determined by the statistics of random choice, and depends on the number of copies of identical target molecules in the collection compared to the diversity of labels. The size of the resulting set of labeled target molecules can be determined by the stochastic nature of the barcoding process, and analysis of the number of barcodes detected then allows calculation of the number of target molecules present in the original collection or sample. When the ratio of the number of copies of a target molecule present to the number of unique barcodes is low, the labeled target molecules are highly unique (i.e., there is a very low probability that more than one target molecule will have been labeled with a given label).

As used herein, the term "nucleic acid" refers to a polynucleotide sequence, or fragment thereof. A nucleic acid can comprise nucleotides. A nucleic acid can be exogenous or endogenous to a cell. A nucleic acid can exist in a cell-free environment. A nucleic acid can be a gene or fragment thereof. A nucleic acid can be DNA. A nucleic acid can be RNA. A nucleic acid can comprise one or more analogs (e.g., altered backbone, sugar, or nucleobase). Some non-limiting examples of analogs include: 5-bromouracil, peptide nucleic acid, xeno nucleic acid, morpholinos, locked nucleic acids, glycol nucleic acids, threose nucleic acids, dideoxynucleotides, cordycepin, 7-deaza-GTP, fluorophores (e.g., rhodamine or fluorescein linked to the sugar), thiol containing nucleotides, biotin linked nucleotides, fluorescent base analogs, CpG islands, methyl-7-guanosine, methylated nucleotides, inosine, thiouridine, pseudouridine, dihydrouridine, queuosine, and wyosine. "Nucleic acid", "polynucleotide, "target polynucleotide", and "target nucleic acid" can be used interchangeably.

A nucleic acid can comprise one or more modifications (e.g., a base modification, a backbone modification), to provide the nucleic acid with a new or enhanced feature (e.g., improved stability). A nucleic acid can comprise a nucleic acid affinity tag. A nucleoside can be a base-sugar combination. The base portion of the nucleoside can be a heterocyclic base. The two most common classes of such heterocyclic bases are the purines and the pyrimidines. Nucleotides can be nucleosides that further include a phosphate group covalently linked to the sugar portion of the nucleoside. For those nucleosides that include a pentofuranosyl sugar, the phosphate group can be linked to the 2', the 3', or the 5' hydroxyl moiety of the sugar. In forming nucleic acids, the phosphate groups can covalently link adjacent nucleosides to one another to form a linear polymeric compound. In turn, the respective ends of this linear polymeric compound can be further joined to form a circular compound; however, linear compounds are generally suitable. In addition, linear compounds may have internal nucleotide base complementarity and may therefore fold in a manner as to produce a fully or partially double-stranded compound. Within nucleic acids, the phosphate groups can commonly be referred to as forming the internucleoside backbone of the nucleic acid. The linkage or backbone can be a 3' to 5' phosphodiester linkage.

A nucleic acid can comprise a modified backbone and/or modified internucleoside linkages. Modified backbones can include those that retain a phosphorus atom in the backbone and those that do not have a phosphorus atom in the backbone. Suitable modified nucleic acid backbones containing a phosphorus atom therein can include, for example, phosphorothioates, chiral phosphorothioates, phosphorodithioates, phosphotriesters, aminoalkyl phosphotriesters, methyl and other alkyl phosphonate such as 3'-alkylene phosphonates, 5'-alkylene phosphonates, chiral phosphonates, phosphinates, phosphoramidates including 3'-amino phosphoramidate and aminoalkyl phosphoramidates, phosphorodiamidates, thionophosphoramidates, thionoalkylphosphonates, thionoalkylphosphotriesters, selenophosphates, and boranophosphates having normal 3'-5' linkages, 2'-5' linked analogs, and those having inverted polarity wherein one or more internucleotide linkages is a 3' to 3', a 5' to 5' or a 2' to 2' linkage.

A nucleic acid can comprise polynucleotide backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These can include those having morpholino linkages (formed in part from the sugar portion of a nucleoside): siloxane backbones; sulfide, sulfoxide and sulfone backbones: formacetyl and thioformacetyl backbones; methylene formacetyl and thioformacetyl backbones: riboacetyl backbones: alkene containing backbones; sulfamate backbones; methyleneimino and methylenehydrazino backbones; sulfonate and sulfonamide backbones; amide backbones: and others having mixed N, O, S and CH₂ component parts.

A nucleic acid can comprise a nucleic acid mimetic. The term "mimetic" can be intended to include polynucleotides wherein only the furanose ring or both the furanose ring and the intemucleotide linkage are replaced with non-furanose groups, replacement of only the furanose ring can also be referred as being a sugar surrogate. The heterocyclic base moiety or a modified heterocyclic base moiety can be maintained for hybridization with an appropriate target nucleic acid. One such nucleic acid can be a peptide nucleic acid (PNA). In a PNA, the sugar-backbone of a polynucleotide can be replaced with an amide containing backbone, in particular an aminoethylglycine backbone. The nucleotides can be retained and are bound directly or indirectly to aza nitrogen atoms of the amide portion of the backbone. The backbone in PNA compounds can comprise two or more linked aminoethylglycine units which gives PNA an amide containing backbone. The heterocyclic base moieties can be bound directly or indirectly to aza nitrogen atoms of the amide portion of the backbone.

A nucleic acid can comprise a morpholino backbone structure. For example, a nucleic acid can comprise a 6-membered morpholino ring in place of a ribose ring. In some of these embodiments, a phosphorodiamidate or other non-phosphodiester internucleoside linkage can replace a phosphodiester linkage.

A nucleic acid can comprise linked morpholino units (e.g., morpholino nucleic acid) having heterocyclic bases attached to the morpholino ring. Linking groups can link the morpholino monomeric units in a morpholino nucleic acid. Non-ionic morpholino-based oligomeric compounds can have less undesired interactions with cellular proteins. Morpholino-based polynucleotides can be nonionic mimics of nucleic acids. A variety of compounds within the morpholino class can be joined using different linking groups. A further class of polynucleotide mimetic can be referred to as cyclohexenyl nucleic acids (CeNA). The furanose ring normally present in a nucleic acid molecule can be replaced with a cyclohexenyl ring. CeNA DMT protected phosphoramidite monomers can be prepared and used for oligomeric compound synthesis using phosphoramidite chemistry. The incorporation of CeNA monomers into a nucleic acid chain can increase the stability of a DNA/RNA hybrid. CeNA oligoadenylates can form complexes with nucleic acid complements with similar stability to the native complexes. A further modification can include Locked Nucleic Acids (LNAs) in which the 2'-hydroxyl group is linked to the 4' carbon atom of the sugar ring thereby forming a 2'-C, 4'-C-oxymethylene linkage thereby forming a bicyclic sugar moiety. The linkage can be a methylene (-CH₂), group bridging the 2' oxygen atom and the 4' carbon atom wherein *n* is 1 or 2. LNA and LNA analogs can display very high duplex thermal stabilities with complementary nucleic acid (Tm=+3 to +10 °C), stability towards 3'-exonucleolytic degradation and good solubility properties.

A nucleic acid may also include nucleobase (often referred to simply as "base") modifications or substitutions. As used herein, "unmodified" or "natural" nucleobases can include the purine bases, (e.g., adenine (A) and guanine (G)), and the pyrimidine bases, (e.g., thymine (T), cytosine (C) and uracil (U)). Modified nucleobases can include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl (-C=C-CH3) uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 2-F-adenine, 2-aminoadenine, 8-azaguanine and 8-azaadenine, 7-deazaguanine and 7-deazaadenine and 3-deazaguanine and 3-deazaadenine. Modified nucleobases can include tricyclic pyrimidines such as phenoxazine cytidine(1H-pyrimido(5,4-b)(1,4)benzoxazin-2(3H)-one), phenothiazine cytidine (1H-pyrimido(5,4-b)(1,4)benzothiazin-2(3H)-one), G-clamps such as a substituted phenoxazine cytidine (e.g., 9-(2-aminoethoxy)-H-pyrimido(5,4-(b) (1,4)benzoxazin-2(3H)-one), phenothiazine cytidine (1H-pyrimido(5,4-b)(1,4)benzothiazin-2(3H)-one), G-clamps such as a substituted phenoxazine cytidine (e.g., 9-(2-aminoethoxy)-H-pyrimido(5,4-(b) (1,4)benzoxazin-2(3H)-one), carbazole cytidine (2H-pyrimido(4,5-b)indol-2-one), pyridoindole cytidine (H-pyrido(3',2':4,5)pyrrolo[2,3-d]pyrimidin-2-one).

As used herein, the term "sample" can refer to a composition comprising targets. Suitable samples for analysis by the disclosed methods, devices, and systems include cells, tissues, organs, or organisms.

As used herein, the term "sampling device" or "device" can refer to a device which may take a section of a sample and/or place the section on a substrate. A sample device can refer to, for example, a fluorescence activated cell sorting (FACS) machine, a cell sorter machine, a biopsy needle, a biopsy device, a tissue sectioning device, a microfluidic device, a blade grid, and/or a microtome.

As used herein, the term "solid support" can refer to discrete solid or semi-solid surfaces to which a plurality of barcodes (e.g., stochastic barcodes) may be attached. A solid support may encompass any type of solid, porous, or hollow sphere, ball, bearing, cylinder, or other similar configuration composed of plastic, ceramic, metal, or polymeric material (e.g., hydrogel) onto which a nucleic acid may be immobilized (e.g., covalently or non-covalently). A solid support may comprise a discrete particle that may be spherical (e.g., microspheres) or have a non-spherical or irregular shape, such as cubic, cuboid, pyramidal, cylindrical, conical, oblong, or disc-shaped, and the like. A bead can be non-spherical in shape. A plurality of solid supports spaced in an array may not comprise a substrate. A solid support may be used interchangeably with the term "bead."

As used herein, the term "stochastic barcode" can refer to a polynucleotide sequence comprising labels of the present disclosure. A stochastic barcode can be a polynucleotide sequence that can be used for stochastic barcoding. Stochastic barcodes can be used to quantify targets within a sample. Stochastic barcodes can be used to control for errors which may occur after a label is associated with a target. For example, a stochastic barcode can be used to assess amplification or sequencing errors. A stochastic barcode associated with a target can be called a stochastic barcode-target or stochastic barcode-tag-target.

As used herein, the term "gene-specific stochastic barcode" can refer to a polynucleotide sequence comprising labels and a target-binding region that is gene-specific. A stochastic barcode can be a polynucleotide sequence that can be used for stochastic barcoding. Stochastic barcodes can be used to quantify targets within a sample. Stochastic barcodes can be used to control for errors which may occur after a label is associated with a target. For example, a stochastic barcode can be used to assess amplification or sequencing errors. A stochastic barcode associated with a target can be called a stochastic barcode-target or stochastic barcode-tag-target.

As used herein, the term "stochastic barcoding" can refer to the random labeling (e.g., barcoding) of nucleic acids. Stochastic barcoding can utilize a recursive Poisson strategy to associate and quantify labels associated with targets. As used herein, the term "stochastic barcoding" can be used interchangeably with "stochastic labeling."

As used here, the term "target" can refer to a composition which can be associated with a barcode (e.g., a stochastic barcode). Exemplary suitable targets for analysis by the disclosed methods, devices, and systems include oligonucleotides, DNA, RNA, mRNA, microRNA, tRNA, and the like. Targets can be single or double stranded. In some embodiments, targets can be proteins, peptides, or polypeptides. In some embodiments, targets are lipids. As used herein, "target" can be used interchangeably with "species."

As used herein, the term "reverse transcriptases" can refer to a group of enzymes having reverse transcriptase activity (i.e., that catalyze synthesis of DNA from an RNA template). In general, such enzymes include, but are not limited to, retroviral reverse transcriptase, retrotransposon reverse transcriptase, retroplasmid reverse transcriptases, retron reverse transcriptases, bacterial reverse transcriptases, group II intron-derived reverse transcriptase, and mutants, variants or derivatives thereof. Non-retroviral reverse transcriptases include non-LTR retrotransposon reverse transcriptases, retroplasmid reverse transcriptases, retron reverse transciptases, and group II intron reverse transcriptases. Examples of group II intron reverse transcriptases include the *Lactococcus lactis* LI.LtrB intron reverse transcriptase, the *Thermosynechococcus* elongatus TeI4c intron reverse transcriptase, or the *Geobacillus stearothermophilus* GsI-IIC intron reverse transcriptase. Other classes of reverse transcriptases can include many classes of non-retroviral reverse transcriptases (i.e., retrons, group II introns, and diversity-generating retroelements among others).

The terms "universal adaptor primer," "universal primer adaptor" or "universal adaptor sequence" are used interchangeably to refer to a nucleotide sequence that can be used to hybridize to barcodes (e.g., stochastic barcodes) to generate gene-specific barcodes. A universal adaptor sequence can, for example, be a known sequence that is universal across all barcodes used in methods of the disclosure. For example, when multiple targets are being labeled using the methods disclosed herein, each of the target-specific sequences may be linked to the same universal adaptor sequence. In some embodiments, more than one universal adaptor sequences may be used in the methods disclosed herein. For example, when multiple targets are being labeled using the methods disclosed herein, at least two of the target-specific sequences are linked to different universal adaptor sequences. A universal adaptor primer and its complement may be included in two oligonucleotides, one of which comprises a target-specific sequence and the other comprises a barcode. For example, a universal adaptor sequence may be part of an oligonucleotide comprising a target-specific sequence to generate a nucleotide sequence that is complementary to a target nucleic acid. A second oligonucleotide comprising a barcode and a complementary sequence of the universal adaptor sequence may hybridize with the nucleotide sequence and generate a target-specific barcode (e.g., a target-specific stochastic barcode). In some embodiments, a universal adaptor primer has a sequence that is different from a universal PCR primer used in the methods of this disclosure.

### Barcodes

Barcoding, such as stochastic barcoding, has been described in, for example, Fu et al., Proc Natl Acad Sci US.A., 2011 May 31,108(22):9026-31; U.S. Patent Application Publication No. US2011/0160078: Fan et al., Science, 2015 February 6, 347(6222):1258367; US Patent Application Publication No. US2015/0299784; and PCT Application Publication No. WO2015/031691: the content of each of these, including any supporting or supplemental information or material, is incorporated herein by reference in its entirety. In some embodiments, the barcode disclosed herein can be a stochastic barcode which can be a polynucleotide sequence that may be used to stochastically label (e.g., barcode, tag) a target. Barcodes can be referred to stochastic barcodes if the ratio of the number of different barcode sequences of the stochastic barcodes and the number of occurrence of any of the targets to be labeled can be, or be about, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 30:1, 40:1, 50:1, 60:1, 70:1, 80:1, 90:1, 100:1, or a number or a range between any two of these values. A target can be an mRNA species comprising mRNA molecules with identical or nearly identical sequences. Barcodes can be referred to as stochastic barcodes if the ratio of the number of different barcode sequences of the stochastic barcodes and the number of occurrence of any of the targets to be labeled is at least or is at most, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 30:1, 40:1, 50:1, 60:1, 70:1, 80:1, 90:1, or 100:1. Barcode sequences of stochastic barcodes can be referred to as molecular labels.

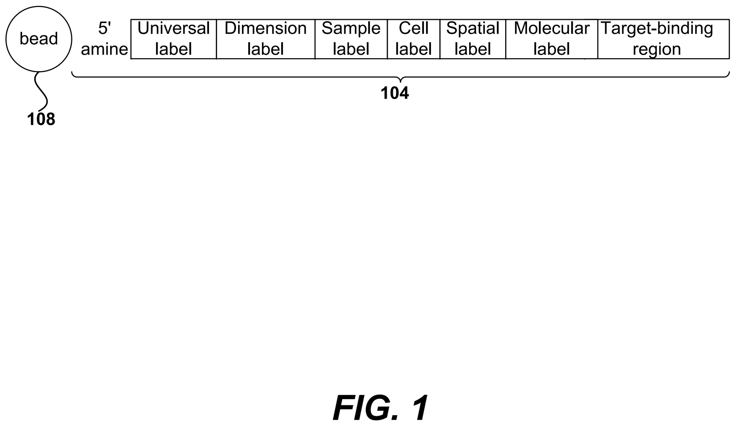

A barcode, for example a stochastic barcode, can comprise one or more labels. Exemplary labels can include a universal label, a cell label, a barcode sequence (e.g., a molecular label), a sample label, a plate label, a spatial label, and/or a pre-spatial label. FIG. 1 illustrates an exemplary barcode 104 with a spatial label. The barcode 104 can comprise a 5'amine that may link the barcode to a solid support 105. The barcode can comprise a universal label, a dimension label, a spatial label, a cell label, and/or a molecular label. The order of different labels (including but not limited to the universal label, the dimension label, the spatial label, the cell label, and the molecule label) in the barcode can vary. For example, as shown in FIG. 1, the universal label may be the 5'-most label, and the molecular label may be the 3'-most label. The spatial label, dimension label, and the cell label may be in any order. In some embodiments, the universal label, the spatial label, the dimension label, the cell label, and the molecular label are in any order. The barcode can comprise a target-binding region. The target-binding region can interact with a target (e.g., target nucleic acid, RNA, mRNA, DNA) in a sample. For example, a target-binding region can comprise an oligo(dT) sequence which can interact with poly(A) tails of mRNAs. In some instances, the labels of the barcode (e.g., universal label, dimension label, spatial label, cell label, and barcode sequence) may be separated by 1, 2, 3, 4, 5, 6, 7, 8, 9, 10, 11, 12, 13, 14, 15, 16, 17, 18, 19, or 20 or more nucleotides.

A label, for example the cell label, can comprise a unique set of nucleic acid sub-sequences of defined length, e.g., seven nucleotides each (equivalent to the number of bits used in some Hamming error correction codes), which can be designed to provide error correction capability. The set of error correction sub-sequences comprise seven nucleotide sequences can be designed such that any pairwise combination of sequences in the set exhibits a defined "genetic distance" (or number of mismatched bases), for example, a set of error correction sub-sequences can be designed to exhibit a genetic distance of three nucleotides. In this case, review of the error correction sequences in the set of sequence data for labeled target nucleic acid molecules (described more fully below) can allow one to detect or correct amplification or sequencing errors. In some embodiments, the length of the nucleic acid sub-sequences used for creating error correction codes can vary, for example, they can be, or be about 1, 2, 3, 4, 5, 6, 7, 8, 9, 10, 15, 20, 30, 31, 40, 50, or a number or a range between any two of these values, nucleotides in length. In some embodiments, nucleic acid sub-sequences of other lengths can be used for creating error correction codes.

The barcode can comprise a target-binding region. The target-binding region can interact with a target in a sample. The target can be, or comprise, ribonucleic acids (RNAs), messenger RNAs (mRNAs), microRNAs, small interfering RNAs (siRNAs), RNA degradation products, RNAs each comprising a poly(A) tail, or any combination thereof. In some embodiments, the plurality of targets can include deoxyribonucleic acids (DNAs).

In some embodiments, a target-binding region can comprise an oligo(dT) sequence which can interact with poly(A) tails of mRNAs. One or more of the labels of the barcode (e.g., the universal label, the dimension label, the spatial label, the cell label, and the barcode sequences (e.g., molecular label)) can be separated by a spacer from another one or two of the remaining labels of the barcode. The spacer can be, for example, 1, 2, 3, 4, 5, 6, 7, 8, 9, 10, 11, 12, 13, 14, 15, 16, 17, 18, 19, or 20, or more nucleotides. In some embodiments, none of the labels of the barcode is separated by spacer.

### Universal Labels

A barcode can comprise one or more universal labels. In some embodiments, the one or more universal labels can be the same for all barcodes in the set of barcodes attached to a given solid support. In some embodiments, the one or more universal labels can be the same for all barcodes attached to a plurality of beads. In some embodiments, a universal label can comprise a nucleic acid sequence that is capable of hybridizing to a sequencing primer. Sequencing primers can be used for sequencing barcodes comprising a universal label. Sequencing primers (e.g., universal sequencing primers) can comprise sequencing primers associated with high-throughput sequencing platforms. In some embodiments, a universal label can comprise a nucleic acid sequence that is capable of hybridizing to a PCR primer. In some embodiments, the universal label can comprise a nucleic acid sequence that is capable of hybridizing to a sequencing primer and a PCR primer. The nucleic acid sequence of the universal label that is capable of hybridizing to a sequencing or PCR primer can be referred to as a primer binding site. A universal label can comprise a sequence that can be used to initiate transcription of the barcode. A universal label can comprise a sequence that can be used for extension of the barcode or a region within the barcode. A universal label can be, or be about, 1, 2, 3, 4, 5, 10, 15, 20, 25, 30, 35, 40, 45, 50, or a number or a range between any two of these values, nucleotides in length. For example, a universal label can comprise at least about 10 nucleotides. A universal label can be at least, or be at most, 1, 2, 3, 4, 5, 10, 15, 20, 25, 30, 35, 40, 45, 50, 100, 200, or 300 nucleotides in length. In some embodiments, a cleavable linker or modified nucleotide can be part of the universal label sequence to enable the barcode to be cleaved off from the support.

### Dimension Labels

A barcode can comprise one or more dimension labels. In some embodiments, a dimension label can comprise a nucleic acid sequence that provides information about a dimension in which the labeling (e.g., stochastic labeling) occurred. For example, a dimension label can provide information about the time at which a target was barcoded. A dimension label can be associated with a time of barcoding (e.g., stochastic barcoding) in a sample. A dimension label can be activated at the time of labeling. Different dimension labels can be activated at different times. The dimension label provides information about the order in which targets, groups of targets, and/or samples were barcoded. For example, a population of cells can be barcoded at the G0 phase of the cell cycle. The cells can be pulsed again with barcodes (e.g., stochastic barcodes) at the G1 phase of the cell cycle. The cells can be pulsed again with barcodes at the S phase of the cell cycle, and so on. Barcodes at each pulse (e.g., each phase of the cell cycle), can comprise different dimension labels. In this way, the dimension label provides information about which targets were labelled at which phase of the cell cycle. Dimension labels can interrogate many different biological times. Exemplary biological times can include, but are not limited to, the cell cycle, transcription (e.g., transcription initiation), and transcript degradation. In another example, a sample (e.g., a cell, a population of cells) can be labeled before and/or after treatment with a drug and/or therapy. The changes in the number of copies of distinct targets can be indicative of the sample's response to the drug and/or therapy.

A dimension label can be activatable. An activatable dimension label can be activated at a specific time point. The activatable label can be, for example, constitutively activated (e.g., not turned off). The activatable dimension label can be, for example, reversibly activated (e.g., the activatable dimension label can be turned on and turned off). The dimension label can be, for example, reversibly activatable at least 1, 2, 3, 4, 5, 6, 7, 8, 9, 10, or more times. The dimension label can be reversibly activatable, for example, at least 1, 2, 3, 4, 5, 6, 7, 8, 9,, 10 or more times. In some embodiments, the dimension label can be activated with fluorescence, light, a chemical event (e.g., cleavage, ligation of another molecule, addition of modifications (e.g., pegylated, sumoylated, acetylated, methylated, deacetylated, demethylated), a photochemical event (e.g., photocaging), and introduction of a non-natural nucleotide.

The dimension label can, in some embodiments, be identical for all barcodes (e.g., stochastic barcodes) attached to a given solid support (e.g., a bead), but different for different solid supports (e.g., beads). In some embodiments, at least 60%, 70%, 80%, 85%, 90%, 95%, 97%, 99% or 100%, of barcodes on the same solid support can comprise the same dimension label. In some embodiments, at least 60% of barcodes on the same solid support can comprise the same dimension label. In some embodiments, at least 95% of barcodes on the same solid support can comprise the same dimension label.

There can be as many as 10⁶ or more unique dimension label sequences represented in a plurality of solid supports (e.g., beads). A dimension label can be, or be about 1, 2, 3, 4, 5, 10, 15, 20, 25, 30, 35, 40, 45, 50, or a number or a range between any two of these values, nucleotides in length. A dimension label can be at least or be at most, 1, 2, 3, 4, 5, 10, 15, 20, 25, 30, 35, 40, 45, 50, 100, 200, or 300, nucleotides in length. A dimension label can comprise between about 5 to about 200 nucleotides. A dimension label can comprise between about 10 to about 150 nucleotides. A dimension label can comprise between about 20 to about 125 nucleotides in length.

### Spatial Labels

A barcode can comprise one or more spatial labels. In some embodiments, a spatial label can comprise a nucleic acid sequence that provides information about the spatial orientation of a target molecule which is associated with the barcode. A spatial label can be associated with a coordinate in a sample. The coordinate can be a fixed coordinate. For example, a coordinate can be fixed in reference to a substrate. A spatial label can be in reference to a two or three-dimensional grid. A coordinate can be fixed in reference to a landmark. The landmark can be identifiable in space. A landmark can be a structure which can be imaged. A landmark can be a biological structure, for example an anatomical landmark. A landmark can be a cellular landmark, for instance an organelle. A landmark can be a non-natural landmark such as a structure with an identifiable identifier such as a color code, bar code, magnetic property, fluorescents, radioactivity, or a unique size or shape. A spatial label can be associated with a physical partition (e.g., A well, a container, or a droplet). In some embodiments, multiple spatial labels are used together to encode one or more positions in space.

The spatial label can be identical for all barcodes attached to a given solid support (e.g., a bead), but different for different solid supports (e.g., beads). In some embodiments, the percentage of barcodes on the same solid support comprising the same spatial label can be, or be about, 60%, 70%, 80%, 85%, 90%, 95%, 97%, 99%, 100%, or a number or a range between any two of these values. In some embodiments, the percentage of barcodes on the same solid support comprising the same spatial label can be at least, or be at most, 60%, 70%, 80%, 85%, 90%, 95%, 97%, 99%, or 100%. In some embodiments, at least 60% of barcodes on the same solid support can comprise the same spatial label. In some embodiments, at least 95% of barcodes on the same solid support can comprise the same spatial label.

There can be as many as 10⁶ or more unique spatial label sequences represented in a plurality of solid supports (e.g., beads). A spatial label can be, or be about, 1, 2, 3, 4, 5, 10, 15, 20, 25, 30, 35, 40, 45, 50, or a number or a range between any two of these values, nucleotides in length. A spatial label can be at least or at most 1, 2, 3, 4, 5, 10, 15, 20, 25, 30, 35, 40, 45, 50, 100, 200, or 300 nucleotides in length. A spatial label can comprise between about 5 to about 200 nucleotides. A spatial label can comprise between about 10 to about 150 nucleotides. A spatial label can comprise between about 20 to about 125 nucleotides in length.

### Cell labels

A barcode (e.g., a stochastic barcode) can comprise one or more cell labels. In some embodiments, a cell label can comprise a nucleic acid sequence that provides information for determining which target nucleic acid originated from which cell. In some embodiments, the cell label is identical for all barcodes attached to a given solid support (e.g., a bead), but different for different solid supports (e.g., beads). In some embodiments, the percentage of barcodes on the same solid support comprising the same cell label can be, or be about 60%, 70%, 80%, 85%, 90%, 95%, 97%, 99%, 100%, or a number or a range between any two of these values. In some embodiments, the percentage of barcodes on the same solid support comprising the same cell label can be, or be about 60%, 70%, 80%, 85%, 90%, 95%, 97%, 99%, or 100%. For example, at least 60% of barcodes on the same solid support can comprise the same cell label. As another example, at least 95% of barcodes on the same solid support can comprise the same cell label.

There can be as many as 10⁶ or more unique cell label sequences represented in a plurality of solid supports (e.g., beads). A cell label can be, or be about, 1, 2, 3, 4, 5, 10, 15, 20, 25, 30, 35, 40, 45, 50, or a number or a range between any two of these values, nucleotides in length. A cell label can be at least, or be at most, 1, 2, 3, 4, 5, 10, 15, 20, 25, 30, 35, 40, 45, 50, 100, 200, or 300 nucleotides in length. For example, a cell label can comprise between about 5 to about 200 nucleotides. As another example, a cell label can comprise between about 10 to about 150 nucleotides. As yet another example, a cell label can comprise between about 20 to about 125 nucleotides in length.

### Barcode Sequences

A barcode can comprise one or more barcode sequences. In some embodiments, a barcode sequence can comprise a nucleic acid sequence that provides identifying information for the specific type of target nucleic acid species hybridized to the barcode. A barcode sequence can comprise a nucleic acid sequence that provides a counter (e.g., that provides a rough approximation) for the specific occurrence of the target nucleic acid species hybridized to the barcode (e.g., target-binding region).

In some embodiments, a diverse set of barcode sequences are attached to a given solid support (e.g., a bead). In some embodiments, there can be, or be about, 10², 10³, 10⁴, 10⁵, 10⁶, 10⁷, 10⁸, 10⁹, or a number or a range between any two of these values, unique molecular label sequences. For example, a plurality of barcodes can comprise about 6561 barcodes sequences with distinct sequences. As another example, a plurality of barcodes can comprise about 65536 barcode sequences with distinct sequences. In some embodiments, there can be at least, or be at most, 10², 10³, 10⁴, 10⁵, 10⁶, 10⁷, 10⁸, or 10⁹, unique barcode sequences. The unique molecular label sequences can be attached to a given solid support (e.g., a bead). In some embodiments, the unique molecular label sequence is partially or entirely encompassed by a particle (e.g., a hydrogel bead).

The length of a barcode can be different in different implementations. For example, a barcode can be, or be about, 1, 2, 3, 4, 5, 10, 15, 20, 25, 30, 35, 40, 45, 50, or a number or a range between any two of these values, nucleotides in length. As another example, a barcode can be at least, or be at most, 1, 2, 3, 4, 5, 10, 15, 20, 25, 30, 35, 40, 45, 50, 100, 200, or 300 nucleotides in length.

### Molecular Labels

A barcode (e.g., a stochastic barcode) can comprise one or more molecular labels. Molecular labels can include barcode sequences. In some embodiments, a molecular label can comprise a nucleic acid sequence that provides identifying information for the specific type of target nucleic acid species hybridized to the barcode. A molecular label can comprise a nucleic acid sequence that provides a counter for the specific occurrence of the target nucleic acid species hybridized to the barcode (e.g., target-binding region).

In some embodiments, a diverse set of molecular labels are attached to a given solid support (e.g., a bead). In some embodiments, there can be, or be about, 10², 10³, 10⁴, 10⁵, 10⁶, 10⁷, 10⁸, 10⁹, or a number or a range between any two of these values, of unique molecular label sequences. For example, a plurality of barcodes can comprise about 6561 molecular labels with distinct sequences. As another example, a plurality of barcodes can comprise about 65536 molecular labels with distinct sequences. In some embodiments, there can be at least, or be at most, 10², 10³, 10⁴, 10⁵, 10⁶, 10⁷, 10⁸, or 10⁹, unique molecular label sequences. Barcodes with unique molecular label sequences can be attached to a given solid support (e.g., a bead).

For barcoding (e.g., stochastic barcoding) using a plurality of stochastic barcodes, the ratio of the number of different molecular label sequences and the number of occurrence of any of the targets can be, or be about 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 30:1, 40:1, 50:1, 60:1, 70:1, 80:1, 90:1, 100:1, or a number or a range between any two of these values. A target can be an mRNA species comprising mRNA molecules with identical or nearly identical sequences. In some embodiments, the ratio of the number of different molecular label sequences and the number of occurrence of any of the targets is at least, or is at most, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 30:1, 40:1, 50:1, 60:1, 70:1, 80:1, 90:1, or 100:1.

A molecular label can be, or be about 1, 2, 3, 4, 5, 10, 15, 20, 25, 30, 35, 40, 45, 50, or a number or a range between any two of these values, nucleotides in length. A molecular label can be at least, or be at most, 1, 2, 3, 4, 5, 10, 15, 20, 25, 30, 35, 40, 45, 50, 100, 200, or 300 nucleotides in length.

### Target-Binding Region

A barcode can comprise one or more target binding regions, such as capture probes. In some embodiments, a target-binding region can hybridize with a target of interest. In some embodiments, the target binding regions can comprise a nucleic acid sequence that hybridizes specifically to a target (e.g., target nucleic acid, target molecule, e.g., a cellular nucleic acid to be analyzed), for example to a specific gene sequence. In some embodiments, a target binding region can comprise a nucleic acid sequence that can attach (e.g., hybridize) to a specific location of a specific target nucleic acid. In some embodiments, the target binding region can comprise a nucleic acid sequence that is capable of specific hybridization to a restriction enzyme site overhang (e.g., an EcoRI sticky-end overhang). The barcode can then ligate to any nucleic acid molecule comprising a sequence complementary to the restriction site overhang.

In some embodiments, a target binding region can comprise a non-specific target nucleic acid sequence. A non-specific target nucleic acid sequence can refer to a sequence that can bind to multiple target nucleic acids, independent of the specific sequence of the target nucleic acid. For example, target binding region can comprise a random multimer sequence, a poly(dA) sequence, a poly(dT) sequence, a poly(dG) sequence, a poly(dC) sequence, or a combination thereof. For example, the target binding region can be an oligo(dT) sequence that hybridizes to the poly(A) tail on mRNA molecules. A random multimer sequence can be, for example, a random dimer, trimer, quatramer, pentamer, hexamer, septamer, octamer, nonamer, decamer, or higher multimer sequence of any length. In some embodiments, the target binding region is the same for all barcodes attached to a given bead. In some embodiments, the target binding regions for the plurality of barcodes attached to a given bead can comprise two or more different target binding sequences. A target binding region can be, or be about 5, 10, 15, 20, 25, 30, 35, 40, 45, 50, or a number or a range between any two of these values, nucleotides in length. A target binding region can be at most about 5, 10, 15, 20, 25, 30, 35, 40, 45, 50 or more nucleotides in length. For example, an mRNA molecule can be reverse transcribed using a reverse transcriptase, such as Moloney murine leukemia virus (MMLV) reverse transcriptase, to generate a cDNA molecule with a poly(dC) tail. A barcode can include a target binding region with a poly(dG) tail. Upon base pairing between the poly(dG) tail of the barcode and the poly(dC) tail of the cDNA molecule, the reverse transcriptase switches template strands, from cellular RNA molecule to the barcode, and continues replication to the 5' end of the barcode. By doing so, the resulting cDNA molecule contains the sequence of the barcode (such as the molecular label) on the 3' end of the cDNA molecule.

In some embodiments, a target-binding region can comprise an oligo(dT) which can hybridize with mRNAs comprising polyadenylated ends. A target-binding region can be gene-specific. For example, a target-binding region can be configured to hybridize to a specific region of a target. A target-binding region can be, or be about, 1, 2, 3, 4, 5, 6, 7, 8, 9, 10, 11, 12, 13, 14, 15, 16, 17, 18, 19, 20, 21, 22, 23, 24, 25, 26 27, 28, 29, 30, or a number or a range between any two of these values, nucleotides in length. A target-binding region can be at least, or be at most, 1, 2, 3, 4, 5, 6, 7, 8, 9, 10, 11, 12, 13, 14, 15, 16, 17, 18, 19, 20, 21, 22, 23, 24, 25, 26 27, 28, 29, or 30, nucleotides in length. A target-binding region can be about 5-30 nucleotides in length. When a barcode comprises a gene-specific target-binding region, the barcode can be referred to herein as a gene-specific barcode.

### Orientation Property

A stochastic barcode (e.g., a stochastic barcode) can comprise one or more orientation properties which can be used to orient (e.g., align) the barcodes. A barcode can comprise a moiety for isoelectric focusing. Different barcodes can comprise different isoelectric focusing points. When these barcodes are introduced to a sample, the sample can undergo isoelectric focusing in order to orient the barcodes into a known way. In this way, the orientation property can be used to develop a known map of barcodes in a sample. Exemplary orientation properties can include, electrophoretic mobility (e.g., based on size of the barcode), isoelectric point, spin, conductivity, and/or self-assembly. For example, barcodes with an orientation property of self-assembly, can self-assemble into a specific orientation (e.g., nucleic acid nanostructure) upon activation.

### Affinity Property

A barcode (e.g., a stochastic barcode) can comprise one or more affinity properties. For example, a spatial label can comprise an affinity property. An affinity property can include a chemical and/or biological moiety that can facilitate binding of the barcode to another entity (e.g., cell receptor). For example, an affinity property can comprise an antibody, for example, an antibody specific for a specific moiety (e.g., receptor) on a sample. In some embodiments, the antibody can guide the barcode to a specific cell type or molecule. Targets at and/or near the specific cell type or molecule can be labeled (e.g., stochastically labeled). The affinity property can, in some embodiments, provide spatial information in addition to the nucleotide sequence of the spatial label because the antibody can guide the barcode to a specific location. The antibody can be a therapeutic antibody, for example a monoclonal antibody or a polyclonal antibody. The antibody can be humanized or chimeric. The antibody can be a naked antibody or a fusion antibody.

The antibody can be a full-length (i.e., naturally occurring or formed by normal immunoglobulin gene fragment recombinatorial processes) immunoglobulin molecule (e.g., an IgG antibody) or an immunologically active (i.e., specifically binding) portion of an immunoglobulin molecule, like an antibody fragment.

The antibody fragment can be, for example, a portion of an antibody such as F(ab')2, Fab', Fab, Fv, sFv and the like. In some embodiments, the antibody fragment can bind with the same antigen that is recognized by the full-length antibody. The antibody fragment can include isolated fragments consisting of the variable regions of antibodies, such as the "Fv" fragments consisting of the variable regions of the heavy and light chains and recombinant single chain polypeptide molecules in which light and heavy variable regions are connected by a peptide linker ("scFv proteins"). Exemplary antibodies can include, but are not limited to, antibodies for cancer cells, antibodies for viruses, antibodies that bind to cell surface receptors (CD8, CD34, CD45), and therapeutic antibodies.

### Universal Adaptor Primer

A barcode can comprise one or more universal adaptor primers. For example, a gene-specific barcode, such as a gene-specific stochastic barcode, can comprise a universal adaptor primer. A universal adaptor primer can refer to a nucleotide sequence that is universal across all barcodes. A universal adaptor primer can be used for building gene-specific barcodes. A universal adaptor primer can be, or be about, 1, 2, 3, 4, 5, 6, 7, 8, 9, 10, 11, 12, 13, 14, 15, 16, 17, 18, 19, 20, 21, 22, 23, 24, 25, 26 27, 28, 29, 30, or a number or a range between any two of these nucleotides in length. A universal adaptor primer can be at least, or be at most, 1, 2, 3, 4, 5, 6, 7, 8, 9, 10, 11, 12, 13, 14, 15, 16, 17, 18, 19, 20, 21, 22, 23, 24, 25, 26 27, 28, 29, or 30 nucleotides in length. A universal adaptor primer can be from 5-30 nucleotides in length.

### Linker

When a barcode comprises more than one of a type of label (e.g., more than one cell label or more than one barcode sequence, such as one molecular label), the labels may be interspersed with a linker label sequence. A linker label sequence can be at least about 5, 10, 15, 20, 25, 30, 35, 40, 45, 50 or more nucleotides in length. A linker label sequence can be at most about 5, 10, 15, 20, 25, 30, 35, 40, 45, 50 or more nucleotides in length. In some instances, a linker label sequence is 12 nucleotides in length. A linker label sequence can be used to facilitate the synthesis of the barcode. The linker label can comprise an error-correcting (e.g., Hamming) code.

### Solid Supports

Barcodes, such as stochastic barcodes, disclosed herein can, in some embodiments, be associated with a solid support. The solid support can be, for example, a synthetic particle. In some embodiments, some or all of the barcode sequences, such as molecular labels for stochastic barcodes (e.g., the first barcode sequences) of a plurality of barcodes (e.g., the first plurality of barcodes) on a solid support differ by at least one nucleotide. The cell labels of the barcodes on the same solid support can be the same. The cell labels of the barcodes on different solid supports can differ by at least one nucleotide. For example, first cell labels of a first plurality of barcodes on a first solid support can have the same sequence, and second cell labels of a second plurality of barcodes on a second solid support can have the same sequence. The first cell labels of the first plurality of barcodes on the first solid support and the second cell labels of the second plurality of barcodes on the second solid support can differ by at least one nucleotide. A cell label can be, for example, about 5-20 nucleotides long. A barcode sequence can be, for example, about 5-20 nucleotides long. The synthetic particle can be, for example, a bead.

The bead can be, for example, a silica gel bead, a controlled pore glass bead, a magnetic bead, a Dynabead, a Sephadex/Sepharose bead, a cellulose bead, a polystyrene bead, or any combination thereof. The bead can comprise a material such as polydimethylsiloxane (PDMS), polystyrene, glass, polypropylene, agarose, gelatin, hydrogel, paramagnetic, ceramic, plastic, glass, methylstyrene, acrylic polymer, titanium, latex, Sepharose, cellulose, nylon, silicone, or any combination thereof.

In some embodiments, the bead can be a polymeric bead, for example a deformable bead or a gel bead, functionalized with barcodes or stochastic barcodes (such as gel beads from 10X Genomics (San Francisco, CA). In some implementation, a gel bead can comprise a polymer based gels. Gel beads can be generated, for example, by encapsulating one or more polymeric precursors into droplets. Upon exposure of the polymeric precursors to an accelerator (e.g., tetramethylethylenediamine (TEMED)), a gel bead may be generated.

In some embodiments, the particle can be disruptable (e.g., dissolvable, degradable). For example, the polymeric bead can dissolve, melt, or degrade, for example, under a desired condition. The desired condition can include an environmental condition. The desired condition may result in the polymeric bead dissolving, melting, or degrading in a controlled manner. A gel bead may dissolve, melt, or degrade due to a chemical stimulus, a physical stimulus, a biological stimulus, a thermal stimulus, a magnetic stimulus, an electric stimulus, a light stimulus, or any combination thereof.

Analytes and/or reagents, such as oligonucleotide barcodes, for example, may be coupled/immobilized to the interior surface of a gel bead (e.g., the interior accessible via diffusion of an oligonucleotide barcode and/or materials used to generate an oligonucleotide barcode) and/or the outer surface of a gel bead or any other microcapsule described herein. Coupling/immobilization may be via any form of chemical bonding (e.g., covalent bond, ionic bond) or physical phenomena (e.g., Van der Waals forces, dipole-dipole interactions, etc.). In some embodiments, coupling/immobilization of a reagent to a gel bead or any other microcapsule described herein may be reversible, such as, for example, via a labile moiety (e.g., via a chemical cross-linker, including chemical cross-linkers described herein). Upon application of a stimulus, the labile moiety may be cleaved and the immobilized reagent set free. In some embodiments, the labile moiety is a disulfide bond. For example, in the case where an oligonucleotide barcode is immobilized to a gel bead via a disulfide bond, exposure of the disulfide bond to a reducing agent can cleave the disulfide bond and free the oligonucleotide barcode from the bead. The labile moiety may be included as part of a gel bead or microcapsule, as part of a chemical linker that links a reagent or analyte to a gel bead or microcapsule, and/or as part of a reagent or analyte. In some embodiments, at least one barcode of the plurality of barcodes can be immobilized on the particle, partially immobilized on the particle, enclosed in the particle, partially enclosed in the particle, or any combination thereof.

In some embodiments, a gel bead can comprise a wide range of different polymers including but not limited to: polymers, heat sensitive polymers, photosensitive polymers, magnetic polymers, pH sensitive polymers, salt-sensitive polymers, chemically sensitive polymers, polyelectrolytes, polysaccharides, peptides, proteins, and/or plastics. Polymers may include but are not limited to materials such as poly(N-isopropylacrylamide) (PNIPAAm), poly(styrene sulfonate) (PSS), poly(allyl amine) (PAAm), poly(acrylic acid) (PAA), poly(ethylene imine) (PEI), poly(diallyldimethyl-ammonium chloride) (PDADMAC), poly(pyrolle) (PPy), poly(vinylpyrrolidone) (PVPON), poly(vinyl pyridine) (PVP), poly(methacrylic acid) (PMAA), poly(methyl methacrylate) (PMMA), polystyrene (PS), poly(tetrahydrofuran) (PTHF), poly(phthaladehyde) (PTHF), poly(hexyl viologen) (PHV), poly(L-lysine) (PLL), poly(L-arginine) (PARG), poly(lactic-co-glycolic acid) (PLGA).

Numerous chemical stimuli can be used to trigger the disruption, dissolution, or degradation of the beads. Examples of these chemical changes may include, but are not limited to pH-mediated changes to the bead wall, disintegration of the bead wall via chemical cleavage of crosslink bonds, triggered depolymerization of the bead wall, and bead wall switching reactions. Bulk changes may also be used to trigger disruption of the beads.

Bulk or physical changes to the microcapsule through various stimuli also offer many advantages in designing capsules to release reagents. Bulk or physical changes occur on a macroscopic scale, in which bead rupture is the result of mechano-physical forces induced by a stimulus. These processes may include, but are not limited to pressure induced rupture, bead wall melting, or changes in the porosity, of the bead wall.

Biological stimuli may also be used to trigger disruption, dissolution, or degradation of beads. Generally, biological triggers resemble chemical triggers, but many examples use biomolecules, or molecules commonly found in living systems such as enzymes, peptides, saccharides, fatty acids, nucleic acids and the like. For example, beads may comprise polymers with peptide cross-links that are sensitive to cleavage by specific proteases. More specifically, one example may comprise a microcapsule comprising GFLGK peptide cross links. Upon addition of a biological trigger such as the protease Cathepsin B, the peptide cross links of the shell well are cleaved and the contents of the beads are released. In other cases, the proteases may be heat-activated. In another example, beads comprise a shell wall comprising cellulose. Addition of the hydrolytic enzyme chitosan serves as biologic trigger for cleavage of cellulosic bonds, depolymerization of the shell wall, and release of its inner contents.

The beads may also be induced to release their contents upon the application of a thermal stimulus. A change in temperature can cause a variety changes to the beads. A change in heat may cause melting of a bead such that the bead wall disintegrates. In other cases, the heat may increase the internal pressure of the inner components of the bead such that the bead ruptures or explodes. In still other cases, the heat may transform the bead into a shrunken dehydrated state. The heat may also act upon heat-sensitive polymers within the wall of a bead to cause disruption of the bead.

Inclusion of magnetic nanoparticles to the bead wall of microcapsules may allow triggered rupture of the beads as well as guide the beads in an array. A device of this disclosure may comprise magnetic beads for either purpose. In one example, incorporation of Fe₃O₄ nanoparticles into polyelectrolyte containing beads triggers rupture in the presence of an oscillating magnetic field stimulus.

A bead may also be disrupted, dissolved, or degraded as the result of electrical stimulation. Similar to magnetic particles described in the previous section, electrically sensitive beads can allow for both triggered rupture of the beads as well as other functions such as alignment in an electric field, electrical conductivity or redox reactions. In one example, beads containing electrically sensitive material are aligned in an electric field such that release of inner reagents can be controlled. In other examples, electrical fields may induce redox reactions within the bead wall itself that may increase porosity.

A light stimulus may also be used to disrupt the beads. Numerous light triggers are possible and may include systems that use various molecules such as nanoparticles and chromophores capable of absorbing photons of specific ranges of wavelengths. For example, metal oxide coatings can be used as capsule triggers. UV irradiation of polyelectrolyte capsules coated with SiO₂ may result in disintegration of the bead wall. In yet another example, photo switchable materials such as azobenzene groups may be incorporated in the bead wall. Upon the application of UV or visible light, chemicals such as these undergo a reversible cis-to-trans isomerization upon absorption of photons. In this aspect, incorporation of photon switches result in a bead wall that may disintegrate or become more porous upon the application of a light trigger.

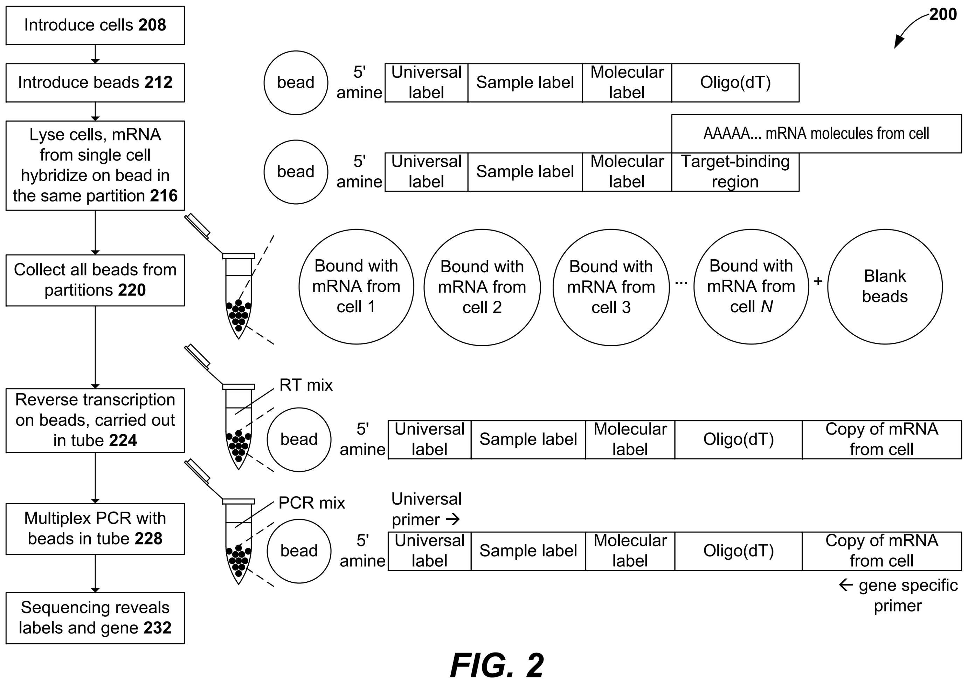

For example, in a non-limiting example of barcoding (e.g., stochastic barcoding) illustrated in FIG. 2, after introducing cells such as single cells onto a plurality of microwells of a microwell array at block 208, beads can be introduced onto the plurality of microwells of the microwell array at block 212. Each microwell can comprise one bead. The beads can comprise a plurality, of barcodes. A barcode can comprise a 5' amine region attached to a bead. The barcode can comprise a universal label, a barcode sequence (e.g., a molecular label), a target-binding region, or any combination thereof.

The barcodes disclosed herein can be associated with (e.g., attached to) a solid support (e.g., a bead). The barcodes associated with a solid support can each comprise a barcode sequence selected from a group comprising at least 100 or 1000 barcode sequences with unique sequences. In some embodiments, different barcodes associated with a solid support can comprise barcode with different sequences. In some embodiments, a percentage of barcodes associated with a solid support comprises the same cell label. For example, the percentage can be, or be about 60%, 70%, 80%, 85%, 90%, 95%, 97%, 99%, 100%, or a number or a range between any two of these values. As another example, the percentage can be at least, or be at most 60%, 70%, 80%, 85%, 90%, 95%, 97%, 99%, or 100%. In some embodiments, barcodes associated with a solid support can have the same cell label. The barcodes associated with different solid supports can have different cell labels selected from a group comprising at least 100 or 1000 cell labels with unique sequences.

The barcodes disclosed herein can be associated to (e.g., attached to) a solid support (e.g., a bead). In some embodiments, barcoding the plurality of targets in the sample can be performed with a solid support including a plurality of synthetic particles associated with the plurality of barcodes. In some embodiments, the solid support can include a plurality of synthetic particles associated with the plurality of barcodes. The spatial labels of the plurality of barcodes on different solid supports can differ by at least one nucleotide. The solid support can, for example, include the plurality of barcodes in two dimensions or three dimensions. The synthetic particles can be beads. The beads can be silica gel beads, controlled pore glass beads, magnetic beads, Dynabeads, Sephadex/Sepharose beads, cellulose beads, polystyrene beads, or any combination thereof. The solid support can include a polymer, a matrix, a hydrogel, a needle array device, an antibody, or any combination thereof. In some embodiments, the solid supports can be free floating. In some embodiments, the solid supports can be embedded in a semi-solid or solid array. The barcodes may not be associated with solid supports. The barcodes can be individual nucleotides. The barcodes can be associated with a substrate.

As used herein, the terms "tethered," "attached," and "immobilized," are used interchangeably, and can refer to covalent or non-covalent means for attaching barcodes to a solid support. Any of a variety of different solid supports can be used as solid supports for attaching pre-synthesized barcodes or for *in situ* solid-phase synthesis of barcode.

In some embodiments, the solid support is a bead. The bead can comprise one or more types of solid, porous, or hollow sphere, ball, bearing, cylinder, or other similar configuration which a nucleic acid can be immobilized (e.g., covalently or non-covalently). The bead can be, for example, composed of plastic, ceramic, metal, polymeric material, or any combination thereof. A bead can be, or comprise, a discrete particle that is spherical (e.g., microspheres) or have a non-spherical or irregular shape, such as cubic, cuboid, pyramidal, cylindrical, conical, oblong, or disc-shaped, and the like. In some embodiments, a bead can be non-spherical in shape.

Beads can comprise a variety of materials including, but not limited to, paramagnetic materials (e.g., magnesium, molybdenum, lithium, and tantalum), superparamagnetic materials (e.g., ferrite (Fe₃O₄; magnetite) nanoparticles), ferromagnetic materials (e.g., iron, nickel, cobalt, some alloys thereof, and some rare earth metal compounds), ceramic, plastic, glass, polystyrene, silica, methylstyrene, acrylic polymers, titanium, latex, Sepharose, agarose, hydrogel, polymer, cellulose, nylon, or any combination thereof.

In some embodiments, the bead (e.g., the bead to which the labels are attached) is a hydrogel bead. In some embodiments, the bead comprises hydrogel.

Some embodiments disclosed herein include one or more particles (for example, beads). Each of the particles can comprise a plurality of oligonucleotides (e.g., barcodes). Each of the plurality of oligonucleotides can comprise a barcode sequence (e.g., a molecular label sequence), a cell label, and a target-binding region (e.g., an oligo(dT) sequence, a gene-specific sequence, a random multimer, or a combination thereof). The cell label sequence of each of the plurality of oligonucleotides can be the same. The cell label sequences of oligonucleotides on different particles can be different such that the oligonucleotides on different particles can be identified. The number of different cell label sequences can be different in different implementations. In some embodiments, the number of cell label sequences can be, or be about 10, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 10⁶, 10⁷, 10⁸, 10⁹, a number or a range between any two of these values, or more. In some embodiments, the number of cell label sequences can be at least, or be at most 10, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 10⁶, 10⁷, 10⁸, or 10⁹. In some embodiments, no more than 1, 2, 3, 4, 5, 6, 7, 8, 9, 10, 20, 30, 40, 50, 60, 70, 80, 90, 100, 200, 300, 400, 500, 600, 700, 800, 900, 1000, or more of the plurality of the particles include oligonucleotides with the same cell sequence. In some embodiment, the plurality of particles that include oligonucleotides with the same cell sequence can be at most 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, or more. In some embodiments, none of the plurality of the particles has the same cell label sequence.

The plurality, of oligonucleotides on each particle can comprise different barcode sequences (e.g., molecular labels). In some embodiments, the number of barcode sequences can be, or be about 10, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 10⁶, 10⁷, 10⁸, 10⁹, or a number or a range between any two of these values. In some embodiments, the number of barcode sequences can be at least, or be at most 10, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 10⁶, 10⁷, 10⁸, or 10⁹. For example, at least 100 of the plurality of oligonucleotides comprise different barcode sequences. As another example, in a single particle, at least 100, 500, 1000, 5000, 10000, 15000, 20000, 50000, a number or a range between any two of these values, or more of the plurality of oligonucleotides comprise different barcode sequences. Some embodiments provide a plurality of the particles comprising barcodes. In some embodiments, the ratio of an occurrence (or a copy or a number) of a target to be labeled and the different barcode sequences can be at least 1:1, 1:2, 1:3, 1:4, 1:5, 1:6, 1:7, 1:8, 1:9, 1:10, 1:11, 1:12, 1:13, 1:14, 1:15, 1:16, 1:17, 1:18, 1:19, 1:20, 1:30, 1:40, 1:50, 1:60, 1:70, 1:80, 1:90, or more. In some embodiments, each of the plurality of oligonucleotides further comprises a sample label, a universal label, or both. The particle can be, for example, a nanoparticle or microparticle.

The size of the beads can vary. For example, the diameter of the bead can range from 0.1 micrometer to 50 micrometer. In some embodiments, the diameter of the bead can be, or be about, 0.1, 0.5, 1, 2, 3, 4, 5, 6, 7, 8, 9, 10, 20, 30, 40, 50 micrometer, or a number or a range between any two of these values.

The diameter of the bead can be related to the diameter of the wells of the substrate. In some embodiments, the diameter of the bead can be, or be about, 10%, 20%, 30%, 40%, 50%, 60%, 70%, 80%, 90%, 100%, or a number or a range between any two of these values, longer or shorter than the diameter of the well. The diameter of the beads can be related to the diameter of a cell (e.g., a single cell entrapped by a well of the substrate). In some embodiments, the diameter of the bead can be at least, or be at most, 10%, 20%, 30%, 40%, 50%, 60%, 70%, 80%, 90%, or 100% longer or shorter than the diameter of the well. The diameter of the beads can be related to the diameter of a cell (e.g., a single cell entrapped by a well of the substrate). In some embodiments, the diameter of the bead can be, or be about, 10%, 20%, 30%, 40%, 50%, 60%, 70%, 80%, 90%, 100%, 150%, 200%, 250%, 300%, or a number or a range between any two of these values, longer or shorter than the diameter of the cell. In some embodiments, the diameter of the beads can be at least, or be at most, 10%, 20%, 30%, 40%, 50%, 60%, 70%, 80%, 90%, 100%, 150%, 200%, 250%, or 300% longer or shorter than the diameter of the cell.

A bead can be attached to and/or embedded in a substrate. A bead can be attached to and/or embedded in a gel, hydrogel, polymer and/or matrix. The spatial position of a bead within a substrate (e.g., gel, matrix, scaffold, or polymer) can be identified using the spatial label present on the barcode on the bead which can serve as a location address.

Examples of beads can include, but are not limited to, streptavidin beads, agarose beads, magnetic beads, Dynabeads^{®}, MACS^{®} microbeads, antibody conjugated beads (e.g., anti-immunoglobulin microbeads), protein A conjugated beads, protein G conjugated beads, protein A/G conjugated beads, protein L conjugated beads, oligo(dT) conjugated beads, silica beads, silica-like beads, anti-biotin microbeads, anti-fluorochrome microbeads, and BcMag^{™} Carboxyl-Terminated Magnetic Beads.

A bead can be associated with (e.g., impregnated with) quantum dots or fluorescent dyes to make it fluorescent in one fluorescence optical channel or multiple optical channels. A bead can be associated with iron oxide or chromium oxide to make it paramagnetic or ferromagnetic. Beads can be identifiable. For example, a bead can be imaged using a camera. A bead can have a detectable code associated with the bead. For example, a bead can comprise a barcode. A bead can change size, for example, due to swelling in an organic or inorganic solution. A bead can be hydrophobic. A bead can be hydrophilic. A bead can be biocompatible.

A solid support (e.g., a bead) can be visualized. The solid support can comprise a visualizing tag (e.g., fluorescent dye). A solid support (e.g., a bead) can be etched with an identifier (e.g., a number). The identifier can be visualized through imaging the beads.

A solid support can comprise an insoluble, semi-soluble, or insoluble material. A solid support can be referred to as "functionalized" when it includes a linker, a scaffold, a building block, or other reactive moiety attached thereto, whereas a solid support may be "nonfunctionalized" when it lack such a reactive moiety attached thereto. The solid support can be employed free in solution, such as in a microtiter well format; in a flow-through format, such as in a column; or in a dipstick.

The solid support can comprise a membrane, paper, plastic, coated surface, flat surface, glass, slide, chip, or any combination thereof. A solid support can take the form of resins, gels, microspheres, or other geometric configurations. A solid support can comprise silica chips, microparticles, nanoparticles, plates, arrays, capillaries, flat supports such as glass fiber filters, glass surfaces, metal surfaces (steel, gold silver, aluminum, silicon and copper), glass supports, plastic supports, silicon supports, chips, filters, membranes, microwell plates, slides, plastic materials including multiwell plates or membranes (e.g., formed of polyethylene, polypropylene, polyamide, polyvinylidenedifluoride), and/or wafers, combs, pins or needles (e.g., arrays of pins suitable for combinatorial synthesis or analysis) or beads in an array of pits or nanoliter wells of flat surfaces such as wafers (e.g., silicon wafers), wafers with pits with or without filter bottoms.

The solid support can comprise a polymer matrix (e.g., gel, hydrogel). The polymer matrix may be able to permeate intracellular space (e.g., around organelles). The polymer matrix may able to be pumped throughout the circulatory system.

### Substrates and Microwell Array

As used herein, a substrate can refer to a type of solid support. A substrate can refer to a solid support that can comprise barcodes or stochastic barcodes of the disclosure. A substrate can, for example, comprise a plurality of microwells. For example, a substrate can be a well array comprising two or more microwells. In some embodiments, a microwell can comprise a small reaction chamber of defined volume. In some embodiments, a microwell can entrap one or more cells. In some embodiments, a microwell can entrap only one cell. In some embodiments, a microwell can entrap one or more solid supports. In some embodiments, a microwell can entrap only one solid support. In some embodiments, a microwell entraps a single cell and a single solid support (e.g., a bead). A microwell can comprise barcode reagents of the disclosure.

### Methods of Barcoding

The disclosure provides for methods for estimating the number of distinct targets at distinct locations in a physical sample (e.g., tissue, organ, tumor, cell). The methods can comprise placing barcodes (e.g., stochastic barcodes) in close proximity with the sample, lysing the sample, associating distinct targets with the barcodes, amplifying the targets and/or digitally counting the targets. The method can further comprise analyzing and/or visualizing the information obtained from the spatial labels on the barcodes. In some embodiments, a method comprises visualizing the plurality of targets in the sample. Mapping the plurality of targets onto the map of the sample can include generating a two dimensional map or a three dimensional map of the sample. The two dimensional map and the three dimensional map can be generated prior to or after barcoding (e.g., stochastically barcoding) the plurality of targets in the sample. Visualizing the plurality of targets in the sample can include mapping the plurality of targets onto a map of the sample. Mapping the plurality of targets onto the map of the sample can include generating a two dimensional map or a three dimensional map of the sample. The two dimensional map and the three dimensional map can be generated prior to or after barcoding the plurality of targets in the sample. in some embodiments, the two dimensional map and the three dimensional map can be generated before or after lysing the sample. Lysing the sample before or after generating the two dimensional map or the three dimensional map can include heating the sample, contacting the sample with a detergent, changing the pH of the sample, or any combination thereof.

In some embodiments, barcoding the plurality of targets comprises hybridizing a plurality, of barcodes with a plurality of targets to create barcoded targets (e.g., stochastically barcoded targets). Barcoding the plurality of targets can comprise generating an indexed library of the barcoded targets. Generating an indexed library of the barcoded targets can be performed with a solid support comprising the plurality of barcodes (e.g., stochastic barcodes).

### Contacting a Sample and a Barcode

The disclosure provides for methods for contacting a sample (e.g., cells) to a substrate of the disclosure. A sample comprising, for example, a cell, organ, or tissue thin section, can be contacted to barcodes (e.g., stochastic barcodes). The cells can be contacted, for example, by gravity flow wherein the cells can settle and create a monolayer. The sample can be a tissue thin section. The thin section can be placed on the substrate. The sample can be one-dimensional (e.g., formsa planar surface). The sample (e.g., cells) can be spread across the substrate, for example, by growing/culturing the cells on the substrate.

When barcodes are in close proximity, to targets, the targets can hybridize to the barcode. The barcodes can be contacted at a non-depletable ratio such that each distinct target can associate with a distinct barcode of the disclosure. To ensure efficient association between the target and the barcode, the targets can be cross-linked to barcode.

### Cell Lysis

Following the distribution of cells and barcodes, the cells can be lysed to liberate the target molecules. Cell lysis can be accomplished by any of a variety of means, for example, by chemical or biochemical means, by osmotic shock, or by means of thermal lysis, mechanical lysis, or optical lysis. Cells can be lysed by addition of a cell lysis buffer comprising a detergent (e.g., SDS, Li dodecyl sulfate, Triton X-100, Tween-20, or NP-40), an organic solvent (e.g., methanol or acetone), or digestive enzymes (e.g., proteinase K, pepsin, or trypsin), or any combination thereof. To increase the association of a target and a barcode, the rate of the diffusion of the target molecules can be altered by for example, reducing the temperature and/or increasing the viscosity of the lysate.

In some embodiments, the sample can be lysed using a filter paper. The filter paper can be soaked with a lysis buffer on top of the filter paper. The filter paper can be applied to the sample with pressure which can facilitate lysis of the sample and hybridization of the targets of the sample to the substrate.

In some embodiments, lysis can be performed by mechanical lysis, heat lysis, optical lysis, and/or chemical lysis. Chemical lysis can include the use of digestive enzymes such as proteinase K, pepsin, and trypsin. Lysis can be performed by the addition of a lysis buffer to the substrate. A lysis buffer can comprise Tris HCl. A lysis buffer can comprise at least about 0.01, 0.05, 0.1, 0.5, or 1 M or more Tris HCl. A lysis buffer can comprise at most about 0.01, 0.05, 0.1, 0.5, or 1 M or more Tris HCL. A lysis buffer can comprise about 0.1 M Tris HCl. The pH of the lysis buffer can be at least about 1, 2, 3, 4, 5, 6, 7, 8, 9, 10, or more. The pH of the lysis buffer can be at most about 1, 2, 3, 4, 5, 6, 7, 8, 9,10, or more. In some embodiments, the pH of the lysis buffer is about 7.5. The lysis buffer can comprise a salt (e.g., LiCl). The concentration of salt in the lysis buffer can be at least about 0.1, 0.5, or 1 M or more. The concentration of salt in the lysis buffer can be at most about 0.1, 0.5, or 1 M or more. In some embodiments, the concentration of salt in the lysis buffer is about 0.5M. The lysis buffer can comprise a detergent (e.g., SDS, Li dodecyl sulfate, triton X, tween, NP-40). The concentration of the detergent in the lysis buffer can be at least about 0.0001%, 0.0005%, 0.001%, 0.005%, 0.01%, 0.05%, 0.1%, 0.5%, 1%, 2%, 3%, 4%, 5%, 6%, or 7%, or more. The concentration of the detergent in the lysis buffer can be at most about 0.0001%, 0.0005%, 0.001%, 0.005%, 0.01%, 0.05%, 0.1%, 0.5%, 1%, 2%, 3%, 4%, 5%, 6%, or 7%, or more. In some embodiments, the concentration of the detergent in the lysis buffer is about 1% Li dodecyl sulfate. The time used in the method for lysis can be dependent on the amount of detergent used. In some embodiments, the more detergent used, the less time needed for lysis. The lysis buffer can comprise a chelating agent (e.g., EDTA, EGTA). The concentration of a chelating agent in the lysis buffer can be at least about 1, 5, 10, 15, 20, 25, or 30 mM or more. The concentration of a chelating agent in the lysis buffer can be at most about 1, 5, 10, 15, 20, 25, or 30mM or more. In some embodiments, the concentration of chelating agent in the lysis buffer is about 10 mM. The lysis buffer can comprise a reducing reagent (e.g., beta-mercaptoethanol, DTT). The concentration of the reducing reagent in the lysis buffer can be at least about 1, 5, 10, 15, or 20 mM or more. The concentration of the reducing reagent in the lysis buffer can be at most about 1, 5, 10, 15, or 20 mM or more. In some embodiments, the concentration of reducing reagent in the lysis buffer is about 5 mM. In some embodiments, a lysis buffer can comprise about 0.1M TrisHCl, about pH 7.5, about 0.5M LiCl, about 1% lithium dodecyl sulfate, about 10mM EDTA, and about 5mM DTT.

Lysis can be performed at a temperature of about 4, 10, 15, 20, 25, or 30 °C. Lysis can be performed for about 1, 5, 10, 15, or 20 or more minutes. A lysed cell can comprise at least about 100000, 200000, 300000, 400000, 500000, 600000, or 700000 or more target nucleic acid molecules. A lysed cell can comprise at most about 100000, 200000, 300000, 400000, 500000, 600000, or 700000 or more target nucleic acid molecules.

### Attachment of Barcodes to Target Nucleic Acid Molecules

Following lysis of the cells and release of nucleic acid molecules therefrom, the nucleic acid molecules can randomly associate with the barcodes of the co-localized solid support. Association can comprise hybridization of a barcode's target recognition region to a complementary portion of the target nucleic acid molecule (e.g., oligo(dT) of the barcode can interact with a poly(A) tail of a target). The assay conditions used for hybridization (e.g., buffer pH, ionic strength, temperature, etc.) can be chosen to promote formation of specific, stable hybrids. In some embodiments, the nucleic acid molecules released from the lysed cells can associate with the plurality, of probes on the substrate (e.g., hybridize with the probes on the substrate). When the probes comprise oligo(dT), mRNA molecules can hybridize to the probes and be reverse transcribed. The oligo(dT) portion of the oligonucleotide can act as a primer for first strand synthesis of the cDNA molecule. For example, in a non-limiting example of barcoding illustrated in FIG. 2, at block 216, mRNA molecules can hybridize to barcodes on beads. For example, single-stranded nucleotide fragments can hybridize to the target-binding regions of barcodes.

Attachment can further comprise ligation of a barcode's target recognition region and a portion of the target nucleic acid molecule. For example, the target binding region can comprise a nucleic acid sequence that can be capable of specific hybridization to a restriction site overhang (e.g., an EcoRI sticky-end overhang). The assay procedure can further comprise treating the target nucleic acids with a restriction enzyme (e.g., EcoRI) to create a restriction site overhang. The barcode can then be ligated to any nucleic acid molecule comprising a sequence complementary to the restriction site overhang. A ligase (e.g., T4 DNA ligase) can be used to join the two fragments.

For example, in a non-limiting example of barcoding illustrated in FIG. 2, at block 220, the labeled targets from a plurality of cells (or a plurality of samples) (e.g., target-barcode molecules) can be subsequently pooled, for example, into a tube. The labeled targets can be pooled by, for example, retrieving the barcodes and/or the beads to which the target-barcode molecules are attached.

The retrieval of solid support-based collections of attached target-barcode molecules can be implemented by use of magnetic beads and an externally-applied magnetic field. Once the target-barcode molecules have been pooled, all further processing can proceed in a single reaction vessel. Further processing can include, for example, reverse transcription reactions, amplification reactions, cleavage reactions, dissociation reactions, and/or nucleic acid extension reactions. Further processing reactions can be performed within the microwells, that is, without first pooling the labeled target nucleic acid molecules from a plurality of cells.

### Reverse Transcription or Nucleic Acid Extension

The disclosure provides for a method to create a target-barcode conjugate using reverse transcription (e.g., at block 224 of FIG. 2) or nucleic acid extension. The target-barcode conjugate can comprise the barcode and a complementary sequence of all or a portion of the target nucleic acid (i.e., a barcoded cDNA molecule, such as a stochastically barcoded cDNA molecule). Reverse transcription of the associated RNA molecule can occur by the addition of a reverse transcription primer along with the reverse transcriptase. The reverse transcription primer can be an oligo(dT) primer, a random hexanucleotide primer, or a target-specific oligonucleotide primer. Oligo(dT) primers can be, or can be about, 12-18 nucleotides in length and bind to the endogenous poly(A) tail at the 3' end of mammalian mRNA. Random hexanucleotide primers can bind to mRNA at a variety of complementary sites. Target-specific oligonucleotide primers typically selectively prime the mRNA of interest.

In some embodiments, reverse transcription of an mRNA molecule to a labeled-RNA molecule can occur by the addition of a reverse transcription primer. In some embodiments, the reverse transcription primer is an oligo(dT) primer, random hexanucleotide primer, or a target-specific oligonucleotide primer. Generally, oligo(dT) primers are 12-18 nucleotides in length and bind to the endogenous poly(A) tail at the 3' end of mammalian mRNA. Random hexanucleotide primers can bind to mRNA at a variety of complementary sites. Target-specific oligonucleotide primers typically selectively prime the mRNA of interest.

In some embodiments, a target is a cDNA molecule. For example, an mRNA molecule can be reverse transcribed using a reverse transcriptase, such as Moloney murine leukemia virus (MMLV) reverse transcriptase, to generate a cDNA molecule with a poly(dC) tail. A barcode can include a target binding region with a poly(dG) tail. Upon base pairing between the poly(dG) tail of the barcode and the poly(dC) tail of the cDNA molecule, the reverse transcriptase switches template strands, from cellular RNA molecule to the barcode, and continues replication to the 5' end of the barcode. By doing so, the resulting cDNA molecule contains the sequence of the barcode (such as the molecular label) on the 3' end of the cDNA molecule.

Reverse transcription can occur repeatedly to produce multiple labeled-cDNA molecules. The methods disclosed herein can comprise conducting at least about 1, 2, 3, 4, 5, 6, 7, 8, 9, 10, 11, 12, 13, 14, 15, 16, 17, 18, 19, or 20 reverse transcription reactions. The method can comprise conducting at least about 25, 30, 35, 40, 45, 50, 55, 60, 65, 70, 75, 80, 85, 90, 95, or 100 reverse transcription reactions.

### Amplification

One or more nucleic acid amplification reactions (e.g., at block 228 of FIG. 2) can be performed to create multiple copies of the labeled target nucleic acid molecules. Amplification can be performed in a multiplexed manner, wherein multiple target nucleic acid sequences are amplified simultaneously. The amplification reaction can be used to add sequencing adaptors to the nucleic acid molecules. The amplification reactions can comprise amplifying at least a portion of a sample label, if present. The amplification reactions can comprise amplifying at least a portion of the cellular label and/or barcode sequence (e.g., a molecular label). The amplification reactions can comprise amplifying at least a portion of a sample tag, a cell label, a spatial label, a barcode sequence (e.g., a molecular label), a target nucleic acid, or a combination thereof. The amplification reactions can comprise amplifying 0.5%, 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 97%, 100%, or a range or a number between any two of these values, of the plurality of nucleic acids. The method can further comprise conducting one or more cDNA synthesis reactions to produce one or more cDNA copies of target-barcode molecules comprising a sample label, a cell label, a spatial label, and/or a barcode sequence (e.g., a molecular label).

In some embodiments, amplification can be performed using a polymerase chain reaction (PCR). As used herein, PCR can refer to a reaction for the in vitro amplification of specific DNA sequences by the simultaneous primer extension of complementary strands of DNA. As used herein, PCR can encompass derivative forms of the reaction, including but not limited to, RT-PCR, real-time PCR, nested PCR, quantitative PCR, multiplexed PCR, digital PCR, and assembly PCR.

Amplification of the labeled nucleic acids can comprise non-PCR based methods. Examples of non-PCR based methods include, but are not limited to, multiple displacement amplification (MDA), transcription-mediated amplification (TMA), nucleic acid sequence-based amplification (NASBA), strand displacement amplification (SDA), real-time SDA, rolling circle amplification, or circle-to-circle amplification. Other non-PCR-based amplification methods include multiple cycles of DNA-dependent RNA polymerase-driven RNA transcription amplification or RNA-directed DNA synthesis and transcription to amplify DNA or RNA targets, a ligase chain reaction (LCR), and a Qβ replicase (Qβ) method, use of palindromic probes, strand displacement amplification, oligonucleotide-driven amplification using a restriction endonuclease, an amplification method in which a primer is hybridized to a nucleic acid sequence and the resulting duplex is cleaved prior to the extension reaction and amplification, strand displacement amplification using a nucleic acid polymerase lacking 5' exonuclease activity, rolling circle amplification, and ramification extension amplification (RAM). In some embodiments, the amplification does not produce circularized transcripts.

In some embodiments, the methods disclosed herein further comprise conducting a polymerase chain reaction on the labeled nucleic acid (e.g., labeled-RNA, labeled-DNA, labeled-cDNA) to produce a labeled amplicon (e.g., a stochastically labeled amplicon). The labeled amplicon can be double-stranded molecule. The double-stranded molecule can comprise a double-stranded RNA molecule, a double-stranded DNA molecule, or a RNA molecule hybridized to a DNA molecule. One or both of the strands of the double-stranded molecule can comprise a sample label, a spatial label, a cell label, and/or a barcode sequence (e.g., a molecular label). The labeled amplicon can be a single-stranded molecule. The single-stranded molecule can comprise DNA, RNA, or a combination thereof. The nucleic acids of the disclosure can comprise synthetic or altered nucleic acids.

Amplification can comprise use of one or more non-natural nucleotides. Non-natural nucleotides can comprise photolabile or triggerable nucleotides. Examples of non-natural nucleotides can include, but are not limited to, peptide nucleic acid (PNA), morpholino and locked nucleic acid (LNA), as well as glycol nucleic acid (GNA) and threose nucleic acid (TNA). Non-natural nucleotides can be added to one or more cycles of an amplification reaction. The addition of the non-natural nucleotides can be used to identify products as specific cycles or time points in the amplification reaction.

Conducting the one or more amplification reactions can comprise the use of one or more primers. The one or more primers can comprise, for example, 1, 2, 3, 4, 5, 6, 7, 8, 9, 10, 11, 12, 13, 14, or 15 or more nucleotides. The one or more primers can comprise at least 1, 2, 3, 4, 5, 6, 7, 8, 9, 10, 11, 12, 13, 14, or 15 or more nucleotides. The one or more primers can comprise less than 12-15 nucleotides. The one or more primers can anneal to at least a portion of the plurality of labeled targets (e.g., stochastically labeled targets). The one or more primers can anneal to the 3' end or 5' end of the plurality, of labeled targets. The one or more primers can anneal to an internal region of the plurality, of labeled targets. The internal region can be at least about 50, 100, 150, 200, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 650, 700, 750, 800, 850, 900 or 1000 nucleotides from the 3' ends the plurality of labeled targets. The one or more primers can comprise a fixed panel of primers. The one or more primers can comprise at least one or more custom primers. The one or more primers can comprise at least one or more control primers. The one or more primers can comprise at least one or more gene-specific primers.

The one or more primers can comprise a universal primer. The universal primer can anneal to a universal primer binding site. The one or more custom primers can anneal to a first sample label, a second sample label, a spatial label, a cell label, a barcode sequence (e.g., a molecular label), a target, or any combination thereof. The one or more primers can comprise a universal primer and a custom primer. The custom primer can be designed to amplify one or more targets. The targets can comprise a subset of the total nucleic acids in one or more samples. The targets can comprise a subset of the total labeled targets in one or more samples. The one or more primers can comprise at least 96 or more custom primers. The one or more primers can comprise at least 960 or more custom primers. The one or more primers can comprise at least 9600 or more custom primers. The one or more custom primers can anneal to two or more different labeled nucleic acids. The two or more different labeled nucleic acids can correspond to one or more genes.

Any amplification scheme can be used in the methods of the present disclosure. For example, in one scheme, the first round PCR can amplify molecules attached to the bead using a gene specific primer and a primer against the universal Illumina sequencing primer 1 sequence. The second round of PCR can amplify the first PCR products using a nested gene specific primer flanked by Illumina sequencing primer 2 sequence, and a primer against the universal Illumina sequencing primer 1 sequence. The third round of PCR adds P5 and P7 and sample index to turn PCR products into an Illumina sequencing library. Sequencing using 150 bp x 2 sequencing can reveal the cell label and barcode sequence (e.g., molecular label) on read 1, the gene on read 2, and the sample index on index 1 read.

In some embodiments, nucleic acids can be removed from the substrate using chemical cleavage. For example, a chemical group or a modified base present in a nucleic acid can be used to facilitate its removal from a solid support. For example, an enzyme can be used to remove a nucleic acid from a substrate. For example, a nucleic acid can be removed from a substrate through a restriction endonuclease digestion. For example, treatment of a nucleic acid containing a dUTP or ddUTP with uracil-d-glycosylase (UDG) can be used to remove a nucleic acid from a substrate. For example, a nucleic acid can be removed from a substrate using an enzyme that performs nucleotide excision, such as a base excision repair enzyme, such as an apurinic/apyrimidinic (AP) endonuclease. In some embodiments, a nucleic acid can be removed from a substrate using a photocleavable group and light. In some embodiments, a cleavable linker can be used to remove a nucleic acid from the substrate. For example, the cleavable linker can comprise at least one of biotin/avidin, biotin/streptavidin, biotin/neutravidin, Ig-protein A, a photo-labile linker, acid or base labile linker group, or an aptamer.

When the probes are gene-specific, the molecules can hybridize to the probes and be reverse transcribed and/or amplified. In some embodiments, after the nucleic acid has been synthesized (e.g., reverse transcribed), it can be amplified. Amplification can be performed in a multiplex manner, wherein multiple target nucleic acid sequences are amplified simultaneously. Amplification can add sequencing adaptors to the nucleic acid.

In some embodiments, amplification can be performed on the substrate, for example, with bridge amplification. cDNAs can be homopolymer tailed in order to generate a compatible end for bridge amplification using oligo(dT) probes on the substrate. In bridge amplification, the primer that is complementary to the 3' end of the template nucleic acid can be the first primer of each pair that is covalently attached to the solid particle. When a sample containing the template nucleic acid is contacted with the particle and a single thermal cycle is performed, the template molecule can be annealed to the first primer and the first primer is elongated in the forward direction by addition of nucleotides to form a duplex molecule consisting of the template molecule and a newly formed DNA strand that is complementary to the template. In the heating step of the next cycle, the duplex molecule can be denatured, releasing the template molecule from the particle and leaving the complementary DNA strand attached to the particle through the first primer. In the annealing stage of the annealing and elongation step that follows, the complementary strand can hybridize to the second primer, which is complementary to a segment of the complementary strand at a location removed from the first primer. This hybridization can cause the complementary strand to form a bridge between the first and second primers secured to the first primer by a covalent bond and to the second primer by hybridization. In the elongation stage, the second primer can be elongated in the reverse direction by the addition of nucleotides in the same reaction mixture, thereby converting the bridge to a double-stranded bridge. The next cycle then begins, and the double-stranded bridge can be denatured to yield two single-stranded nucleic acid molecules, each having one end attached to the particle surface via the first and second primers, respectively, with the other end of each unattached. In the annealing and elongation step of this second cycle, each strand can hybridize to a further complementary primer, previously unused, on the same particle, to form new single-strand bridges. The two previously unused primers that are now hybridized elongate to convert the two new bridges to double-strand bridges.

The amplification reactions can comprise amplifying at least 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 97%, or 100% of the plurality of nucleic acids.

Amplification of the labeled nucleic acids can comprise PCR-based methods or non-PCR based methods. Amplification of the labeled nucleic acids can comprise exponential amplification of the labeled nucleic acids. Amplification of the labeled nucleic acids can comprise linear amplification of the labeled nucleic acids. Amplification can be performed by polymerase chain reaction (PCR). PCR can refer to a reaction for the in vitro amplification of specific DNA sequences by the simultaneous primer extension of complementary strands of DNA. PCR can encompass derivative forms of the reaction, including but not limited to, RT-PCR, real-time PCR, nested PCR, quantitative PCR, multiplexed PCR, digital PCR, suppression PCR, semi-suppressive PCR and assembly PCR.

In some embodiments, amplification of the labeled nucleic acids comprises non-PCR based methods. Examples of non-PCR based methods include, but are not limited to, multiple displacement amplification (MDA), transcription-mediated amplification (TMA), nucleic acid sequence-based amplification (NASBA), strand displacement amplification (SDA), real-time SDA, rolling circle amplification, or circle-to-circle amplification. Other non-PCR-based amplification methods include multiple cycles of DNA-dependent RNA polymerase-driven RNA transcription amplification or RNA-directed DNA synthesis and transcription to amplify DNA or RNA targets, a ligase chain reaction (LCR), a Qβ replicase (Qβ), use of palindromic probes, strand displacement amplification, oligonucleotide-driven amplification using a restriction endonuclease, an amplification method in which a primer is hybridized to a nucleic acid sequence and the resulting duplex is cleaved prior to the extension reaction and amplification, strand displacement amplification using a nucleic acid polymerase lacking 5' exonuclease activity, rolling circle amplification, and/or ramification extension amplification (RAM).

In some embodiments, the methods disclosed herein further comprise conducting a nested polymerase chain reaction on the amplified amplicon (e.g., target). The amplicon can be double-stranded molecule. The double-stranded molecule can comprise a double-stranded RNA molecule, a double-stranded DNA molecule, or a RNA molecule hybridized to a DNA molecule. One or both of the strands of the double-stranded molecule can comprise a sample tag or molecular identifier label. Alternatively, the amplicon can be a single-stranded molecule. The single-stranded molecule can comprise DNA, RNA, or a combination thereof. The nucleic acids of the present invention can comprise synthetic or altered nucleic acids.

In some embodiments, the method comprises repeatedly amplifying the labeled nucleic acid to produce multiple amplicons. The methods disclosed herein can comprise conducting at least about 1, 2, 3, 4, 5, 6, 7, 8, 9, 10, 11, 12, 13, 14, 15, 16, 17, 18, 19, or 20 amplification reactions. Alternatively, the method comprises conducting at least about 25, 30, 35, 40, 45, 50, 55, 60, 65, 70, 75, 80, 85, 90, 95, or 100 amplification reactions.

Amplification can further comprise adding one or more control nucleic acids to one or more samples comprising a plurality of nucleic acids. Amplification can further comprise adding one or more control nucleic acids to a plurality of nucleic acids. The control nucleic acids can comprise a control label.

Amplification can comprise use of one or more non-natural nucleotides. Non-natural nucleotides can comprise photolabile and/or triggerable nucleotides. Examples of non-natural nucleotides include, but are not limited to, PNA, morpholino and LNA, as well as GNA and TNA. Non-natural nucleotides can be added to one or more cycles of an amplification reaction. The addition of the non-natural nucleotides can be used to identify products as specific cycles or time points in the amplification reaction.

Conducting the one or more amplification reactions can comprise the use of one or more primers. The one or more primers can comprise one or more oligonucleotides. The one or more oligonucleotides can comprise at least about 7-9 nucleotides. The one or more oligonucleotides can comprise less than 12-15 nucleotides. The one or more primers can anneal to at least a portion of the plurality of labeled nucleic acids. The one or more primers can anneal to the 3' end and/or 5' end of the plurality of labeled nucleic acids. The one or more primers can anneal to an internal region of the plurality of labeled nucleic acids. The internal region can be at least about 50, 100, 150, 200, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 650, 700, 750, 800, 850, 900 or 1000 nucleotides from the 3' ends the plurality of labeled nucleic acids. The one or more primers can comprise a fixed panel of primers. The one or more primers can comprise at least one or more custom primers. The one or more primers can comprise at least one or more control primers. The one or more primers can comprise at least one or more housekeeping gene primers. The one or more primers can comprise a universal primer. The universal primer can anneal to a universal primer binding site. The one or more custom primers can anneal to the first sample tag, the second sample tag, the molecular identifier label, the nucleic acid or a product thereof. The one or more primers can comprise a universal primer and a custom primer. The custom primer can be designed to amplify one or more target nucleic acids. The target nucleic acids can comprise a subset of the total nucleic acids in one or more samples. In some embodiments, the primers are the probes attached to the array of the disclosure.

In some embodiments, barcoding (e.g., stochastically barcoding) the plurality of targets in the sample further comprises generating an indexed library of the barcoded targets (e.g., stochastically barcoded targets) or barcoded fragments of the targets. The barcode sequences of different barcodes (e.g., the molecular labels of different stochastic barcodes) can be different from one another. Generating an indexed library of the barcoded targets includes generating a plurality of indexed polynucleotides from the plurality of targets in the sample. For example, for an indexed library of the barcoded targets comprising a first indexed target and a second indexed target, the label region of the first indexed polynucleotide can differ from the label region of the second indexed polynucleotide by, by about, by at least, or by at most, 1, 2, 3, 4, 5, 6, 7, 8, 9, 10, 20, 30, 40, 50, or a number or a range between any two of these values, nucleotides. In some embodiments, generating an indexed library of the barcoded targets includes contacting a plurality, of targets, for example mRNA molecules, with a plurality of oligonucleotides including a poly(T) region and a label region; and conducting a first strand synthesis using a reverse transcriptase to produce single-strand labeled cDNA molecules each comprising a cDNA region and a label region, wherein the plurality of targets includes at least two mRNA molecules of different sequences and the plurality of oligonucleotides includes at least two oligonucleotides of different sequences. Generating an indexed library of the barcoded targets can further comprise amplifying the single-strand labeled cDNA molecules to produce double-strand labeled cDNA molecules; and conducting nested PCR on the double-strand labeled cDNA molecules to produce labeled amplicons. In some embodiments, the method can include generating an adaptor-labeled amplicon.

Barcoding (e.g., stochastic barcoding) can include using nucleic acid barcodes or tags to label individual nucleic acid (e.g., DNA or RNA) molecules. In some embodiments, it involves adding DNA barcodes or tags to cDNA molecules as they are generated from mRNA. Nested PCR can be performed to minimize PCR amplification bias. Adaptors can be added for sequencing using, for example, next generation sequencing (NGS). The sequencing results can be used to determine cell labels, molecular labels, and sequences of nucleotide fragments of the one or more copies of the targets, for example at block 232 of FIG. 2.

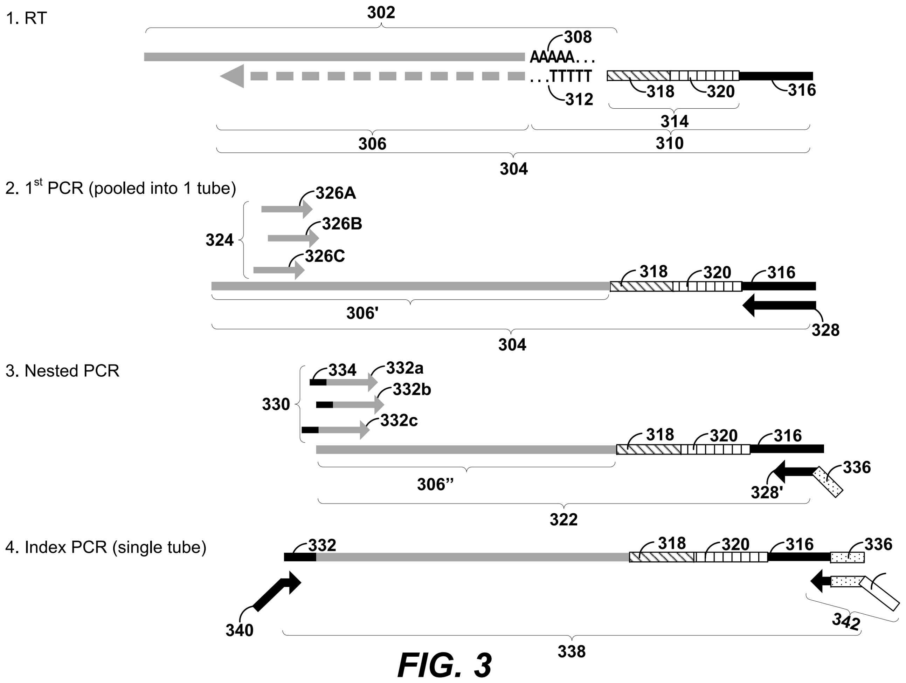

FIG. 3 is a schematic illustration showing a non-limiting exemplary process of generating an indexed library of the barcoded targets (e.g., stochastically barcoded targets), such as barcoded mRNAs or fragments thereof. As shown in step 1, the reverse transcription process can encode each mRNA molecule with a unique molecular label sequence, a cell label sequence, and a universal PCR site. In particular, RNA molecules 302 can be reverse transcribed to produce labeled cDNA molecules 304, including a cDNA region 306, by hybridization (e.g., stochastic hybridization) of a set of barcodes (e.g., stochastic barcodes) 310 to the poly(A) tail region 308 of the RNA molecules 302. Each of the barcodes 310 can comprise a target-binding region, for example a poly(dT) region 312, a label region 314 (e.g., a barcode sequence or a molecule), and a universal PCR region 316.

In some embodiments, the cell label sequence can include 3 to 20 nucleotides. In some embodiments, the molecular label sequence can include 3 to 20 nucleotides. In some embodiments, each of the plurality, of stochastic barcodes further comprises one or more of a universal label and a cell label, wherein universal labels are the same for the plurality of stochastic barcodes on the solid support and cell labels are the same for the plurality of stochastic barcodes on the solid support. In some embodiments, the universal label can include 3 to 20 nucleotides. In some embodiments, the cell label comprises 3 to 20 nucleotides.

In some embodiments, the label region 314 can include a barcode sequence or a molecular label 318 and a cell label 320. In some embodiments, the label region 314 can include one or more of a universal label, a dimension label, and a cell label. The barcode sequence or molecular label 318 can be, can be about, can be at least, or can be at most, 1, 2, 3, 4, 5, 6, 7, 8, 9, 10, 20, 30, 40, 50, 60, 70, 80, 90, 100, or a number or a range between any of these values, of nucleotides in length. The cell label 320 can be, can be about, can be at least, or can be at most, 1, 2, 3, 4, 5, 6, 7, 8, 9, 10, 20, 30, 40, 50, 60, 70, 80, 90, 100, or a number or a range between any of these values, of nucleotides in length. The universal label can be, can be about, can be at least, or can be at most, 1, 2, 3, 4, 5, 6, 7, 8, 9, 10, 20, 30, 40, 50, 60, 70, 80, 90, 100, or a number or a range between any of these values, of nucleotides in length. Universal labels can be the same for the plurality of stochastic barcodes on the solid support and cell labels are the same for the plurality of stochastic barcodes on the solid support. The dimension label can be, can be about, can be at least, or can be at most 1, 2, 3, 4, 5, 6, 7, 8, 9, 10, 20, 30, 40, 50, 60, 70, 80, 90, 100, or a number or a range between any of these values, of nucleotides in length.

In some embodiments, the label region 314 can comprise, comprise about, comprise at least, or comprise at most, 1, 2, 3, 4, 5, 6, 7, 8, 9, 10, 20, 30, 40, 50, 60, 70, 80, 90, 100, 200, 300, 400, 500, 600, 700, 800, 900, 1000, or a number or a range between any of these values, different labels, such as a barcode sequence or a molecular label 318 and a cell label 320. Each label can be, can be about, can be at least, or can be at most 1, 2, 3, 4, 5, 6, 7, 8, 9, 10, 20, 30, 40, 50, 60, 70, 80, 90, 100, or a number or a range between any of these values, of nucleotides in length. A set of barcodes or stochastic barcodes 310 can contain, contain about, contain at least, or can be at most, 10, 20, 40, 50, 70, 80, 90, 10², 10³, 10⁴, 10⁵, 10⁶, 10⁷, 10⁸, 10⁹, 10¹⁰, 10¹¹, 10¹², 10¹³, 10¹⁴, 10¹⁵, 10²⁰, or a number or a range between any of these values, barcodes or stochastic barcodes 310. And the set of barcodes or stochastic barcodes 310 can, for example, each contain a unique label region 314. The labeled cDNA molecules 304 can be purified to remove excess barcodes or stochastic barcodes 310. Purification can comprise Ampure bead purification.

As shown in step 2, products from the reverse transcription process in step 1 can be pooled into 1 tube and PCR amplified with a 1^{st} PCR primer pool and a 1^{st} universal PCR primer. Pooling is possible because of the unique label region 314. In particular, the labeled cDNA molecules 304 can be amplified to produce nested PCR labeled amplicons 322. Amplification can comprise multiplex PCR amplification. Amplification can comprise a multiplex PCR amplification with 96 multiplex primers in a single reaction volume. In some embodiments, multiplex PCR amplification can utilize, utilize about, utilize at least, or utilize at most, 10, 20, 40, 50, 70, 80, 90, 10², 10³, 10⁴, 10⁵, 10⁶, 10⁷, 10⁸, 10⁹, 10¹⁰, 10¹¹, 10¹², 10¹³, 10¹⁴, 10¹⁵, 10²⁰, or a number or a range between any of these values, multiplex primers in a single reaction volume. Amplification can comprise using a 1^{st} PCR primer pool 324 comprising custom primers 326A-C targeting specific genes and a universal primer 328. The custom primers 326 can hybridize to a region within the cDNA portion 306' of the labeled cDNA molecule 304. The universal primer 328 can hybridize to the universal PCR region 316 of the labeled cDNA molecule 304.

As shown in step 3 of FIG. 3, products from PCR amplification in step 2 can be amplified with a nested PCR primers pool and a 2^{nd} universal PCR primer. Nested PCR can minimize PCR amplification bias. In particular, the nested PCR labeled amplicons 322 can be further amplified by nested PCR. The nested PCR can comprise multiplex PCR with nested PCR primers pool 330 of nested PCR primers 332a-c and a 2^{nd} universal PCR primer 328' in a single reaction volume. The nested PCR primer pool 328 can contain, contain about contain at least, or contain at most, 1, 2, 3, 4, 5, 6, 7, 8, 9, 10, 20, 30, 40, 50, 60, 70, 80, 90, 100, 200, 300, 400, 500, 600, 700, 800, 900, 1000, or a number or a range between any of these values, different nested PCR primers 330. The nested PCR primers 332 can contain an adaptor 334 and hybridize to a region within the cDNA portion 306'' of the labeled amplicon 322. The universal primer 328' can contain an adaptor 336 and hybridize to the universal PCR region 316 of the labeled amplicon 322. Thus, step 3 produces adaptor-labeled amplicon 338. In some embodiments, nested PCR primers 332 and the 2^{nd} universal PCR primer 328' may not contain the adaptors 334 and 336. The adaptors 334 and 336 can instead be ligated to the products of nested PCR to produce adaptor-labeled amplicon 338.

As shown in step 4, PCR products from step 3 can be PCR amplified for sequencing using library amplification primers. In particular, the adaptors 334 and 336 can be used to conduct one or more additional assays on the adaptor-labeled amplicon 338. The adaptors 334 and 336 can be hybridized to primers 340 and 342. The one or more primers 340 and 342 can be PCR amplification primers. The one or more primers 340 and 342 can be sequencing primers. The one or more adaptors 334 and 336 can be used for further amplification of the adaptor-labeled amplicons 338. The one or more adaptors 334 and 336 can be used for sequencing the adaptor-labeled amplicon 338. The primer 342 can contain a plate index 344 so that amplicons generated using the same set of barcodes or stochastic barcodes 310 can be sequenced in one sequencing reaction using next generation sequencing (NGS).

### Reference Particles for Linking Imaging and Sequencing Readouts with Single-Cell Resolution

There are provided, in some embodiments, reference particles (e.g., reference beads) for linking imaging based readouts with sequencing based readouts with single-cell resolution. As used herein, the terms "reference particle" and "reference bead" can be used interchangeably. Some embodiments of the compositions and methods provided herein utilize reference particles to link imaging based readouts with sequencing based readouts. The disclosed compositions and methods enable new applications for existing platforms, especially for single cell sequencing platforms based on micro-well or droplet based approaches. In some embodiments, the approach is based on reference particles that are uniquely identifiable by both imaging (through a combination of defined bead size, and/or the profile of fluorescence emission intensity and wavelength) and oligonucleotide sequence of conjugated probes that allows for enumeration of each bead type. Also provided, in some embodiments, are approaches to load each droplet or micro-well with a unique combination of reference particles such that no two microwells or microdroplets contain the same combination of reference particles. By enumerating the number of reference particles (e.g., reference beads) within each microwell / microdroplet both by imaging and sequencing, the imaging based and sequencing based readouts can be linked.

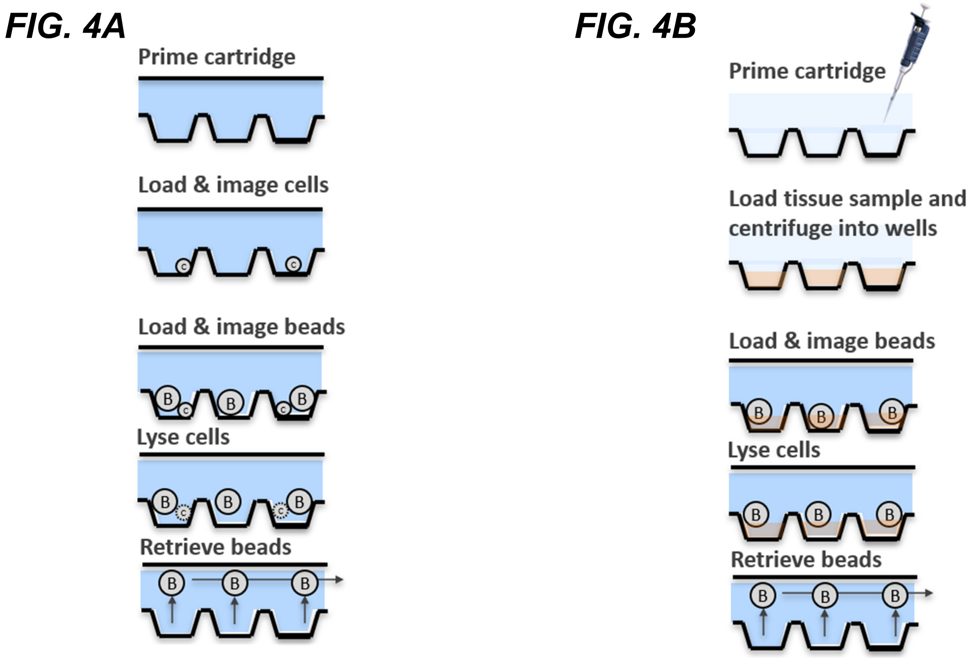

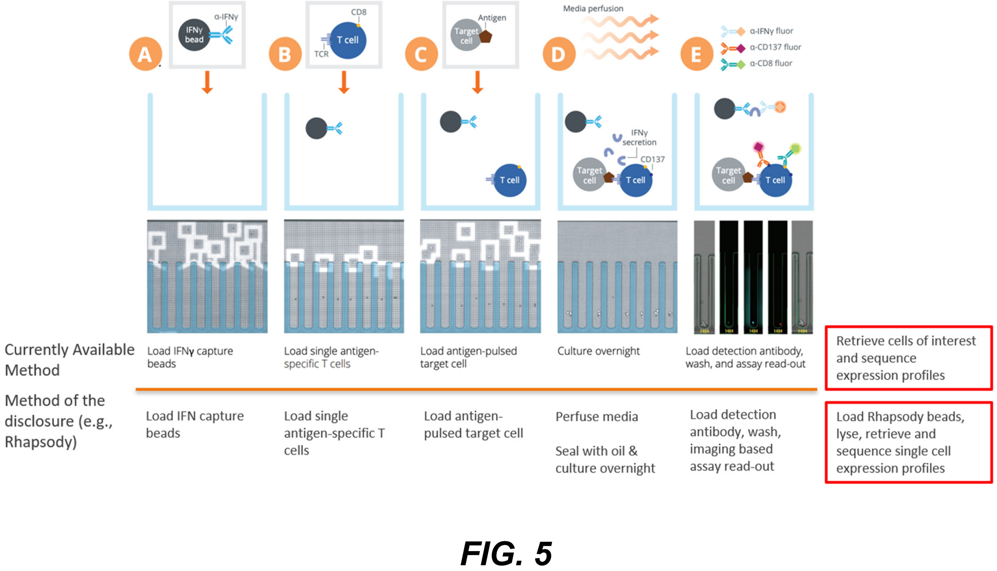

FIG. 4A depicts a non-limiting exemplary workflow provided herein. One application of the disclosed methods and compositions is improving the value of the currently existing platforms by better utilizing the scanner, which can enable, for example, (i) high-confidence cell multiplet identification and removal from the sequencing data, (ii) viability detection & association with sequencing endpoints, (iii) association of cell or nuclear size/morphology with sequencing endpoints, and/or (iv) combining imaging based assays with Rhapsody (e.g., single cell capture and barcoding) assays (e.g., Immunofluorescence, FISH). FIG. 4B depicts a non-limiting exemplary workflow provided herein. One application of the disclosed methods and compositions is addressing the need for spatial transcriptomics platforms. Pipetting barcoded oligos to wells is not scalable or manufacturable because: a) technology exists to print pL droplets, but throughput does not meet needs, and b) lithographic in-well oligonucleotide synthesis is hypothetically possible, but it is expensive to develop and commercialize. FIG. 5 depict non-limiting exemplary workflows of a currently available method and a method described herein (e.g., a cell secretion assay).

A currently available alternative approach to the reference particle-based methods provided herein is the optical barcoding of beads. However, this approach has several downsides, including, but not limited to: (i) laborious workflow for readout, (ii) expensive to automate, and (iii) can pre-load beads and readout at manufacturing, but pre-loading presents S/N challenge (cell stacked on beads vs. bead stacked on cells) and expensive development.

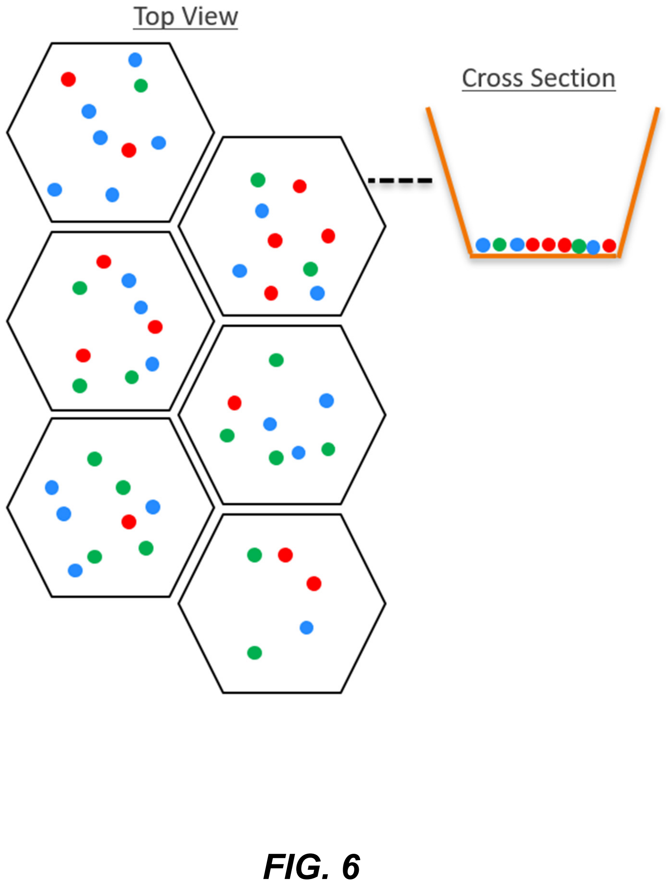

FIG. 6 depicts a non-limiting exemplary schematic of the reference particles (e.g., reference beads) provided herein situated within partitions (e.g., microwells). In some embodiments, reference beads are loaded such that each micro-well contains a unique combination of bead types. In some embodiments, the number of each bead type within each micro-well is enumerated by imaging. Bead types can be identified by the spectrum of their fluorescence emission, by fluorescence intensity, by size, by properties identifiable in phase contrast microscopy, or by other imaging modalities disclosed herein. In some embodiments, the types of reference bead per micro-well are enumerated by sequencing through a cleavable barcoded oligonucleotide on each reference bead, which can comprise: (a) a bead type identifier (BTI) and (b) a unique bead identifier (UBI) to enumerate the number of beads having the same BTI. In some embodiments, the size of reference beads does not restrict subsequent cell and bead (e.g., solid support) loading.

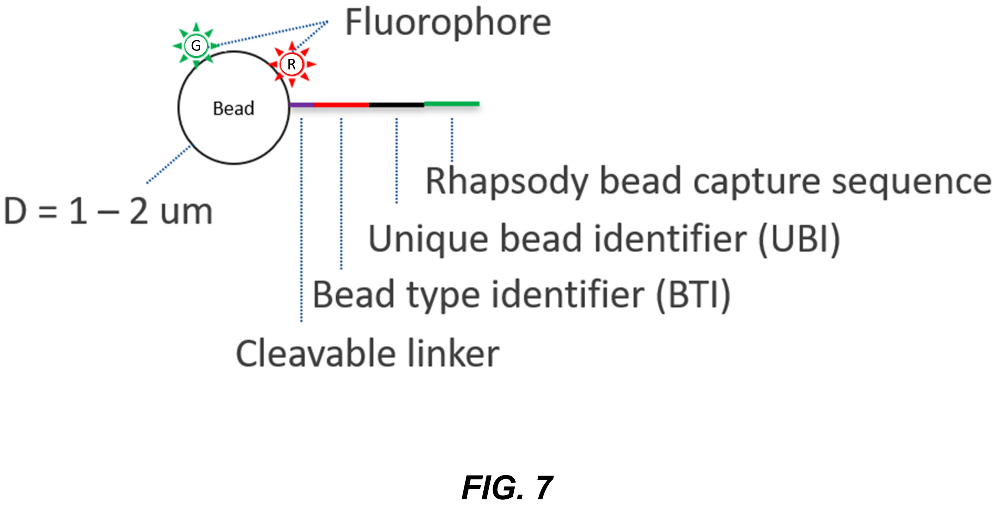

FIG. 7 depicts a non-limiting exemplary schematic of a reference particle (e.g., reference bead) provided herein. In some embodiments, reference particle types (e.g., reference bead types) are generated through co-conjugation of detectable moieties (e.g, fluorophores) along with matching barcoded oligonucleotides (e.g., particle indexing oligonucleotide) to each reference particle (e.g, reference bead). Reference particles can be associated with (e.g., conjugated with) different colors of fluorophores, and each bead type can have a unique combination of fluorophores that can be identified by imaging. Reference particles can be associated with (e.g., conjugated with) with particle indexing oligonucleotides. Particle indexing oligonucleotides can comprise one or more of: (i) a cleavable linker; (ii) a particle type identifier (PTI) (e.g., bead type identifier (BTI)), which can identify the detectable moiety (e.g., fluorophores) conjugated to the bead type; (iii) a unique particle identifier (UPI) (e.g., a unique bead identifier (UBI)) barcode, and each bead within a population of the same bead type can have a unique UPI; and (iv) a capture sequence complementary to the oligonucleotide capture sequence on the barcoding solid support (e.g., a Rhapsody oligonucleotide capture beads).

In some embodiments, the reference particles (e.g., beads) are 1-2 micrometers in diameter. Such beads can be commercially available. In some embodiments, the reference particle are large enough to image with standard imaging systems such as commercial microscopes or the Rhapsody scanner. In some embodiments, the small size of the reference particles does not impede subsequent loading of cells and/or Rhapsody capture beads.

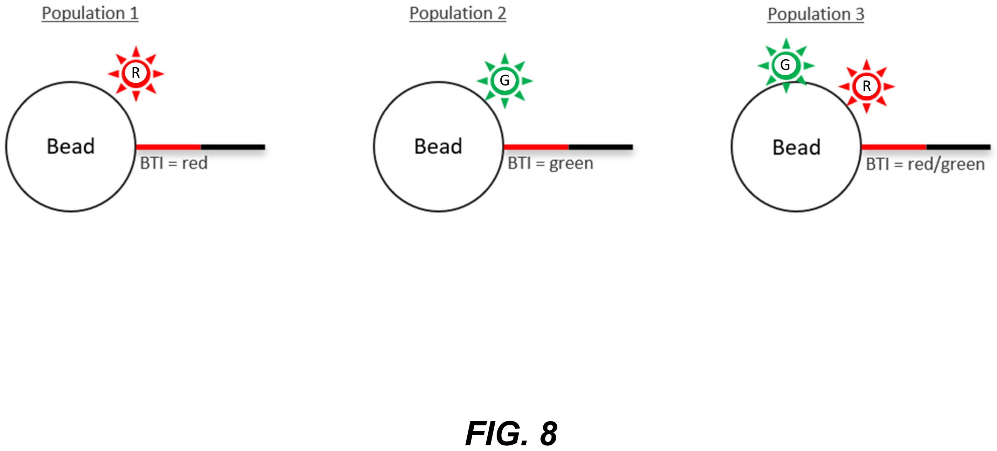

FIG. 8 depicts a non-limiting exemplary schematic of reference particle populations (e.g., reference bead populations) provided herein. The BTI (e.g., PTI) can identify the detectable moieties (e.g,. fluor colors) conjugated to a population of beads (e.g., particles). Increasing the number of unique fluor colors can increase the number of unique FI identifiers, equivalent to the combination of unique fluor colors, that can be manufactured. The particle indexing oligonucleotide (e.g., barcodes) can comprise bead type identifiers (BTI) and/or reference bead identifiers (RBI).

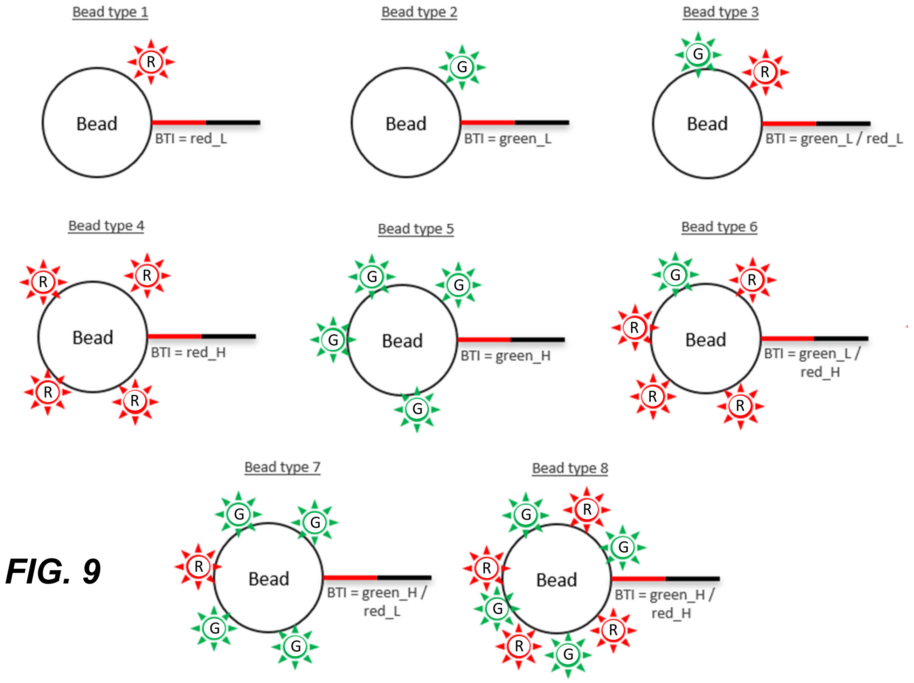

FIG. 9 depicts a non-limiting exemplary schematic of reference particle populations (e.g., reference bead populations) provided herein wherein a variety of reference particle types (e.g., reference bead types) can be generated by varying the density of detectable moieties (e.g., fluor colors) conjugated to the reference beads. In some embodiments, varying the density, of detectable moieties (e.g., fluor colors) conjugated to the reference beads results in corresponding fluorescence emission intensity. In some embodiments, this approach of varying the density of detectable moieties increases the unique reference bead types measurable by a two color fluor imager from 3 bead types to 8 bead types. In some embodiments, the BTI (e.g., PTI) identifies both the detectable moieties (e.g., fluor colors) and the density of detectable moieties (e.g., fluor colors) conjugated to a population of beads (e.g., the 9 bead types shown in FIG. 9). The particle indexing oligonucleotide (e.g., barcodes) can comprise bead type identifiers (BTI) and/or reference bead identifiers (RBI).

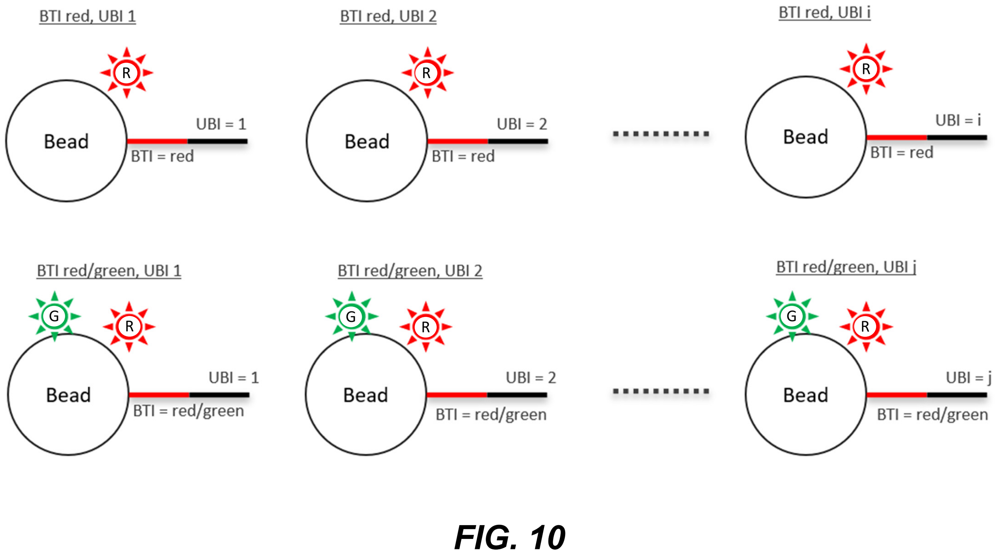

FIG. 10 depicts a non-limiting exemplary schematic of reference particle populations (e.g., reference bead populations) provided herein. In some embodiments, the particle indexing oligonucleotide (e.g., barcodes) comprises a unique particle identifier (e.g., unique bead identifier). In some embodiments, the unique particle identifier (UPI) allows enumeration of particles (e.g., beads) of a specific particle (e.g., bead) type by oligonucleotide sequencing. In some embodiments of the compositions and methods provided herein, to ensure that each bead gets a unique bead identifier, the pool of unique bead identifiers is sufficiently large such that it is greater in size (preferably 100x to 1000x greater) than the number of beads of each bead type loaded to each microwell. For example, if an average of 10 beads of type red_low are loaded to a microwell, then, in some embodiments, the number of unique UBIs for this type of bead should be ~1000 to 10,000. Reference beads can be conjugated with barcoded oligos (e.g., particle indexing oligonucleotide) and with different colors of fluorophores. The barcodes can comprise fluorophore identifier (FI), and unique bead identifier (UBI) barcodes.

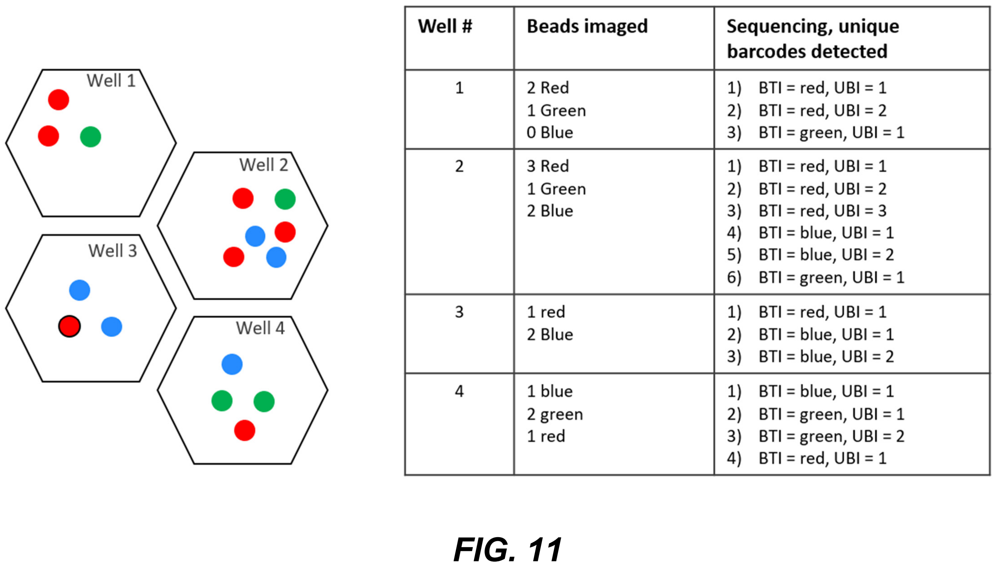

FIG. 11 depicts a non-limiting exemplary schematic of 4 partitions (e.g., microwells), each with a unique combination of reference beads, and how the reference beads can be counted by imaging and by sequencing readouts. Provided herein include methods and compositions for linking partition (e.g, well) images with sequences. The number of different beads in a partition can be counted by imaging and by sequencing. For example, the beads in the 4 wells shown in FIG. 11 can be counted via both imaging readouts and sequencing readouts.

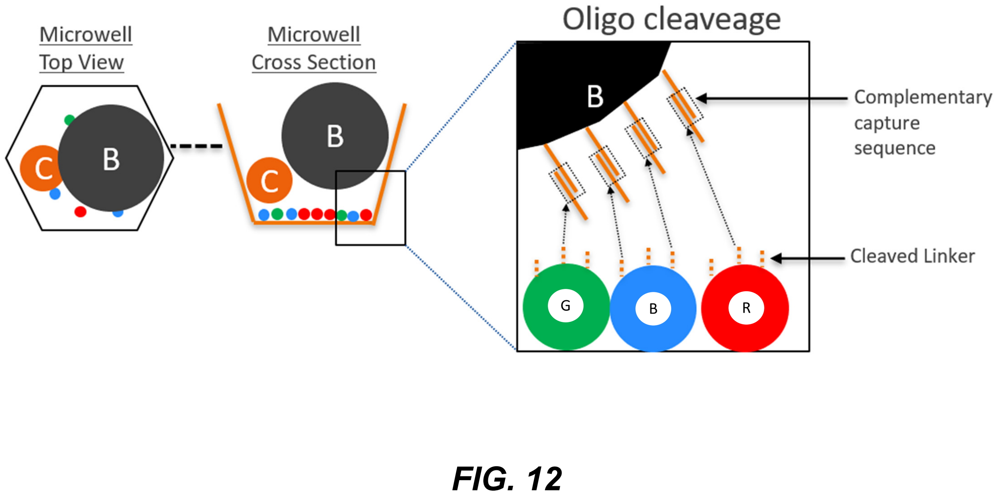

FIG. 12 depicts a non-limiting exemplary schematic of a reference particle (e.g., reference bead) provided herein. FIG. 12 illustrates how cleavable linkers between the reference bead oligonucleotide (e.g., particle indexing oligonucleotide) and the reference bead can allow for capture of the reference bead oligos on the Rhapsody capture bead. The same Rhapsody capture bead can capture target molecules that are released from target cells, which are loaded to the same microwell as the reference beads and the Rhapsody bead. FIG. 12 shows a top view and cross section of a microwell prior to cell lysis, where reference beads, target cells, and Rhapsody capture beads are loaded. Particle indexing oligonucleotide can be bound to reference beads via a cleavable linker. Particle indexing oligonucleotide can comprise a capture sequence compatible with capture on Rhapsody beads.

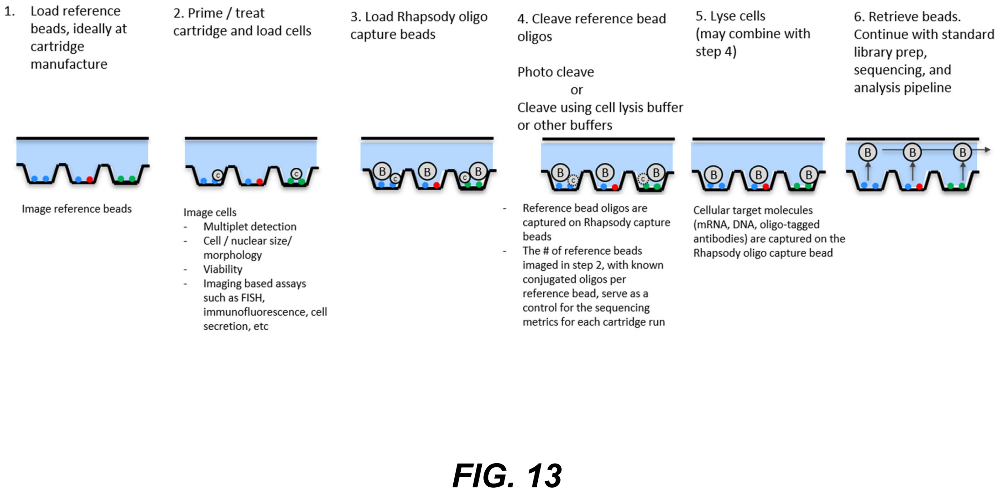

FIG. 13 depicts a non-limiting exemplary sample workflow provided herein. In some embodiments, reference beads are pre-loaded to the cartridge prior to shipping of the cartridge to customers. Pre-loading of beads can reduce the workflow complexity performed by the end-user. Reference oligos (e.g., particle indexing oligonucleotides) on the reference particles (e.g., reference beads) can be photo-cleavable or chemically cleavable. Chemically cleavable linkers can be preferable in some embodiments, as this can allow for formulation of lysis buffers that lyse target cells and cleave the reference oligos on reference beads in the same workflow step.

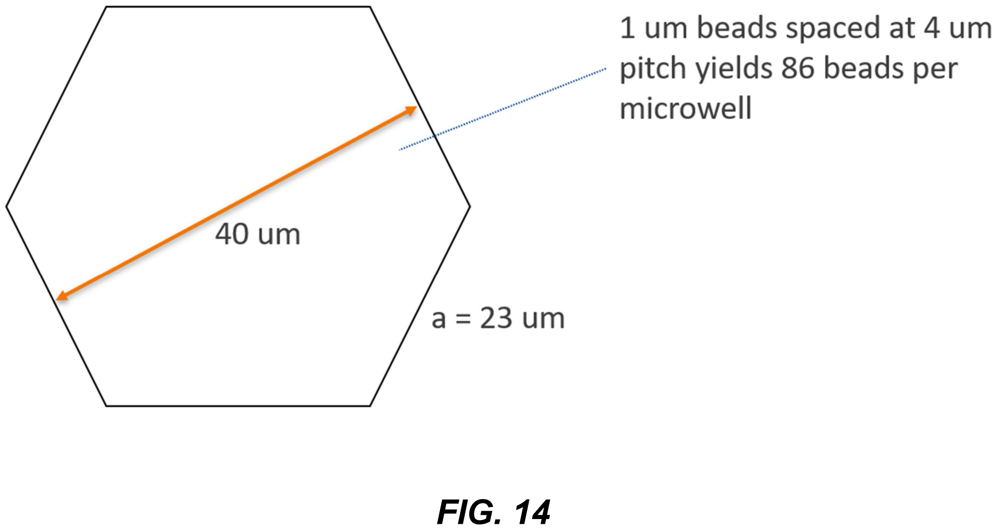

FIG. 14 depicts a calculation for the number of reference beads that can be loaded for a given microwell geometry and imaging system. In this example, the edge-to-edge distance of hexagonal wells is 40 um. In some embodiments, the imaging resolution, ~1.6 um, is able to resolve reference beads with separation of roughly 3.2 um. Both the hexagonal well geometry and the imager resolution are specs for the Rhapsody single cell analysis system v1.0. In some embodiments, given these well dimension and mean bead-to-bead pitch of 4 um, it is possible in some embodiments for the imager to resolve roughly 80 - 90 beads. If the bead types are imaged in a independent fashion, for example if the emission spectrum of one bead type does not overlap with the emission spectrum of a second bead type, then it can be possible in some embodiments to image roughly 80-90 beads per microwell of each bead type. If the emission spectrum of the bead types overlaps, for example if beads are conjugated with overlapping emission spectra, then the total beads that can be resolved is the sum of the various bead types. There can be a 40 um edge to edge of hexagon (a = 23.09, area = 1385 um2).

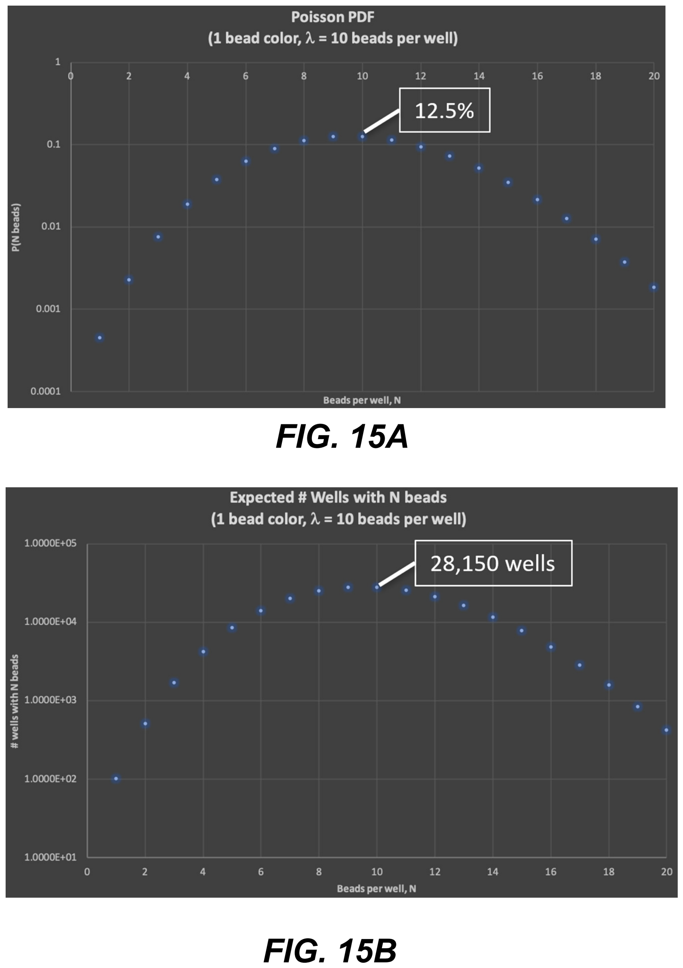

FIG. 15A depicts non-limiting exemplary probability distribution function (PDF) for reference beads loaded microwells for a given lambda (mean beads per microwell) and with 1 bead type. FIG. 15B depicts a non-limiting expected number of microwells containing a given number of beads for a given lambda. FIG. 15B indicates the number of microwells with a non-unique number of beads loaded. 1 color reference beads, λ = 10 beads loaded per well.

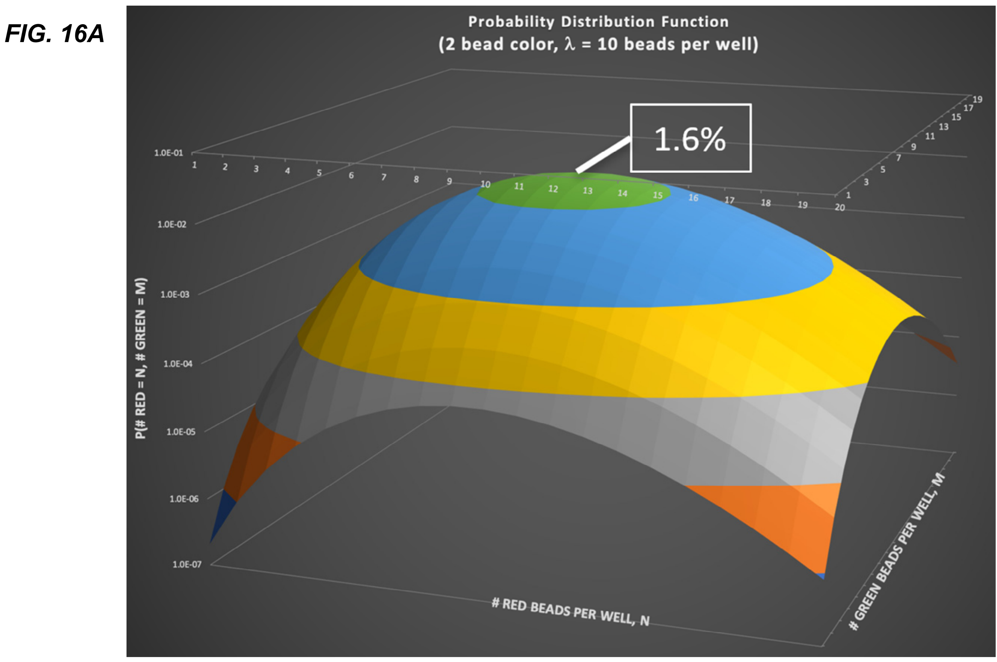

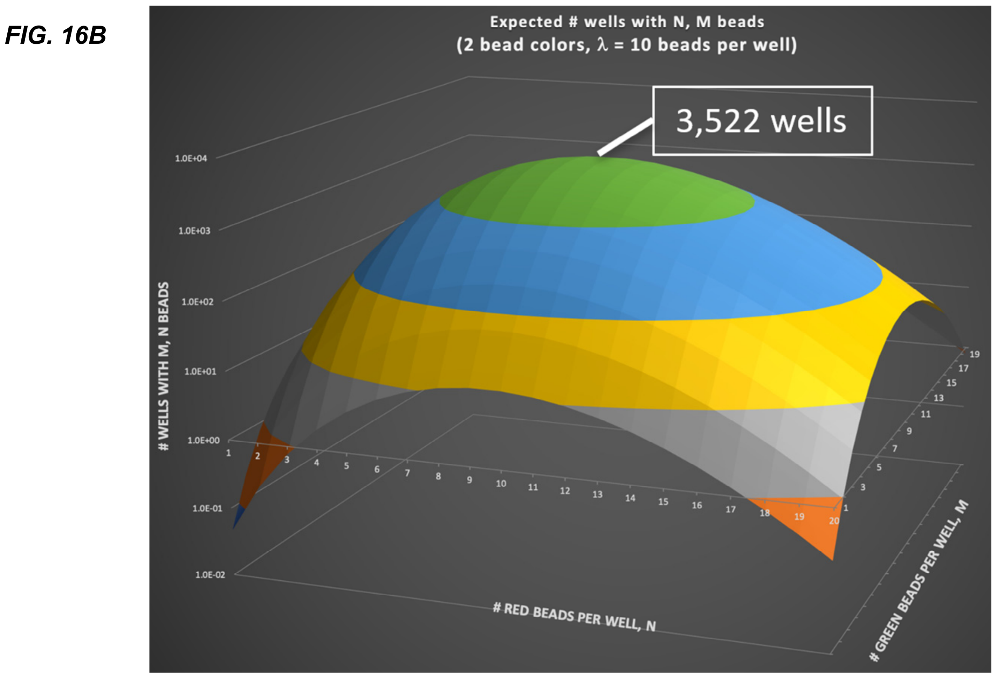

FIGS. 16A-16B depict the same analysis as FIGS. 15A-15B if the number of bead types is increased from 1 to 2. This demonstrates that increasing the number of bead types dramatically reduces the number of microwells containing a non-unique combination of bead type 1 and bead type 2.

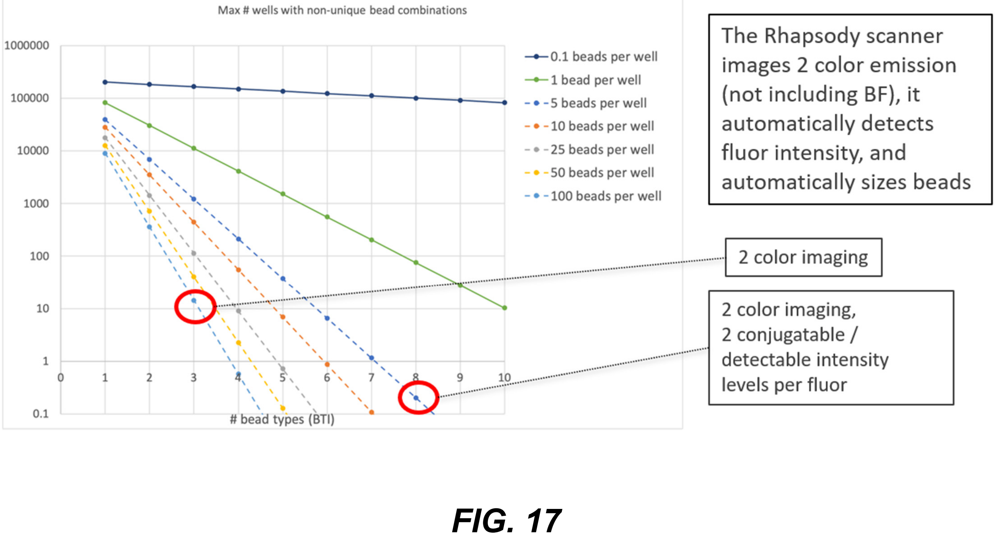

FIG. 17 shows the same analysis as FIGS. 15A-15B and FIGS. 16A-16B, extended for a range of bead types (0-10) and a range of lambdas (0.1 - 100). This is useful for understanding the tradeoffs between the complexity of the system that should be designed for bead imaging versus the complexity of the beads that need to be manufactured. The Rhapsody scanner is able to image in 3 modes: brightfield, fluorescent blue excitation / green emission, and fluorescent red excitation / far red emission. Its custom image analysis software is able to enumerate the number of beads per well, the size of each bead, and the fluorescent intensity of each bead. If the only parameter used to identify bead types is used is the fluor emission spectrum, then the imager can only resolve 3 bead types: beads conjugated with 1) green fluors, 2) red fluors, and 3) red and green fluors. If one assumes that the imager is able to also differentiate between fluor emission intensity (low and high), and reference beads can be conjugated with 2 levels of defined emission intensity, then it would be possible to generate 8 identifiable bead types: (1) Green_ Bright; (2) Green_Dim; (3) Red_Bright / Green_Bright; (4) Red_Bright / Green_Dim; (5) Green_Bright / Red_Dim: (6) Green_Dim / Red_Dim; (7) Red_Bright; and (8) Red_Dim. Without being bound by any particular theory, there can be a trade-off between instrument and bead complexity required to uniquely label 225,000 wells.

In some embodiments, other bead types such as 'blank' beads without conjugated fluors can be employed in the methods and compositions provided herein. In some embodiments, these can be more difficult to detect if the bead size is roughly on the same scale as the resolution of the imager.

Assuming 3 bead types are identifiable, the analysis indicates that with lambda ~ 100 beads / well one can expect to see on the order of tens of microwells with non-unique combinations of cells. This configuration can be acceptable, as there will be a few wells with non-unique reference beads loaded. However, if cell loading is at a rate of ~1 cell per 10 wells or 1 cell per 5 wells, it can be statistically likely that few wells with cells will have non-unique reference beads loaded relative to other wells with cells. However, with 100 beads (per bead type) loaded per microwell, the scanner (e.g Rhapsody scanner) may not be able to resolve individual reference beads as this is near the resolution limit for this particular imager. Therefore, in some embodiments, the scanner optics are configured to handle such an embodiment or a configuration with lower lambda may be required. Preferably, in some embodiments, the methods and compositions comprise 8 bead types that are identifiable and manufacturable. In this embodiment, reference beads can be loaded at low density, ~ 5 beads per well, that can be straightforward for the scanner to resolve. In this embodiment, the statistics would also favor loading of unique combinations of reference beads to all microwells.

In some embodiments, the reference particles (e.g., reference beads) provided herein can serve as control beads. In some embodiments, there is a calibrated oligonucleotide density for each bead type. Different bead types can be conjugated with different oligonucleotide density. In some embodiments, low, medium, high expressing beads are provided. In some embodiments, said reference particles cover the entire dynamic range detectable by Rhapsody. In some embodiments, images inform and verify number of each bead type present per microwell. In some embodiments, bead sizing improves accuracy of expected oligonucleotide load for each bead type. Different fluor colors can serve as an analog for low oligonucleotide expressing cells, an analog for medium oligonucleotide expressing cells, and/or an analog for high oligonucleotide expressing cells. Some embodiments of the methods and compositions provided herein enable a user to determine capture efficient of the Rhapsody capture beads using the reference beads provided herein.

There is provided, in some embodiments, non-poisson loading of reference beads to the microwells. It can be preferable, depending on the embodiment, to avoid uniform distribution of reference beads across the microwell array in order to force a non-poissonian distribution of beads. By deterministically introducing density gradients of reference beads as loaded across the microwell array, the likelihood of loading unique combinations of reference beads is increased. This may be done in various ways, such as loading reference beads under laminar flow conditions (highest bead density is loaded at the flowcell center longitudinal to the flow direction, and the lowest bead density is loaded at the flowcell boundary longitudinal to the flowcell direction) or with loading of multiple buffer plugs containing different bead concentrations, where each buffer plug is loaded to only a portion of the flowcell. Reference beads may also be loaded with a varying flow rate (e.g. slow flow rates ramping to high flow rates or vice versa). This assumes that reference beads are loaded by sedimentation, for which case the highest density of reference beads loaded to microwells would be achieved for loading at low flow rates.

Disclosed herein include methods comprising loading of reference beads to droplet based single cell analysis systems. The reference bead approach described herein can apply to both microwell and droplet based analysis systems. In droplet based systems, it can be more difficult to image small beads since beads are not deposited on a single optical focal plane, as they are for the microwell based approach. However, the droplet based systems also tend to have droplets that are larger than microwells (diameter of ~120 - 130 um for droplets vs. diameter of 40 um for microwell systems). Therefore, it is possible, in some embodiments, to compensate for the resolution deficiencies associated with 3 dimensional bead distribution by increasing the size of the reference beads.

Disclosed herein include methods comprising loading of reference beads to microwells. For loading of reference beads, it can be desirable to utilize magnetic reference beads for loading purposes. This is especially true if reference beads are small - on the order of 2 um or smaller as bead are colloidal and with slow sedimentation rates that do not readily permit bead loading by gravitational settling. In some such embodiments, it may be useful to utilize a magnet to pull reference beads to the well bottom.

The systems, methods, compositions, and kits provided herein can, in some embodiments, be employed in concert with systems, methods, compositions, and kits for associating single cell sequencing data with phenotypic data and/or agent exposure described in U.S. Patent Publication No. 20200332351, the content of which is incorporated herein by reference in its entirety.

The methods and compositions provided herein possess various advantages over currently available altemate approaches for linking images with sequencing readouts (e.g., optically readout the cell label on Rhapsody beads loaded to a cartridge via sequential probe hybridization and imaging). The primary downside to these approaches is that a relatively complex workflow is necessitated by the multiple load / denature / wash / image steps associated with sequential probe hybridization and imaging approach. This can lead to either a complex workflow for the customer / end user, a complex instrument to automate the workflow, or necessitate pre-loading & readout of Rhapsody beads at cartridge manufacture. While pre-loading of beads, followed by drying, storage at room temp, and shipping, was demonstrated successfully, the signal:noise achieved in the sequencing readout with the approach was negatively impacted. The signal reduction and noise increase resulted from vertical stacking of cells on beads (in the case of pre-loaded beads) versus stacking of beads on cells (in the case of the standard Rhapsody workflow). Therefore, the approach for reading out the Rhapsody bead cell label by sequential probe hybridization and imaging also requires further development to address this deficiency. The methods and compositions disclosed herein provides solutions to problems of the prior art in that it can conceptually be implemented with a straightforward workflow that does not necessitate development of a modified consumable or instrument.

In some embodiments, the methods and compositions provided herein employ a configuration with up to 8 bead types and a 3 color / 2 intensity level (2 fluorescent channels) readout. This embodiment can be compatible with current hardware configurations (e.g., on the Rhapsody scanner).

In some embodiments of the methods and compositions provided herein employing a high-end imager, such as an imager that supports high resolution multi-spectral imaging, it is possible to support a configuration where beads are loaded at very low bead:well ratios (lambda 0.1 or less). In some such embodiments, the tradeoff between bead manufacturing complexity vs. instrument complexity would shift to a 'simple' bead that may not require the UBI for enumeration of beads within a 'bead type' population but would be coupled with a relative high end imager that would provide further value to perform high value image based assays that to complement the sequencing based readouts.

The methods and systems described herein can be used with methods and systems using antibodies associated with (e.g., attached to or conjugated with) oligonucleotides (also referred to herein as AbOs or AbOligos). Some embodiments of using AbOs to determine protein expression profiles in single cells and tracking sample origins have been described in US2018/0088112, and US2018/0346970; the content of each is incorporated by reference herein in its entirety. In some embodiments, the method disclosed herein allows V(D)J profiling of T cells and B cells, 3' targeted, 5' targeted, 3' whole transcriptome amplification (WTA), 5' WTA, protein expression profiling with AbO, and/or sample multiplexing on a single experiment. Methods for determining the sequences of a nucleic acid target (e.g., the V(D)J region of an immune receptor) using 5' barcoding and/or 3' barcoding are described in US2020/0109437; the content of which is incorporated herein by reference in its entirety. Systems, methods, compositions, and kits for molecular barcoding on the 5'-end of a nucleic acid target have been described in, for example, US2019/0338278, the content of which is incorporated herein by reference in its entirety. The systems, methods, compositions, and kits provided herein can, in some embodiments, be employed in concert with the methods to obtain full-length V(D)J information (e.g., by Illumina sequencing on the Rhapsody system) using a combined 5' barcoding and random priming approach described in US20210139970A1, the content of which is incorporated herein by reference in its entirety. The systems, methods, compositions, and kits provided herein can, in some embodiments, be employed in concert with random priming and extension (RPE)-based whole transcriptome analysis methods and compositions have been described in U.S. Patent Application No. 16/677,012; the content of which is incorporated herein by reference in its entirety. The systems, methods, compositions, and kits provided herein can, in some embodiments, be employed in concert with the blocker oligonucleotides described in US20210238661A1, the content of which is incorporated herein by reference in its entirety. Some embodiments of the compositions and methods disclosed herein comprise a first plurality of oligonucleotide barcodes and a second plurality of oligonucleotide barcodes, which have described in US20210371909A1 and content of which is incorporated herein by reference in its entirety. The systems, methods, compositions, and kits provided herein can, in some embodiments, be employed in concert with template switch oligonucleotides comprising a blocking sequence, which, in some embodiments, can reduce the generation of undesirable extension products during library preparation, and which have described in PCT Patent Application Number PCT/US22/75577, filed on August 29, 2022, and entitled "TEMPLATE SWITCH OLIGONUCLEOTIDE (TSO) FOR MRNA 5' ANALYSIS", the content of which is incorporated herein by reference in its entirety.

There are provided, in some embodiments, reference particles. In some embodiments, the reference particle comprises: one or more detectable moieties, or precursors thereof; and a particle indexing oligonucleotide, wherein the particle indexing oligonucleotide comprises a particle type identifier (PTI) and/or a unique particle identifier (UPI).

In some embodiments, the particle type identifier identifies the one or more detectable moieties associated with the reference particle. In some embodiments, the particle type identifier identifies the one or more detectable moieties associated with the reference particle and the density of said one or more detectable moieties associated with the reference particle. In some embodiments, the unique particle identifier is selected from a diverse set of unique particle identifiers. In some embodiments, the diverse set of unique particle identifiers comprises at least about 100 different unique particle identifiers, at least about 1000 different unique particle identifiers, at least about 10000 different unique particle identifiers, or at least about 100000 different unique particle identifiers.

In some embodiments, the one or more detectable moieties is at least about 2, 3, 4, 5, 6, or 7, different detectable moieties. In some embodiments, the detectable moiety comprises an optical moiety, a luminescent moiety, a magnetic moiety, an electrochemically active moiety, a nanoparticle, or a combination thereof. In some embodiments, the luminescent moiety comprises a chemiluminescent moiety, an electroluminescent moiety, a photoluminescent moiety, or a combination thereof. In some embodiments, the photoluminescent moiety comprises a fluorescent moiety, a phosphorescent moiety, or a combination thereof. In some embodiments, the fluorescent moiety comprises a fluorescent dye. In some embodiments, the nanoparticle comprises a quantum dot. In some embodiments, the particle type identifier and/or unique particle identifier is at least about 4 nucleotides in length.

**In** some embodiments, the reference particle comprises a synthetic particle. In some embodiments, the reference particle is disruptable. In some embodiments, the reference particle comprises a bead. In some embodiments, the bead comprises a Sepharose bead, a streptavidin bead, an agarose bead, a magnetic bead, a conjugated bead, a protein A conjugated bead, a protein G conjugated bead, a protein A/G conjugated bead, a protein L conjugated bead, an oligo(dT) conjugated bead, a silica bead, a silica-like bead, an anti-biotin microbead, an anti-fluorochrome microbead, or any combination thereof. In some embodiments, the reference particle comprises a material selected from the group consisting of polydimethylsiloxane (PDMS), polystyrene, glass, polypropylene, agarose, gelatin, hydrogel, paramagnetic, ceramic, plastic, glass, methylstyrene, acrylic polymer, titanium, latex, Sepharose, cellulose, nylon, silicone, and any combination thereof. In some embodiments, the reference particle comprises a disruptable hydrogel particle.

**In** some embodiments, the particle indexing oligonucleotide is 6-60 nucleotides in length. In some embodiments, the particle indexing oligonucleotide is immobilized on the reference particle, partially immobilized on the reference particle, enclosed in the reference particle, partially enclosed in the reference particle, or a combination thereof. In some embodiments, the particle indexing oligonucleotide is associated with the reference particle via one or more cleavable linkers. In some embodiments, the one or more cleavable linkers comprise acid-labile linkers, base-labile linkers, photocleavable linkers, enzyme-cleavable linkers, or any combination thereof. In some embodiments, the particle indexing oligonucleotide comprises a sequence complementary to a capture sequence configured to capture the particle indexing oligonucleotide. In some embodiments, the sequence complementary to the capture sequence comprises a poly(dA) region. In some embodiments, the particle indexing oligonucleotide comprises a particle linker functional group. In some embodiments, the reference particle comprises a particle functional group. In some embodiments, the particle functional group and the particle linker functional group are associated with each other. In some embodiments, the particle linker functional group and the particle functional group are individually selected from the group consisting of C6, biotin, streptavidin, primary amine(s), aldehyde(s), ketone(s), and any combination thereof. In some embodiments, the particle indexing oligonucleotide is associated with the reference particle through a particle linker. In some embodiments, the particle linker comprises a carbon chain, optionally the carbon chain comprises 2-30 carbons. In some embodiments, the particle linker comprises 5' amino modifier C12 (5AmMC 12), or a derivative thereof. In some embodiments, the particle indexing oligonucleotide is 50-500 nucleotides in length. In some embodiments, the particle indexing oligonucleotide is conjugated to the reference particle through a chemical group selected from the group consisting of a UV photocleavable group, a streptavidin, a biotin, an amine, and a combination thereof. In some embodiments, the particle indexing oligonucleotide is non-covalently attached to the particle. In some embodiments, the particle indexing oligonucleotide is double-stranded or single-stranded, and wherein the particle indexing oligonucleotide comprises DNA and/or RNA.

There are provided, in some embodiments, populations of reference particles. In some embodiments, the population of reference particles comprises: two or more reference particles disclosed herein, wherein at least two of the two or more reference particles differ from each other with respect to the identity of the one or more detectable moieties associated with the reference particle and/or the density of said one or more detectable moieties associated with the reference particle. In some embodiments, at least 3, 4, 5, 6, 7, 8, 9, 10, 11, or 12, of the two or more reference particles differ from each other with respect to the one or more detectable moieties associated with the reference particle and/or the density of said one or more detectable moieties on the reference particle.

There are provided, in some embodiments, methods of assigning sequencing data to partitions. In some embodiments, the method comprises: partitioning a plurality of solid supports and a plurality of single cells to the plurality of partitions, wherein the plurality of solid supports each comprise a plurality of oligonucleotide barcodes each comprising a cell label, wherein oligonucleotide barcodes associated with the same solid support comprise the same cell label sequence, and wherein oligonucleotide barcodes associated with different solid supports comprise different cell label sequences; partitioning the reference particles disclosed herein and/or the population of reference particle disclosed herein to the plurality of partitions, barcoding the particle indexing oligonucleotides using the plurality of oligonucleotide barcodes to generate a plurality of barcoded particle indexing oligonucleotides; barcoding the copies of a nucleic acid target from at least one of the plurality of single cells using the plurality of oligonucleotide barcodes to generate a plurality of barcoded nucleic acid molecules; obtaining imaging data of the plurality of partitions to identify the detectable signature of each partition; obtaining sequencing data comprising a plurality of sequencing reads of the barcoded particle indexing oligonucleotides, or products thereof, and the plurality of barcoded nucleic acid molecules, or products thereof; identifying the particle type identifier (PTI) and/or unique particle identifier (UPI) associated with each cell label sequence in the sequencing data; and assigning each of the plurality of sequencing reads to a partition of the plurality of partitions based on the particle type identifier (PTI) and/or unique particle identifier (UPI) associated with each cell label sequence in the sequencing data.

The method can comprise: obtaining phenotypic data of the plurality of single cells; and associating the sequencing data and the phenotypic data of at least one cell of the plurality of single cells based on the particle type identifier (PTI) and/or unique particle identifier (UPI) of at least one barcoded particle indexing oligonucleotide, or product thereof, of the plurality of barcoded particle indexing oligonucleotides, or products thereof, in the sequencing data. In some embodiments, imaging each partition yields phenotypic data. In some embodiments, imaging comprises microscopy, time-lapse imaging microscopy, fluorescence microscopy, multi-photon microscopy, quantitative phase microscopy, surface enhanced Raman spectroscopy, videography, manual visual analysis, automated visual analysis, and combinations thereof.

The method can comprise: associating the plurality of sequencing reads of the barcoded particle indexing oligonucleotides, or products thereof, of each partition, with the detectable signature of each partition. In some embodiments, each partition of the plurality of partitions comprises a unique detectable signature, and wherein the detectable signature of each partition varies depending on the identity of the reference particle(s) in said partitions. In some embodiments, identifying the detectable signature of each partition comprises detecting the emissions of the one or more detectable moieties of each partition, optionally said emissions comprise fluorescence emissions.

In some embodiments, the particle indexing oligonucleotide is configured to be detachable from the reference particle. In some embodiments, the particle indexing oligonucleotide is configured detach from the reference particle during cell lysis. The method can comprise: dissociating the particle indexing oligonucleotide from the reference particle. In some embodiments, dissociating the particle indexing oligonucleotide from the reference particle comprises detaching the partition indexing oligonucleotide from the partition by UV photocleaving, chemical treatment, heating, enzyme treatment, or any combination thereof. In some embodiments, the dissociating occurs after barcoding the particle indexing oligonucleotides. In some embodiments, the dissociating occurs before barcoding the particle indexing oligonucleotides. In some embodiments, the dissociating occurs during cell lysis. In some embodiments, the partitions comprise micro-wells or droplets.

There are provided, in some embodiments, compositions. In some embodiments, the composition comprises: a micro-well array, wherein the micro-well array comprises at least 100 micro-wells, wherein each micro-well has a volume ranging from about 1,000 µm3 to about 786,000 µm3, wherein each micro-well comprises one or more reference particles disclosed herein. The composition can comprise: a cartridge, wherein the cartridge comprises at least one of: an inlet port, an outlet port, a pump, a valve, a vent, a reservoir, a sample collection chamber, a temperature control apparatus, or any combination thereof.

In some embodiments, the composition comprises: a cartridge, wherein the cartridge comprises at least one of: an inlet port, an outlet port, a pump, a valve, a vent, a reservoir, a sample collection chamber, a temperature control apparatus, or any combination thereof, wherein the cartridge comprises a micro-well array, wherein the micro-well array comprises at least 100 micro-wells, wherein each micro-well has a volume ranging from about 1,000 µm3 to about 786,000 µm3, wherein each micro-well comprises one or more reference particles disclosed herein.

The composition can comprise: a buffer. The composition can comprise: one or more reagents for a reverse transcription reaction. The composition can comprise: one or more reagents for an amplification reaction. In some embodiments, the cartridge comprises a transparent window for optical imaging of the at least 100 microwells. The composition can comprise: an imaging system configured to capture and process images of all or a portion of the at least 100 microwells, wherein the imaging system further comprises an illumination subsystem, an imaging subsystem, and a processor. In some embodiments, the imaging system is configured to perform bright-field, dark-field, fluorescence, or quantitative phase imaging.

### Detectable Moieties

In some embodiments, the detectable moiety (e.g., detectable label) comprises an optical moiety, a luminescent moiety, an electrochemically active moiety, a nanoparticle, or a combination thereof. In some embodiments, the luminescent moiety comprises a chemiluminescent moiety, an electroluminescent moiety, a photoluminescent moiety, or a combination thereof. In some embodiments, the photoluminescent moiety comprises a fluorescent moiety, a phosphorescent moiety, or a combination thereof. In some embodiments, the fluorescent moiety comprises a fluorescent dye. In some embodiments, the nanoparticle comprises a quantum dot. In some embodiments, the methods comprise performing a reaction to convert the detectable moiety precursor into the detectable moiety. In some embodiments, performing a reaction to convert the detectable moiety precursor into the detectable moiety comprises contacting the detectable moiety precursor with a substrate. In some such embodiments, contacting the detectable moiety precursor with a substrate yields a detectable byproduct of a reaction between the two molecules.

### Detectable Moiety Properties and Structures

In some embodiments, detectable labels, moieties, or markers can be detectible based on, for example, fluorescence emission, absorbance, fluorescence polarization, fluorescence lifetime, fluorescence wavelength, absorbance wavelength, Stokes shift, light scatter, mass, molecular mass, redox, acoustic, Raman, magnetism, radio frequency, enzymatic reactions (including chemiluminescence and electro- chemiluminescence) or combinations thereof. For example, the label may be a fluorophore, a chromophore, an enzyme, an enzyme substrate, a catalyst, a redox label, a radio label, an acoustic label, a Raman (SERS) tag, a mass tag, an isotope tag (e.g., isotopically pure rare earth element), a magnetic particle, a microparticle, a nanoparticle, an oligonucleotide, or any combination thereof. In some embodiments, the label is a fluorophore (i.e., a fluorescent label, fluorescent dye, etc.). Fluorophores of interest may include but are not limited to dyes suitable for use in analytical applications (e.g., flow cytometry, imaging, etc.) , such as an acridine dye, anthraquinone dyes, arylmethane dyes, diarylmethane dyes (e.g., diphenyl methane dyes), chlorophyll containing dyes, triarylmethane dyes (e.g., triphenylmethane dyes), azo dyes, diazonium dyes, nitro dyes, nitroso dyes, phthalocyanine dyes, cyanine dyes, asymmetric cyanine dyes, quinon-imine dyes, azine dyes, eurhodin dyes, safranin dyes, indamins, indophenol dyes, fluorine dyes, oxazine dye, oxazone dyes, thiazine dyes, thiazole dyes, xanthene dyes, fluorene dyes, pyronin dyes, fluorine dyes, rhodamine dyes, phenanthridine dyes, as well as dyes combining two or more of the aforementioned dyes (e.g., in tandem), polymeric dyes having one or more monomeric dye units and mixtures of two or more of the aforementioned dyes thereof. A large number of dyes are commercially available from a variety of sources, such as, for example, Molecular Probes (Eugene, OR), Dyomics GmbH (Jena, Germany), Sigma-Aldrich (St. Louis, MO), Sirigen, Inc. (Santa Barbara, CA) and Exciton (Dayton, OH). For example, the fluorophore may include 4-acetamido-4'-isothiocyanatostilbene-2,2'disulfonic acid: acridine and derivatives such as acridine, acridine orange, acridine yellow, acridine red, and acridine isothiocyanate; allophycocyanin, phycoerythrin, peridinin-chlorophyll protein, 5-(2'-aminoethyl)aminonaphthalene-1-sulfonic acid (EDANS); 4-amino-N-[3-vinylsulfonyl)phenyl]naphthalimide-3,5 disulfonate (Lucifer Yellow VS); N-(4-anilino-1-naphthyl)maleimide; anthranilamide; Brilliant Yellow: coumarin and derivatives such as coumarin, 7-amino-4-methylcoumarin (AMC, Coumarin 120), 7-amino-4-trifluoromethylcouluarin (Coumaran 151); cyanine and derivatives such as cyanosine, Cy3, Cy3.5, Cy5, Cy5.5, and Cy7; 4',6-diaminidino-2-phenylindole (DAPI); 5', 5"-dibromopyrogallol-sulfonephthalein (Bromopyrogallol Red); 7-diethylamino-3-(4'-isothiocyanatophenyl)-4-methylcoumarin: diethylaminocoumarin; diethylenetriamine pentaacetate; 4,4'-diisothiocyanatodihydro-stilbene-2,2'-disulfonic acid; 4,4'-diisothiocyanatostilbene-2,2'-disulfonic acid: 5-[dimethylamino]naphthalene-1-sulfonyl chloride (DNS, dansyl chloride); 4-(4'-dimethylaminophenylazo)benzoic acid (DABCYL); 4-dimethylaminophenylazophenyl-4'-isothiocyanate (DABITC); eosin and derivatives such as eosin and eosin isothiocyanate; erythrosin and derivatives such as erythrosin B and erythrosin isothiocyanate; ethidium: fluorescein and derivatives such as 5- carboxyfluorescein (FAM), 5-(4,6-dichlorotriazin-2-yl)aminofluorescein (DTAF), 2'7'-dimethoxy-4'5'-dichloro-6-carboxyfluorescein (JOE), fluorescein isothiocyanate (FITC), fluorescein chlorotriazinyl, naphthofluorescein, and QFITC (XRITC); fluorescamine; IR144; IR1446; Green Fluorescent Protein (GFP); Reef Coral Fluorescent Protein (RCFP); Lissamine^{™}; Lissamine rhodamine, Lucifer yellow; Malachite Green isothiocyanate; 4-methylumbelliferone; ortho cresolphthalein; nitrotyrosine; pararosaniline: Nile Red: Oregon Green; Phenol Red; B-phycoerythrin; o-phthaldialdehyde: pyrene and derivatives such as pyrene, pyrene butyrate and succinimidyl 1-pyrene butyrate; Reactive Red 4 (Cibacron^{™} Brilliant Red 3B-A); rhodamine and derivatives such as 6-carboxy-X-rhodamine (ROX), 6-carboxyrhodamine (R6G), 4,7-dichlororhodamine lissamine, rhodamine B sulfonyl chloride, rhodamine (Rhod), rhodamine B, rhodamine 123, rhodamine X isothiocyanate, sulforhodamine B, sulforhodamine 101, sulfonyl chloride derivative of sulforhodamine 101 (Texas Red), N,N,N',N'-tetramethyl-6-carboxyrhodamine (TAMRA), tetramethyl rhodamine, and tetramethyl rhodamine isothiocyanate (TRITC); riboflavin; rosolic acid and terbium chelate derivatives: xanthene: dye-conjugated polymers (i.e., polymer-attached dyes) such as fluorescein isothiocyanate-dextran as well as dyes combining two or more dyes (e.g., in tandem), polymeric dyes having one or more monomeric dye units and mixtures of two or more of the aforementioned dyes or combinations thereof.

The detectable moiety can be selected from a group of spectrally-distinct detectable moieties. Spectrally-distinct detectable moieties include detectable moieties with distinguishable emission spectra even if their emission spectral may overlap. Non-limiting examples of detectable moieties include Xanthene derivatives: fluorescein, rhodamine, Oregon green, eosin, and Texas red; Cyanine derivatives: cyanine, indocarbocyanine, oxacarbocyanine, thiacarbocyanine, and merocyanine; Squaraine derivatives and ring-substituted squaraines, including Seta, SeTau, and Square dyes; Naphthalene derivatives (dansyl and prodan derivatives); Coumarin derivatives; oxadiazole derivatives: pyridyloxazole, nitrobenzoxadiazole and benzoxadiazole; Anthracene derivatives: anthraquinones, including DRAQ5, DRAQ7 and CyTRAK Orange; Pyrene derivatives: cascade blue: Oxazine derivatives: Nile red, Nile blue, cresyl violet, oxazine 170; Acridine derivatives: proflavin, acridine orange, acridine yellow; Arylmethine derivatives: auramine, crystal violet, malachite green; and Tetrapyrrole derivatives: porphin, phthalocyanine, bilirubin. Other non-limiting examples of detectable moieties include Hydroxycoumarin, Aminocoumarin, Methoxycoumarin, Cascade Blue, Pacific Blue, Pacific Orange, Lucifer yellow, NBD, R-Phycoerythrin (PE), PE-Cy5 conjugates, PE-Cy7 conjugates, Red 613, PerCP, TruRed, FluorX, Fluorescein, BODIPY-FL, Cy2, Cy3, Cy3B, Cy3.5, Cy5, Cy5.5, Cy7, TRITC, X-Rhodamine, Lissamine Rhodamine B, Texas Red, Allophycocyanin (APC), APC-Cy7 conjugates, Hoechst 33342, DAPI, Hoechst 33258, SYTOX Blue, Chromomycin A3, Mithramycin, YOYO-1, Ethidium Bromide, Acridine Orange, SYTOX Green, TOTO-1, TO-PRO-1, TO-PRO: Cyanine Monomer, Thiazole Orange, CyTRAK Orange, Propidium Iodide (PI), LDS 751, 7-AAD, SYTOX Orange, TOTO-3, TO-PRO-3, DRAQ5, DRAQ7, Indo-1, Fluo-3, Fluo-4, DCFH, DHR, and SNARF.

In some embodiments, fluorophores of interest may include, but are not limited to, dyes suitable for use in analytical applications (e.g., flow cytometry, imaging, etc.), such as an acridine dye, anthraquinone dyes, arylmethane dyes, diarylmethane dyes (e.g., diphenyl methane dyes), chlorophyll containing dyes, triarylmethane dyes (e.g., triphenylmethane dyes), azo dyes, diazonium dyes, nitro dyes, nitroso dyes, phthalocyanine dyes, cyanine dyes, asymmetric cyanine dyes, quinon-imine dyes, azine dyes, eurhodin dyes, safranin dyes, indamins, indophenol dyes, fluorine dyes, oxazine dye, oxazone dyes, thiazine dyes, thiazole dyes, xanthene dyes, fluorene dyes, pyronin dyes, fluorine dyes, rhodamine dyes, phenanthridine dyes, as well as dyes combining two or more dyes (e.g., in tandem) as well as polymeric dyes having one or more monomeric dye units, as well as mixtures of two or more dyes thereof. For example, the fluorophore may be 4- acetamido-4'-isothiocyanatostilbene-2,2'disulfonic acid; acridine and derivatives such as acridine, acridine orange, acrindine yellow, acridine red, and acridine isothiocyanate; allophycocyanin, phycoerythrin, peridinin-chlorophyll protein, 5-(2'- aminoethyl)aminonaphthalene-1-sulfonic acid (EDANS); 4-amino-N-[3-vinylsulfonyl)phenyl]naphthalimide-3,5 disulfonate (Lucifer Yellow VS); N-(4-anilino-1-naphthyl)maleimide; anthranilamide; Brilliant Yellow: coumarin and derivatives such as coumarin, 7-amino-4-methylcoumarin (AMC, Coumarin 120), 7-amino-4-trifluoromethylcouluarin (Coumaran 151); cyanine and derivatives such as cyanosine, Cy3, Cy5, Cy5.5, and Cy7; 4',6-diaminidino-2-phenylindole (DAPI); 5',5"-dibromopyrogallol-sulfonephthalein (Bromopyrogallol Red); 7-diethylamino-3-(4'-isothiocyanatophenyl)-4-methylcoumarin; diethylaminocoumarin; diethylenetriamine pentaacetate; 4,4'-diisothiocyanatodihydro-stilbene-2,2'-disulfonic acid; 4,4'- diisothiocyanatostilbene-2,2'-disulfonic acid; 5-[dimethylamino]naphthalene-1-sulfonyl chloride (DNS, dansyl chloride); 4-(4'-dimethylaminophenylazo)benzoic acid (DABCYL); 4-dimethylaminophenylazophenyl-4'-isothiocyanate (DABITC); eosin and derivatives such as eosin and eosin isothiocyanate; erythrosin and derivatives such as erythrosin B and erythrosin isothiocyanate; ethidium; fluorescein and derivatives such as 5- carboxyfluorescein (FAM), 5-(4,6-dichlorotriazin-2-yl)aminofluorescein (DTAF), 2'7'-dimethoxy-4'5'-dichloro-6-carboxyfluorescein (JOE), fluorescein isothiocyanate (FITC), fluorescein chlorotriazinyl, naphthofluorescein, and QFITC (XRITC); fluorescamine: IR144: IR1446: Green Fluorescent Protein (GFP); Reef Coral Fluorescent Protein (RCFP): Lissamine^{™}; Lissamine rhodamine, Lucifer yellow; Malachite Green isothiocyanate; 4-methylumbelliferone; ortho cresolphthalein; nitrotyrosine; pararosaniline; Nile Red: Oregon Green; Phenol Red; B-phycoerythrin; o- phthaldialdehyde; pyrene and derivatives such as pyrene, pyrene butyrate and succinimidyl 1-pyrene butyrate; Reactive Red 4 (Cibacron^{™} Brilliant Red 3B-A); rhodamine and derivatives such as 6-carboxy-X-rhodamine (ROX), 6-carboxyrhodamine (R6G), 4,7-dichlororhodamine lissamine, rhodamine B sulfonyl chloride, rhodamine (Rhod), rhodamine B, rhodamine 123, rhodamine X isothiocyanate, sulforhodamine B, sulforhodamine 101, sulfonyl chloride derivative of sulforhodamine 101 (Texas Red), N,N,N',N'-tetramethyl-6-carboxyrhodamine (TAMRA), tetramethyl rhodamine, and tetramethyl rhodamine isothiocyanate (TRITC); riboflavin; rosolic acid and terbium chelate derivatives; xanthene; dye-conjugated polymers (i.e., polymer-attached dyes) such as fluorescein isothiocyanate-dextran as well as dyes combining two or more of the aforementioned dyes (e.g., in tandem), polymeric dyes having one or more monomeric dye units and mixtures of two or more of the aforementioned dyes thereof.

The group of spectrally distinct detectable moieties can, for example, include five different fluorophores, five different chromophores, a combination of five fluorophores and chromophores, a combination of four different fluorophores and a non-fluorophore, a combination of four chromophores and a non-chromophore, or a combination of four fluorophores and chromophores and a non-fluorophore non-chromophore. In some embodiments, the detectable moieties can be one of 1, 2, 3, 4, 5, 6, 7, 8, 9, 10, 20, 30, 40, 50, 60, 70, 80, 90, 100, 1000, 2000, 3000, 4000, 5000, 6000, 7000, 8000, 9000, 10000, or a number or a range between any two of these values, of spectrally-distinct moieties.

The excitation wavelength of the detectable moieties can vary, for example be, or be about, 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000 nanometers, or a number or a range between any two of these values. The emission wavelength of the detectable moieties can also vary, for example be, or be about, 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000 nanometers, or a number or a range between any two of these values.

The molecular weights of the detectable moieties can vary, for example be, or be about, 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000 Daltons (Da), or a number or a range between any two of these values. The molecular weights of the detectable moieties can also vary, for example be, or be about, 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000 kilo Daltons (kDa), or a number or a range between any two of these values.

### Polymeric Dyes

In some instances, the fluorophore (i.e., dye) is a fluorescent polymeric dye. Fluorescent polymeric dyes that find use in the subject methods and systems can vary. In some instances of the method, the polymeric dye includes a conjugated polymer.

Conjugated polymers (CPs) are characterized by a delocalized electronic structure which includes a backbone of altemating unsaturated bonds (e.g., double and/or triple bonds) and saturated (e.g., single bonds) bonds, where π-electrons can move from one bond to the other. As such, the conjugated backbone may impart an extended linear structure on the polymeric dye, with limited bond angles between repeat units of the polymer. For example, proteins and nucleic acids, although also polymeric, in some cases do not form extended-rod structures but rather fold into higher-order three- dimensional shapes. In addition, CPs may form "rigid-rod" polymer backbones and experience a limited twist (e.g., torsion) angle between monomer repeat units along the polymer backbone chain. In some instances, the polymeric dye includes a CP that has a rigid rod structure. As summarized above, the structural characteristics of the polymeric dyes can have an effect on the fluorescence properties of the molecules.

Any convenient polymeric dye may be utilized in the subject methods and systems. In some instances, a polymeric dye is a multichromophore that has a structure capable of harvesting light to amplify the fluorescent output of a fluorophore. In some instances, the polymeric dye is capable of harvesting light and efficiently converting it to emitted light at a longer wavelength. In some embodiments, the polymeric dye has a light-harvesting multichromophore system that can efficiently transfer energy to nearby luminescent species (e.g., a "signaling chromophore"). Mechanisms for energy transfer include, for example, resonant energy transfer (e.g., Forster (or fluorescence) resonance energy transfer, FRET), quantum charge exchange (Dexter energy transfer) and the like. In some instances, these energy transfer mechanisms are relatively short range; that is, close proximity of the light harvesting multichromophore system to the signaling chromophore provides for efficient energy transfer. Under conditions for efficient energy transfer, amplification of the emission from the signaling chromophore occurs when the number of individual chromophores in the light harvesting multichromophore system is large; that is, the emission from the signaling chromophore is more intense when the incident light (the "excitation light") is at a wavelength which is absorbed by the light harvesting multichromophore system than when the signaling chromophore is directly excited by the pump light.

The multichromophore may be a conjugated polymer. Conjugated polymers (CPs) are characterized by a delocalized electronic structure and can be used as highly responsive optical reporters for chemical and biological targets. Because the effective conjugation length is substantially shorter than the length of the polymer chain, the backbone contains a large number of conjugated segments in close proximity. Thus, conjugated polymers are efficient for light harvesting and enable optical amplification via energy transfer.

In some instances the polymer may be used as a direct fluorescent reporter, for example fluorescent polymers having high extinction coefficients, high brightness, etc. In some instances, the polymer may be used as a strong chromophore where the color or optical density is used as an indicator.

Polymeric dyes of interest include, but are not limited to, those dyes described by Gaylord et al. in US Publication Nos. 20040142344, 20080293164, 20080064042, 20100136702, 20110256549, 20120028828, 20120252986, 20130190193 and 20160025735 the disclosures of which are herein incorporated by reference in their entirety; and Gaylord et al., J. Am. Chem. Soc., 2001, 123 (26), pp 6417-6418: Feng et al., Chem. Soc. Rev., 2010,39, 2411-2419; and Traina et al., J. Am. Chem. Soc., 2011, 133 (32), pp 12600-12607, the disclosures of which are herein incorporated by reference in their entirety.

In some embodiments, the polymeric dye includes a conjugated polymer including a plurality of first optically active units forming a conjugated system, having a first absorption wavelength (e.g., as described herein) at which the first optically active units absorb light to form an excited state. The conjugated polymer (CP) may be polycationic, polyanionic and/or a charge-neutral conjugated polymer.

The CPs may be water soluble for use in biological samples. Any convenient substituent groups may be included in the polymeric dyes to provide for increased water-solubility, such as a hydrophilic substituent group, e.g., a hydrophilic polymer, or a charged substituent group, e.g., groups that are positively or negatively charged in an aqueous solution, e.g., under physiological conditions. Any convenient water-soluble groups (WSGs) may be utilized in the subject light harvesting multichromophores. The term "water-soluble group" refers to a functional group that is well solvated in aqueous environments and that imparts improved water solubility, to the molecules to which it is attached. In some embodiments, a WSG increases the solubility, of the multichromophore in a predominantly aqueous solution (e.g., as described herein), as compared to a multichromophore which lacks the WSG. The water-soluble groups may be any convenient hydrophilic group that is well solvated in aqueous environments. In some embodiments, the hydrophilic water-soluble group is charged, e.g., positively or negatively charged or zwitterionic. In some embodiments, the hydrophilic water-soluble group is a neutral hydrophilic group. In some embodiments, the WSG is a hydrophilic polymer, e.g., a polyethylene glycol, a cellulose, a chitosan, or a derivative thereof.

As used herein, the terms "polyethylene oxide", "PEO", "polyethylene glycol" and "PEG" are used interchangeably and refer to a polymer including a chain described by the formula -(CH₂ - CH₂ - O-)ₙ- or a derivative thereof. In some embodiments, "n" is 5000 or less, such as 1000 or less, 500 or less, 200 or less, 100 or less, 50 or less, 40 or less, 30 or less, 20 or less, 15 or less, such as 5 to 15, or 10 to 15. It is understood that the PEG polymer may be of any convenient length and may include a variety of terminal groups, including but not limited to, alkyl, aryl, hydroxyl, amino, acyl, acyloxy, and amido terminal groups. Functionalized PEGs that may be adapted for use in the subject multichromophores include those PEGs described by S. Zalipsky in "Functionalized poly(ethylene glycol) for preparation of biologically relevant conjugates", Bioconjugate Chemistry 1995, 6 (2), 150-165. Water soluble groups of interest include, but are not limited to, carboxylate, phosphonate, phosphate, sulfonate, sulfate, sulfinate , ester, polyethylene glycols (PEG) and modified PEGs, hydroxyl, amine, ammonium, guanidinium, polyamine and sulfonium, polyalcohols, straight chain or cyclic saccharides, primary, secondary, tertiary, or quaternary amines and polyamines, phosphonate groups, phosphinate groups, ascorbate groups, glycols, including, polyethers, -COOM', -SO₃M', -PO₃M', -NR₃⁺ , Y', (CH₂CH₂O)ₚR and mixtures thereof, where Y' can be any halogen, sulfate, sulfonate, or oxygen containing anion, p can be 1 to 500, each R can be independently H or an alkyl (such as methyl) and M' can be a cationic counterion or hydrogen, - (CH₂CH₂O)_{yy}CH₂CH₂XR^{yy}, -(CH₂CH₂O)_{yy}CH₂CH₂X-, -X(CH₂CH₂O)_{yy}CH₂CH₂-, glycol, and polyethylene glycol, wherein yy is selected from 1 to 1000, X is selected from O, S, and NR^{ZZ}, and R^{ZZ} and R^{YY} are independently selected from H and C1-3 alkyl.

The polymeric dye may have any convenient length. In some embodiments, the particular number of monomeric repeat units or segments of the polymeric dye may fall within the range of 2 to 500,000, such as 2 to 100,000, 2 to 30,000, 2 to 10,000, 2 to 3,000 or 2 to 1,000 units or segments, or such as 100 to 100,000, 200 to 100,000, or 500 to 50,000 units or segments. In some embodiments, the number of monomeric repeat units or segments of the polymeric dye is within the range of 2 to 1000 units or segments, such as from 2 to 750 units or segments, such as from 2 to 500 units or segments, such as from 2 to 250 units or segment, such as from 2 to 150 units or segment, such as from 2 to 100 units or segments, such as from 2 to 75 units or segments, such as from 2 to 50 units or segments and including from 2 to 25 units or segments.

The polymeric dyes may be of any convenient molecular weight (MW). In some embodiments, the MW of the polymeric dye may be expressed as an average molecular weight. In some instances, the polymeric dye has an average molecular weight of from 500 to 500,000, such as from 1,000 to 100,000, from 2,000 to 100,000, from 10,000 to 100,000 or even an average molecular weight of from 50,000 to 100,000. In some embodiments, the polymeric dye has an average molecular weight of 70,000.

In some embodiments, the polymeric dye includes the following structure: 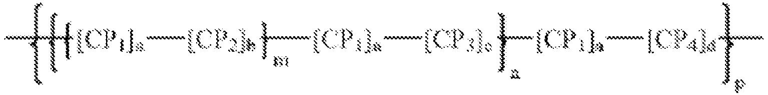 wherein CP₁, CP₂, CP₃ and CP₄ are independently a conjugated polymer segment or an oligomeric structure, wherein one or more of CP₁, CP₂, CP₃ and CP₄ are bandgap-modifying n-conjugated repeat units.

In some embodiments, the conjugated polymer is a polyfluorene conjugated polymer, a polyphenylene vinylene conjugated polymer, a polyphenylene ether conjugated polymer, a polyphenylene polymer, among other types of conjugated polymers.

In some instances, the polymeric dye includes the following structure: 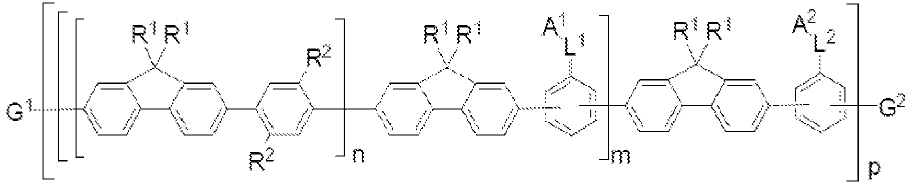 wherein each R¹ is independently a solubilizing group or a linker-dye; L¹ and L² are optional linkers; each R² is independently H or an aryl substituent; each A¹ and A² is independently H, an aryl substituent or a fluorophore; G¹ and G² are each independently selected from the group consisting of a terminal group, a πconjugated segment, a linker and a linked specific binding member; each n and each m are independently 0 or an integer from 1 to 10,000; and p is an integer from 1 to 100,000. Solubilizing groups of interest include, but is not limited to a water-soluble functional group such as a hydrophilic polymer (e.g., polyalkylene oxide, cellulose, chitosan, etc.), as well as alkyl, aryl and heterocycle groups further substituted with a hydrophilic group such as a polyalkylene oxide (e.g., polyethylglycol including a PEG of 2-20 units), an ammonium, a sulphonium, a phosphonium, as well has a charged (positively, negatively or zwitterionic) hydrophilic water soluble group and the like.

**In** some embodiments, the polymeric dye includes, as part of the polymeric backbone, a conjugated segment having one of the following structures: 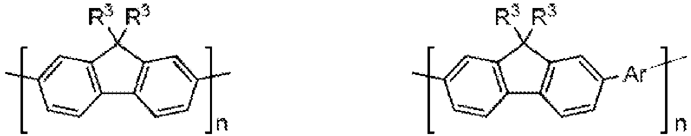 where each R³ is independently an optionally substituted wat -soluble functional group such as a hydrophilic polymer (e.g., polyalkylene oxide, cellulose, chitosan, etc.) or an alkyl or aryl group further substituted with a hydrophilic group such as a polyalkylene oxide (e.g., polyethylglycol including a PEG of 2-20 units), an ammonium, a sulphonium, a phosphonium, as well has a charged (positively, negatively or zwitterionic) hydrophilic water soluble group; Ar is an optionally substituted aryl or heteroaryl group; and n is 1 to 10000. In some embodiments, R3 is an optionally substituted alkyl group. In some embodiments, R³ is an optionally substituted aryl group. In some embodiments, R³ is substituted with a polyethyleneglycol, a dye, a chemoselective functional group or a specific binding moiety. In some embodiments, Ar is substituted with a polyethyleneglycol, a dye, a chemoselective functional group or a specific binding moiety.

In some embodiments, the polymeric dye includes the following structure: 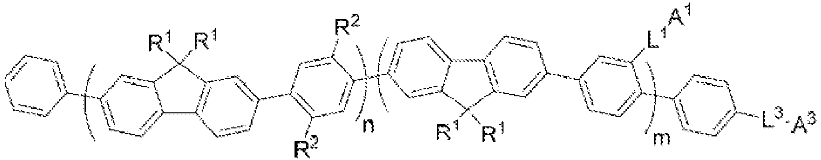 wherein each R¹ is a solubilizing group or a linker dye group; each R²is independently H or an aryl substituent; L₁ and L₂ are optional linkers; each A1 and A3 are independently H, a fluorophore, a functional group or a specific binding moiety (e.g., an antibody); and n and m are each independently 0 to 10000, wherein n+m>1.

The polymeric dye may have one or more desirable spectroscopic properties, such as a particular absorption maximum wavelength, a particular emission maximum wavelength, extinction coefficient, quantum yield, and the like (see e.g., Chattopadhyay et al., "Brilliant violet fluorophores: A new class of ultrabright fluorescent compounds for immunofluorescence experiments." Cytometry Part A, 81A(6), 456-466, 2012).

**In** some embodiments, the polymeric dye has an absorption curve between 280 and 850 nm. In some embodiments, the polymeric dye has an absorption maximum in the range 280 and 850 nm. In some embodiments, the polymeric dye absorbs incident light having a wavelength in the range between 280 and 850 nm, where specific examples of absorption maxima of interest include, but are not limited to: 348nm, 355nm, 405nm, 407nm, 445nm, 488nm, 640nm and 652nm. In some embodiments, the polymeric dye has an absorption maximum wavelength in a range selected from the group consisting of 280-310nm, 305-325nm, 320-350nm, 340-375nm, 370-425nm, 400- 450nm, 440-500nm, 475-550nm, 525-625nm, 625-675nm and 650-750nm. In some embodiments, the polymeric dye has an absorption maximum wavelength of 348nm, 355nm, 405nm, 407nm, 445nm, 488nm, 640nm, 652nm, or a range between any two of these values.

In some embodiments, the polymeric dye has an emission maximum wavelength ranging from 400 to 850 nm, such as 415 to 800 nm, where specific examples of emission maxima of interest include, but are not limited to: 395 nm, 421nm, 445nm, 448nm, 452nm, 478nm, 480nm, 485nm, 491nm, 496nm, 500nm, 510nm, 515nm, 519nm, 520nm, 563nm, 570nm, 578nm, 602nm, 612nm, 650nm, 661nm, 667nm, 668nm, 678nm, 695nm, 702nm, 711nm, 719nm, 737nm, 785nm, 786nm, 805nm. In some embodiments, the polymeric dye has an emission maximum wavelength in a range selected from the group consisting of 380-400nm, 410-430nm, 470-490nm, 490-510nm, 500-520nm, 560-580nm, 570-595nm, 590-610nm, 610-650nm, 640-660nm, 650-700nm, 700-720nm, 710-750nm, 740-780nm and 775-795nm. In some embodiments, the polymeric dye has an emission maximum of 395nm, 421nm, 478nm, 480nm, 485nm, 496nm, 510nm, 570nm, 602nm, 650nm, 711nm, 737nm, 750nm, 786nm, or a range of any two of these values. In some embodiments, the polymeric dye has an emission maximum wavelength of 421nm ± 5nm, 510nm ± 5nm, 570nm ± 5nm, 602nm ± 5nm, 650nm ± 5nm, 711nm ± 5nm, 786nm ± 5nm, or a range of any two of these values. In some embodiments, the polymeric dye has an emission maximum selected from the group consisting of 421nm, 510nm, 570nm, 602nm, 650nm, 711nm and 786nm.

In some embodiments, the polymeric dye has an extinction coefficient of 1 x 106 cm- 1M-1 or more, such as 2 x 10⁶ cm⁻¹M⁻¹ or more, 2.5 x 10⁶ cm⁻¹M⁻¹ or more, 3 x 10⁶ cm⁻¹M⁻¹ or more, 4 x 10⁶ cm⁻¹M⁻¹ or more, 5 x 10⁶ cm⁻¹M⁻¹ or more, 6 x 10⁶ cm⁻¹M⁻¹ or more, 7 x 10⁶ cm⁻¹M⁻¹ or more, or 8 x 10⁶ cm⁻¹M⁻¹ or more. In some embodiments, the polymeric dye has a quantum yield of 0.05 or more, such as 0.1 or more, 0.15 or more, 0.2 or more, 0.25 or more, 0.3 or more, 0.35 or more, 0.4 or more, 0.45 or more, 0.5 or more, 0.6 or more, 0.7 or more, 0.8 or more, 0.9 or more, 0.95 or more, 0.99 or more and including 0.999 or more. For example, the quantum yield of polymeric dyes of interest may range from 0.05 to 1, such as from 0.1 to 0.95, such as from 0.15 to 0.9, such as from 0.2 to 0.85, such as from 0.25 to 0.75, such as from 0.3 to 0.7 and including a quantum yield of from 0.4 to 0.6. In some embodiments, the polymeric dye has a quantum yield of 0.1 or more. In some embodiments, the polymeric dye has a quantum yield of 0.3 or more. In some embodiments, the polymeric dye has a quantum yield of 0.5 or more. In some embodiments, the polymeric dye has a quantum yield of 0.6 or more. In some embodiments, the polymeric dye has a quantum yield of 0.7 or more. In some embodiments, the polymeric dye has a quantum yield of 0.8 or more. In some embodiments, the polymeric dye has a quantum yield of 0.9 or more. In some embodiments, the polymeric dye has a quantum yield of 0.95 or more. In some embodiments, the polymeric dye has an extinction coefficient of 1 x 10⁶ or more and a quantum yield of 0.3 or more. In some embodiments, the polymeric dye has an extinction coefficient of 2 x 106 or more and a quantum yield of 0.5 or more.

### Terminology

In at least some of the previously described embodiments, one or more elements used in an embodiment can interchangeably be used in another embodiment unless such a replacement is not technically feasible. It will be appreciated by those skilled in the art that various other omissions, additions and modifications may be made to the methods and structures described above without departing from the scope of the claimed subject matter. All such modifications and changes are intended to fall within the scope of the subject matter, as defined by the appended claims.

One skilled in the art will appreciate that, for this and other processes and methods disclosed herein, the functions performed in the processes and methods can be implemented in differing order. Furthermore, the outlined steps and operations are only provided as examples, and some of the steps and operations can be optional, combined into fewer steps and operations, or expanded into additional steps and operations without detracting from the essence of the disclosed embodiments.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity. As used in this specification and the appended claims, the singular forms "a," "an," and "the" include plural references unless the context clearly dictates otherwise. Any reference to "or" herein is intended to encompass "and/or" unless otherwise stated.

It will be understood by those within the art that, in general, terms used herein, and especially in the appended claims (e.g., bodies of the appended claims) are generally intended as "open" terms (*e.g.,* the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (*e.g.,* "a" and/or "an" should be interpreted to mean "at least one" or "one or more"); the same holds true for the use of definite articles used to introduce claim recitations. In addition, even if a specific number of an introduced claim recitation is explicitly recited, those skilled in the art will recognize that such recitation should be interpreted to mean at least the recited number (*e.g.,* the bare recitation of "two recitations," without other modifiers, means at least two recitations, or two or more recitations). Furthermore, in those instances where a convention analogous to "at least one of A, B, and C, etc." is used, in general such a construction is intended in the sense one having skill in the art would understand the convention (*e.g.,* " a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (*e.g.,* " a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, such as in terms of providing a written description, all ranges disclosed herein also encompass any and all possible sub-ranges and combinations of sub-ranges thereof. Any listed range can be easily recognized as sufficiently describing and enabling the same range being broken down into at least equal halves, thirds, quarters, fifths, tenths, etc. As a non-limiting example, each range discussed herein can be readily broken down into a lower third, middle third and upper third, etc. As will also be understood by one skilled in the art all language such as "up to," "at least," "greater than," "less than," and the like include the number recited and refer to ranges which can be subsequently broken down into sub-ranges as discussed above. Finally, as will be understood by one skilled in the art, a range includes each individual member. Thus, for example, a group having 1-3 articles refers to groups having 1, 2, or 3 articles. Similarly, a group having 1-5 articles refers to groups having 1, 2, 3, 4, or 5 articles, and so forth.

From the foregoing, it will be appreciated that various embodiments of the present disclosure have been described herein for purposes of illustration, and that various modifications may be made without departing from the scope and spirit of the present disclosure. Accordingly, the various embodiments disclosed herein are not intended to be limiting, with the true scope and spirit being indicated by the following claims.

### The invention also provides the following numbered embodiments:

1. A reference particle, comprising:
   one or more detectable moieties, or precursors thereof; and
   a particle indexing oligonucleotide, wherein the particle indexing oligonucleotide comprises a particle type identifier (PTI) and/or a unique particle identifier (UPI).
2. The reference particle of embodiment 1, wherein the particle type identifier identifies the one or more detectable moieties associated with the reference particle.
3. The reference particle of any one of embodiments 1-2, wherein the particle type identifier identifies the one or more detectable moieties associated with the reference particle and the density of said one or more detectable moieties associated with the reference particle.
4. The reference particle of any one of embodiments 1-3, wherein the unique particle identifier is selected from a diverse set of unique particle identifiers.
5. The reference particle of any one of embodiments 1-4, wherein the diverse set of unique particle identifiers comprises at least about 100 different unique particle identifiers, at least about 1000 different unique particle identifiers, at least about 10000 different unique particle identifiers, or at least about 100000 different unique particle identifiers.
6. The reference particle of any one of embodiments 1-5, wherein the one or more detectable moieties is at least about 2, 3, 4, 5, 6, or 7, different detectable moieties.
7. The reference particle of any one of embodiments 1-6, wherein the detectable moiety comprises an optical moiety, a luminescent moiety, a magnetic moiety, an electrochemically active moiety, a nanoparticle, or a combination thereof.
8. The reference particle of any one of embodiments 1-7, wherein the luminescent moiety comprises a chemiluminescent moiety, an electroluminescent moiety, a photoluminescent moiety, or a combination thereof.
9. The reference particle of any one of embodiments 1-8, wherein the photoluminescent moiety comprises a fluorescent moiety, a phosphorescent moiety, or a combination thereof.
10. The reference particle of any one of embodiments 1-9, wherein the fluorescent moiety comprises a fluorescent dye.
11. The reference particle of any one of embodiments 1-10, wherein the nanoparticle comprises a quantum dot.
12. The reference particle of any one of embodiments 1-11, wherein the particle type identifier and/or unique particle identifier is at least about 4 nucleotides in length.
13. The reference particle of any one of embodiments 1-12, wherein the particle indexing oligonucleotide is 6-60 nucleotides in length.
14. The reference particle of any one of embodiments 1-13, wherein the particle indexing oligonucleotide is immobilized on the reference particle, partially immobilized on the reference particle, enclosed in the reference particle, partially enclosed in the reference particle, or a combination thereof.
15. The reference particle of any one of embodiments 1-14, wherein the reference particle comprises a synthetic particle.
16. The reference particle of any one of embodiments 1-15, wherein the reference particle is disruptable.
17. The reference particle of any one of embodiments 1-16, wherein the reference particle comprises a bead.
18. The reference particle of any one of embodiments 1-17, wherein the bead comprises a Sepharose bead, a streptavidin bead, an agarose bead, a magnetic bead, a conjugated bead, a protein A conjugated bead, a protein G conjugated bead, a protein A/G conjugated bead, a protein L conjugated bead, an oligo(dT) conjugated bead, a silica bead, a silica-like bead, an anti-biotin microbead, an anti-fluorochrome microbead, or any combination thereof.
19. The reference particle of any one of embodiments 1-18, wherein the reference particle comprises a material selected from the group consisting of polydimethylsiloxane (PDMS), polystyrene, glass, polypropylene, agarose, gelatin, hydrogel, paramagnetic, ceramic, plastic, glass, methylstyrene, acrylic polymer, titanium, latex, Sepharose, cellulose, nylon, silicone, and any combination thereof.
20. The reference particle of any one of embodiments 1-19, wherein the reference particle comprises a disruptable hydrogel particle.
21. The reference particle of any one of embodiments 1-20, wherein the particle indexing oligonucleotide is associated with the reference particle via one or more cleavable linkers.
22. The reference particle of any one of embodiments 1-21, wherein the one or more cleavable linkers comprise acid-labile linkers, base-labile linkers, photocleavable linkers, enzyme-cleavable linkers, or any combination thereof.
23. The reference particle of any one of embodiments 1-22, wherein the particle indexing oligonucleotide comprises a sequence complementary to a capture sequence configured to capture the particle indexing oligonucleotide.
24. The reference particle of any one of embodiments 1-23, wherein the sequence complementary to the capture sequence comprises a poly(dA) region.
25. The reference particle of any one of embodiments 1-24,
   wherein the particle indexing oligonucleotide comprises a particle linker functional group,
   wherein the reference particle comprises a particle functional group, and
   wherein the particle functional group and the particle linker functional group are associated with each other.
26. The reference particle of any one of embodiments 1-25, wherein the particle linker functional group and the particle functional group are individually selected from the group consisting of C6, biotin, streptavidin, primary amine(s), aldehyde(s), ketone(s), and any combination thereof.
27. The reference particle of any one of embodiments 1-26, wherein the particle indexing oligonucleotide is associated with the reference particle through a particle linker.
28. The reference particle of any one of embodiments 1-27, wherein the particle linker comprises a carbon chain, optionally the carbon chain comprises 2-30 carbons.
29. The reference particle of any one of embodiments 1-28, wherein the particle linker comprises 5' amino modifier C12 (5AmMC12), or a derivative thereof.
30. The reference particle of any one of embodiments 1-29, wherein the particle indexing oligonucleotide is 50-500 nucleotides in length.
31. The reference particle of any one of embodiments 1-30, wherein the particle indexing oligonucleotide is conjugated to the reference particle through a chemical group selected from the group consisting of a UV photocleavable group, a streptavidin, a biotin, an amine, and a combination thereof.
32. The reference particle of any one of embodiments 1-31, wherein the particle indexing oligonucleotide is non-covalently attached to the particle.
33. The reference particle of any one of embodiments 1-32, wherein the particle indexing oligonucleotide is double-stranded or single-stranded, and wherein the particle indexing oligonucleotide comprises DNA and/or RNA.
34. A population of reference particles, comprising:
   two or more reference particles of any one of embodiments 1-33,
   wherein at least two of the two or more reference particles differ from each other with respect to the identity of the one or more detectable moieties associated with the reference particle and/or the density of said one or more detectable moieties associated with the reference particle.
35. The population of claim 34, wherein at least 3, 4, 5, 6, 7, 8, 9, 10, 11, or 12, of the two or more reference particles differ from each other with respect to the one or more detectable moieties associated with the reference particle and/or the density of said one or more detectable moieties on the reference particle.
36. A method of assigning sequencing data to partitions, comprising:
   partitioning a plurality of solid supports and a plurality of single cells to the plurality of partitions, wherein the plurality of solid supports each comprise a plurality of oligonucleotide barcodes each comprising a cell label, wherein oligonucleotide barcodes associated with the same solid support comprise the same cell label sequence, and wherein oligonucleotide barcodes associated with different solid supports comprise different cell label sequences;
   partitioning the reference particles of any one of embodiments 1-33 and/or the population of reference particle of any one of embodiments 34-35 to the plurality of partitions,
   barcoding the particle indexing oligonucleotides using the plurality of oligonucleotide barcodes to generate a plurality of barcoded particle indexing oligonucleotides;
   barcoding the copies of a nucleic acid target from at least one of the plurality of single cells using the plurality of oligonucleotide barcodes to generate a plurality of barcoded nucleic acid molecules;
   obtaining imaging data of the plurality of partitions to identify the detectable signature of each partition;
   obtaining sequencing data comprising a plurality of sequencing reads of the barcoded particle indexing oligonucleotides, or products thereof, and the plurality of barcoded nucleic acid molecules, or products thereof;
   identifying the particle type identifier (PTI) and/or unique particle identifier (UPI) associated with each cell label sequence in the sequencing data; and
   assigning each of the plurality of sequencing reads to a partition of the plurality of partitions based on the particle type identifier (PTI) and/or unique particle identifier (UPI) associated with each cell label sequence in the sequencing data.
37. The method of claim 36, comprising:
   obtaining phenotypic data of the plurality of single cells;
   associating the sequencing data and the phenotypic data of at least one cell of the plurality of single cells based on the particle type identifier (PTI) and/or unique particle identifier (UPI) of at least one barcoded particle indexing oligonucleotide, or product thereof, of the plurality of barcoded particle indexing oligonucleotides, or products thereof, in the sequencing data.
38. The method of any one of embodiments 36-37, comprising associating the plurality of sequencing reads of the barcoded particle indexing oligonucleotides, or products thereof, of each partition, with the detectable signature of each partition.
39. The method of any one of embodiments 36-38, wherein each partition of the plurality of partitions comprises a unique detectable signature, and wherein the detectable signature of each partition varies depending on the identity of the reference particle(s) in said partitions.
40. The method of any one of embodiments 36-39, wherein identifying the detectable signature of each partition comprises detecting the emissions of the one or more detectable moieties of each partition, optionally said emissions comprise fluorescence emissions.
41. The method of any one of embodiments 36-40, wherein imaging each partition yields phenotypic data.
42. The method of any one of embodiments 36-41, wherein imaging comprises microscopy, time-lapse imaging microscopy, fluorescence microscopy, multi-photon microscopy, quantitative phase microscopy, surface enhanced Raman spectroscopy, videography, manual visual analysis, automated visual analysis, and combinations thereof
43. The method of any one of embodiments 36-42, wherein the particle indexing oligonucleotide is configured to be detachable from the reference particle.
44. The method of any one of embodiments 36-43, wherein the particle indexing oligonucleotide is configured detach from the reference particle during cell lysis.
45. The method of any one of embodiments 36-44, comprising dissociating the particle indexing oligonucleotide from the reference particle.
46. The method of any one of embodiments 36-45, wherein dissociating the particle indexing oligonucleotide from the reference particle comprises detaching the partition indexing oligonucleotide from the partition by UV photocleaving, chemical treatment, heating, enzyme treatment, or any combination thereof.
47. The method of any one of embodiments 36-46, wherein the dissociating occurs after barcoding the particle indexing oligonucleotides.
48. The method of any one of embodiments 36-47, wherein the dissociating occurs before barcoding the particle indexing oligonucleotides.
49. The method of any one of embodiments 36-48, wherein the dissociating occurs during cell lysis.
50. The method of any one of embodiments 36-49, wherein the partitions comprise micro-wells or droplets.
51. A composition comprising:
   a micro-well array,
   wherein the micro-well array comprises at least 100 micro-wells, wherein each micro-well has a volume ranging from about 1,000 µm³ to about 786,000 µm³, wherein each micro-well comprises one or more reference particles of any one of embodiments 1-33.
52. The composition of claim 51, further comprising a cartridge, wherein the cartridge comprises at least one of: an inlet port, an outlet port, a pump, a valve, a vent, a reservoir, a sample collection chamber, a temperature control apparatus, or any combination thereof.
53. A composition comprising:
   a cartridge,
   wherein the cartridge comprises at least one of: an inlet port, an outlet port, a pump, a valve, a vent, a reservoir, a sample collection chamber, a temperature control apparatus, or any combination thereof, wherein the cartridge comprises a micro-well array, wherein the micro-well array comprises at least 100 micro-wells, wherein each micro-well has a volume ranging from about 1,000 µm³ to about 786,000 µm³, wherein each micro-well comprises one or more reference particles of any one of embodiments 1-33.
54. The composition of any one of embodiments 51-53, comprising a buffer.
55. The composition of any one of embodiments 51-54, comprising one or more reagents for a reverse transcription reaction.
56. The composition of any one of embodiments 51-55, comprising one or more reagents for an amplification reaction.
57. The composition of any one of embodiments 51-56, wherein the cartridge comprises a transparent window for optical imaging of the at least 100 microwells.
58. The composition of any one of embodiments 51-57, further comprising an imaging system configured to capture and process images of all or a portion of the at least 100 microwells, wherein the imaging system further comprises an illumination subsystem, an imaging subsystem, and a processor.
59. The composition of any one of embodiments 51-58, wherein the imaging system is configured to perform bright-field, dark-field, fluorescence, or quantitative phase imaging.

## Claims

1. A reference particle, comprising:
one or more detectable moieties, or precursors thereof; and
a particle indexing oligonucleotide, wherein the particle indexing oligonucleotide comprises a particle type identifier (PTI) and/or a unique particle identifier (UPI).

2. The reference particle of claim 1, wherein:
a. the particle type identifier identifies the one or more detectable moieties associated with the reference particle; and/or
b. the particle type identifier identifies the one or more detectable moieties associated with the reference particle and the density of said one or more detectable moieties associated with the reference particle; and/or
c. the unique particle identifier is selected from a diverse set of unique particle identifiers, optionally wherein the diverse set of unique particle identifiers comprises at least about 100 different unique particle identifiers, at least about 1000 different unique particle identifiers, at least about 10000 different unique particle identifiers, or at least about 100000 different unique particle identifiers.

3. The reference particle of any one of claims 1 or 2, wherein:
a. the one or more detectable moieties is at least about 2, 3, 4, 5, 6, or 7, different detectable moieties; and/or
b. the detectable moiety comprises an optical moiety, a luminescent moiety, a magnetic moiety, an electrochemically active moiety, a nanoparticle, or a combination thereof, optionally wherein:
i. the luminescent moiety comprises a chemiluminescent moiety, an electroluminescent moiety, a photoluminescent moiety, or a combination thereof, further optionally wherein the photoluminescent moiety comprises a fluorescent moiety, a phosphorescent moiety, or a combination thereof, further optionally wherein the fluorescent moiety comprises a fluorescent dye; and/or
ii. the nanoparticle comprises a quantum dot.

4. The reference particle of any one of claims 1-3, wherein:
a. the particle type identifier and/or unique particle identifier is at least about 4 nucleotides in length; and/or
b. the particle indexing oligonucleotide is 6-60 nucleotides in length; and/or
c. the particle indexing oligonucleotide is immobilized on the reference particle, partially immobilized on the reference particle, enclosed in the reference particle, partially enclosed in the reference particle, or a combination thereof.

5. The reference particle of any one of claims 1-4, wherein:
a. the reference particle comprises a synthetic particle; and/or
b. the reference particle is disruptable; and/or
c. the reference particle comprises a bead, optionally wherein the bead comprises a Sepharose bead, a streptavidin bead, an agarose bead, a magnetic bead, a conjugated bead, a protein A conjugated bead, a protein G conjugated bead, a protein A/G conjugated bead, a protein L conjugated bead, an oligo(dT) conjugated bead, a silica bead, a silica-like bead, an anti-biotin microbead, an anti-fluorochrome microbead, or any combination thereof; and/or
d. the reference particle comprises a material selected from the group consisting of polydimethylsiloxane (PDMS), polystyrene, glass, polypropylene, agarose, gelatin, hydrogel, paramagnetic, ceramic, plastic, glass, methylstyrene, acrylic polymer, titanium, latex, Sepharose, cellulose, nylon, silicone, and any combination thereof; and/or
e. the reference particle comprises a disruptable hydrogel particle.

6. The reference particle of any one of claims 1-5, wherein the particle indexing oligonucleotide is associated with the reference particle via one or more cleavable linkers, optionally wherein the one or more cleavable linkers comprise acid-labile linkers, base-labile linkers, photocleavable linkers, enzyme-cleavable linkers, or any combination thereof.

7. The reference particle of any one of claims 1-6, wherein the particle indexing oligonucleotide comprises a sequence complementary to a capture sequence configured to capture the particle indexing oligonucleotide.

8. The reference particle of claim 7, wherein the sequence complementary to the capture sequence comprises a poly(dA) region.

9. The reference particle of any one of claims 1-8, wherein:
a. the particle indexing oligonucleotide comprises a particle linker functional group,
wherein the reference particle comprises a particle functional group, and
wherein the particle functional group and the particle linker functional group are associated with each other, optionally wherein the particle linker functional group and the particle functional group are individually selected from the group consisting of C6, biotin, streptavidin, primary amine(s), aldehyde(s), ketone(s), and any combination thereof; and/or
b. the particle indexing oligonucleotide is associated with the reference particle through a particle linker, optionally wherein:
i. the particle linker comprises a carbon chain, optionally the carbon chain comprises 2-30 carbons; and/or
ii. the particle linker comprises 5' amino modifier C12 (5AmMC12), or a derivative thereof; and/or
iii. the particle indexing oligonucleotide is 50-500 nucleotides in length; and/or
c. the particle indexing oligonucleotide is:
i. conjugated to the reference particle through a chemical group selected from the group consisting of a UV photocleavable group, a streptavidin, a biotin, an amine, and a combination thereof; or
ii. non-covalently attached to the particle; and/or
d. the particle indexing oligonucleotide is double-stranded or single-stranded, and wherein the particle indexing oligonucleotide comprises DNA and/or RNA.

10. A population of reference particles, comprising:
two or more reference particles of any one of claims 1-9,
wherein at least two of the two or more reference particles differ from each other with respect to the identity of the one or more detectable moieties associated with the reference particle and/or the density of said one or more detectable moieties associated with the reference particle.

11. The population of claim 10, wherein at least 3, 4, 5, 6, 7, 8, 9, 10, 11, or 12, of the two or more reference particles differ from each other with respect to the one or more detectable moieties associated with the reference particle and/or the density of said one or more detectable moieties on the reference particle.

12. A composition comprising:
a micro-well array,
wherein the micro-well array comprises at least 100 micro-wells, wherein each micro-well has a volume ranging from about 1,000 µm³ to about 786,000 µm³, wherein each micro-well comprises one or more reference particles of any one of claims 1-9.

13. The composition of claim 12, further comprising a cartridge, wherein the cartridge comprises at least one of: an inlet port, an outlet port, a pump, a valve, a vent, a reservoir, a sample collection chamber, a temperature control apparatus, or any combination thereof.

14. A composition comprising:
a cartridge,
wherein the cartridge comprises at least one of: an inlet port, an outlet port, a pump, a valve, a vent, a reservoir, a sample collection chamber, a temperature control apparatus, or any combination thereof, wherein the cartridge comprises a micro-well array, wherein the micro-well array comprises at least 100 micro-wells, wherein each micro-well has a volume ranging from about 1,000 µm³ to about 786,000 µm³, wherein each micro-well comprises one or more reference particles of any one of claims 1-9.

15. The composition of any one of claims 12-14, wherein:
a. the composition comprises a buffer; and/or
b. the composition comprises one or more reagents for a reverse transcription reaction; and/or
c. the composition comprises one or more reagents for an amplification reaction; and/or
d. the cartridge comprises a transparent window for optical imaging of the at least 100 microwells; and/or
e. the composition further comprises an imaging system configured to capture and process images of all or a portion of the at least 100 microwells, wherein the imaging system further comprises an illumination subsystem, an imaging subsystem, and a processor, optionally wherein the imaging system is configured to perform bright-field, dark-field, fluorescence, or quantitative phase imaging.
